# FASCICULE DE BREVET EUROPEEN

(11) **EP 1 370 233 B1**
(45) Date de publication et mention de la délivrance du brevet: **08.08.2007**
(21) Numéro de dépôt: 01271207.1
(22) Date de dépôt: 20.11.2001
(51) Int. Cl.: A61Q 17/04, A61K 8/49, A61K 8/40, A61K 8/37, A61K 8/35

(54) **COMPOSITION FILTRANTE CONTENANT UN DERIVE DE 1,3,5-TRIAZINE, UN DERIVE DU DIBENZOYLMETHANE, ET UN COMPOSE 4,4-DIARYLBUTADIENE**
FILTERZUSAMMENSETZUNG MIT EINEM 1,3,5-TRIAZIN-DERIVAT, EINEM DIBENZOYLMETHAN-DERIVAT UND EINER 4,4-DIARYLBUTADIEN-VERBINDUNG
FILTERING COMPOSITION CONTAINING A 1,3,5-TRIAZINE DERIVATIVE, A DIBENZOYLMETHANE DERIVATIVE, AND A 4,4-DIARYLBUTADIENE COMPOUND

(30) Priorité: 18.12.2000 FR 0016517
(43) Date de publication de la demande: 17.12.2003
(73) Titulaire: L'ORÉAL, 75008 Paris (FR)
(72) Inventeur: CANDAU, Didier, F-91570 Bièvres (FR)
(74) Mandataire: Miszputen, Laurent
(86) Numéro de dépôt international: PCT/FR2001/003639
(87) Numéro de publication internationale: WO 2002/049599

(56) Documents cités:
- EP-A- 0 933 376
- EP-A- 0 967 200
- EP-A- 1 008 586
- WO-A-00/27337
- DE-A- 19 746 654
- DE-A- 19 755 649
- FR-A- 2 440 933
- US-A- 5 738 842
- US-A- 5 928 629

## Description

L'invention se rapporte à une composition cosmétique ou dermatologique, à usage topique, en particulier pour la photoprotection de la peau et des cheveux, caractérisée par le fait qu'elle comprend, dans un support cosmétiquement acceptable :
(a) au moins un dérivé du dibenzoylméthane et
(b) au moins un dérivé de 1,3,5-triazine photosensible en présence d'un dérivé du dibenzoylméthane et
(c) au moins un composé 4,4-diarylbutadiène diarylbutadiène ; le rapport en poids du composé 4,4-diarylbutadiène sur le dérivé de dibenzoylméthane étant supérieur à 2,5 et ladite composition ne contenant pas de dérivé de cinnamate.

L'invention se rapporte également à un procédé pour améliorer la photostabilité d'un dérivé de 1,3,5-triazine photosensible en présence d'un filtre UV du type dérivé de dibenzoylméthane consistant à ajouter à l'association dérivé de triazine/dérivé de dibenzoylméthane une quantité efficace d'au moins un composé 4,4-diarylbutadiène.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible
ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV- A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de "PARSOL 1789" par la Société HOFFMANN LAROCHE.

Les dérivés de 1,3,5-triazine sont particulièrement recherchés dans la cosmétique solaire du fait qu'ils sont fortement actifs dans l' UVB et même dans l'UV-A pour certains de ces composés selon la nature des substituants mis en jeu. Ils sont notamment décrits dans les demandes de brevet US 4367390, EP863145, EP517104, EP570838, EP507691, EP796851, EP775698, EP878469 et EP933376, et on connaît en particulier :
- la 2,4,6-tris[p-(2'-éthylhexyl-1'-oxycarbonyl)anilino]-1,3,5-triazine ou « Ethylhexyl Triazone » (nom INCI), vendue sous la dénomination commerciale « Uvinul T 150 » par la société BASF,
- la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » (nom INCI) vendu sous le nom commercial « UVASORB HEB » par SIGMA 3V. Ils possèdent un fort pouvoir absorbant des UVB et il serait donc très intéressant de pouvoir les utiliser en association avec le 4-tert-butyl-4'-méthoxydibenzoylméthane cité ci-dessus dans le but d'obtenir des produits offrant une protection large et efficace dans l'ensemble du rayonnement UV.

On connaît dans les demandes de brevet EP0967200, DE19746654, DE19755649, EP1008 586, DE 100 07 017, EP 1133980 et EP 1133981 des compositions solaires à base de 4,4-diarylbutadiènes pouvant contenir d'autres filtres complémentaires comme les dérivés de dibenzoylméthane et les dérivés de triazine tels que mentionnés ci-dessus.

Toutefois, la Demanderesse a constaté que certains de ces dérivés de 1,3,5-triazine, lorsqu'ils sont en présence de 4-tert-butyl-4'-méthoxydibenzoylméthane sont photosensibles à savoir : sous irradiation UV, ils présentent l'inconvénient de se dégrader chimiquement de façon importante. Dans ces conditions, l'association des deux filtres ne permet plus une protection solaire large prolongée de la peau et des cheveux.

Or, à la suite d'importantes recherches menées dans le domaine de la photoprotection évoqué ci-dessus, la Demanderesse a maintenant découvert que l'introduction d'un composé 4,4-diarylbutadiène dans une composition contenant du 4-tert-butyl-4'-méthoxydibenzoylméthane en association avec au moins un dérivé de 1,3,5-triazine photosensible en présence dudit dibenzoylméthane, permettait d'améliorer de façon tout à fait remarquable la photostabilité de ce dérivé de 1,3,5-triazine au sein de telles compositions, et donc l'efficacité globale de ces compositions.

Cette découverte est à la base de l'invention.

La présente invention a donc pour objet une composition cosmétique ou dermatologique, à usage topique, en particulier pour la photoprotection de la peau et des cheveux, caractérisée par le fait qu'elle comprend au moins, dans un support cosmétiquement acceptable :
(a) un dérivé du dibenzoylméthane et
(b) un dérivé de 1,3,5-triazine photosensible en présence d'un dérivé du dibenzoylméthane et
(c) un composé 4,4-diarylbutadiène diarylbutadiène ; le rapport en poids du composé 4,4-diarylbutadiène sur le dérivé de dibenzoylméthane étant supérieur à 2,5 et ladite composition ne contenant pas de dérivé de cinnamate.

Ainsi, selon la présente invention, on peut réaliser des compositions cosmétiques et/ou dermatologiques contenant du 4-tert-butyl-4'-méthoxydibenzoylméthane en association avec au moins un dérivé de 1,3,5-Triazine photosensible, compositions dans lesquelles la concentration en dérivé de 1,3,5-triazine reste relativement constante même si ces compositions sont soumises à l'action de la lumière.

La présente invention a encore pour objet l'utilisation d'un composé 4;4-diarylbutadiène dans des compositions cosmétiques contenant un dérivé du dibenzoylméthane en association avec au moins un dérivé de 1,3,5-triazine photosensible en vue d'améliorer dans lesdites compositions la stabilité au rayonnement UV (photostabilité) dudit dérivé de 1,3,5-triazine ; le rapport en poids du composé 4,4diarylbutadiène sur le dérivé de dibenzoylméthane étant supérieur à 2,5 et ladite composition ne contenant pas de dérivé de cinnamate.

La présente invention a également pour objet un procédé pour améliorer la stabilité au rayonnement UV (photostabilité) d'un dérivé de 1,3,5-triazine photosensible en présence d'un dérivé de dibenzoylméthane; ledit procédé consistant à ajouter à ladite association une quantité efficace d'un composé 4;4-diarylbutadiène.

Par quantité efficace de 4,4-diarylbutadiène conforme à l'invention, on entend une quantité suffisante pour obtenir une amélioration notable et significative de la photostabilité du dérivé de 1,3,5-triazine dans la composition cosmétique photoprotectrice. Cette quantité minimale en agent photostabilisant à mettre en oeuvre, qui peut varier selon la nature du support cosmétiquement acceptable retenu pour la composition, peut être déterminée sans aucune difficulté au moyen d'un test classique de mesure de photostabilité.

Par composé 4,4-diarylbutadiène conforme à l'invention, on entend toute molécule comportant au moins un groupe chromophore 4,4-diarylbutadiène. Celle-ci peut se présenter sous forme de composé simple, d'oligomère ou de polymère greffé dans la chaîne par le groupe chromophore.

D'autres caractéristiques, aspects et avantages de la présente invention apparaîtront à la lecture de la description détaillée qui va suivre.

Un premier composé des compositions selon l'invention est donc un dérivé de 1,3,5-triazine photosensible en présence d'un dérivé du dibenzoylméthane.

Parmi les dérivés de 1,3,5-triazine utilisables dans le cadre de la présente invention, on peut utiliser notamment ceux répondant à la formule (I) suivante : 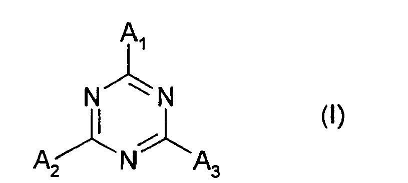 dans laquelle les radicaux A₁, A₂ et A₃, identiques ou différents sont choisis parmi les groupes de formules (II) : 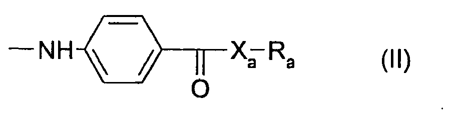 dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (III), (IV) ou (V) suivantes : 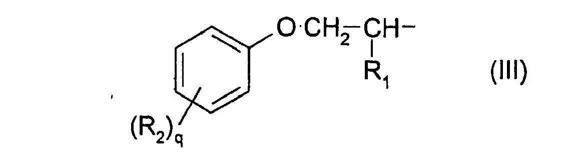 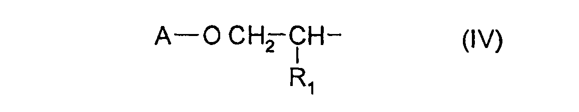 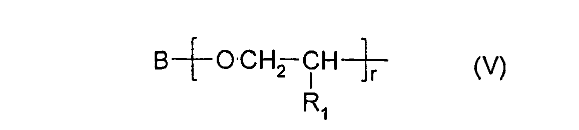
dans lesquelles :
- R₁ est l'hydrogène ou un radical méthyle;
- R₂ est un radical alkyle en C₁-C₉;
- q est un nombre entier allant de 0 à 3;
- r est un nombre entier allant de 1 à 10;
- A est un radical alkyle en C₄-C₈ ou un radical cycloalkyle en C₅-C₈;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.

Une première famille plus particulièrement préférée de dérivés de 1,3,5-triazine, notamment décrite dans le document EP-A-0 517 104, est celle des 1,3,5-triazines répondant à la formule (I) dans laquelle les A₁, A₂ et A₃ sont de formule (II) et présentent les caractéristiques suivantes :
- un Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (III), (IV) ou (V) ci-dessus dans lesquelles :

- B est un radical alkyle en C₁-C₄;
- R₂ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :

- B est un radical alkyle en C₁-C₄;
- R₂ est le radical méthyle.

Une deuxième famille plus particulièrement préférée de dérivés de 1,3,5-triazine, notamment décrite dans le document EP-A-0 570 838, est celle des 1,3,5-triazines répondant à la formule (I) dans laquelle les A₁, A₂ et A₃ sont de formule (II) et présentant l'ensemble des caractéristiques suivantes :
- un ou deux Xₐ-Rₐ représente le radical -NH-Rₐ, avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (III), (IV) ou (V) ci-dessus dans lesquelles:

- B est un radical alkyle en C₁-C₄ ;
- R₂ est le radical méthyle ; le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :

- B est un radical alkyle en C₁-C₄;
- R₂ est le radical méthyle.

Une 1,3,5-triazine particulièrement préférée de cette deuxième famille est la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » vendue sous le nom commercial « UVASORB HEB » par SIGMA 3V et répondant à la formule suivante : 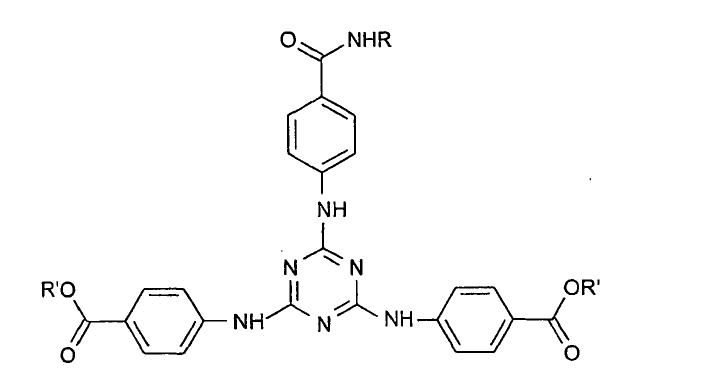 dans laquelle R' désigne un radical éthyl-2 hexyle et R désigne un radical tert-butyle.

Une troisième famille préférée de composés utilisables dans le cadre de la présente invention, et qui est notamment décrite dans le document US 4,724,137, est celle des 1,3,5-triazines répondant à la formule (I) dans laquelle les A₁, A₂ et A₃ sont de formule (II) et présentent les caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine ou « Ethylhexyl Triazone » vendue notamment sous la dénomination commerciale de "UVINUL T 150" par la Société BASF et répond à la formule suivante : 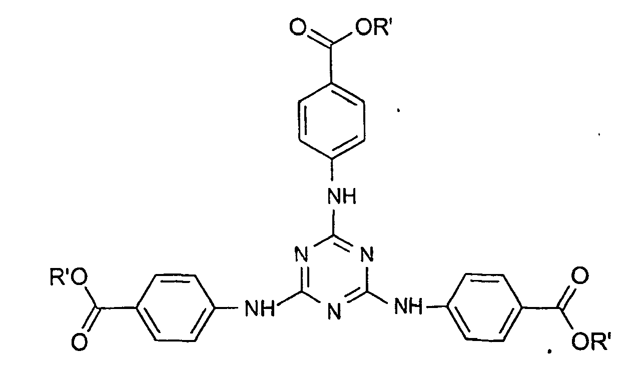 dans laquelle R' désigne un radical 2-éthyl hexyle.

Le ou les dérivés de 1,3,5-triazine sont généralement présents dans les compositions de l'invention à une teneur pouvant aller de 0,5 % à 15 %, de préférence de 1 % à 10 % en poids, par rapport au poids total de la composition.

Un deuxième composé des compositions visées par la présente invention est le dérivé de dibenzoylméthane. Comme indiqué précédemment, les dérivés du dibenzoylméthane visés par la présente invention sont des produits déjà bien connus en soi et décrits notamment dans les documents FR-A- 2 326 405, FR-A- 2 440 933 et EP-A- 0 114 607, documents dont les enseignements sont, pour ce qui touche à la définition même de ces produits, totalement inclus à titre de références dans la présente description.

Parmi les dérivés du dibenzoylméthane plus particulièrement visés par la présente invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

Parmi les dérivés du dibenzoylméthane mentionnés ci-dessus, on préfère tout particulièrement, selon la présente invention, mettre en oeuvre le 4-tert-butyl-4'-méthoxydibenzoylméthane, notamment celui proposé à la vente sous la dénomination commerciale de "PARSOL 1789" par la Société Hoffmann Laroche, ce filtre répondant à la formule développée (VI) suivante :

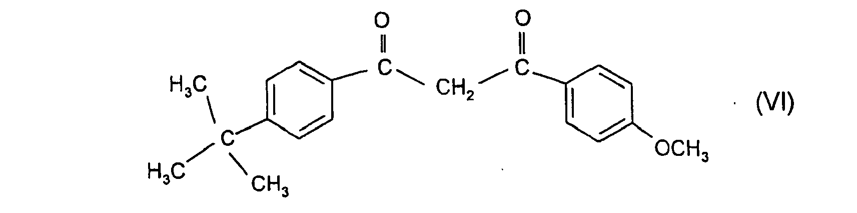

Un autre dérivé du dibenzoylméthane préféré selon la présente invention est le 4-isopropyldibenzoylméthane, filtre vendu sous la dénomination de "EUSOLEX 8020" par la Société Merck, et répondant à la formule développée (VII) suivante

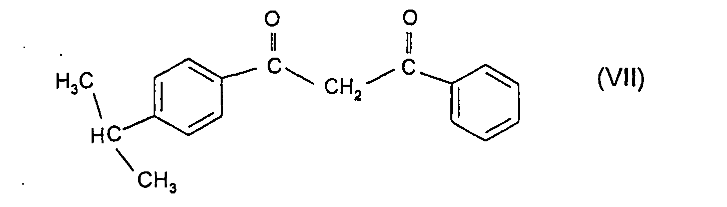

Le ou les dérivés du dibenzoylméthane sont présents dans les compositions conformes à l'invention à des teneurs qui sont de préférence allant de 0,5 à 15% en poids et plus préférentiellement de 1 % à 10 % en poids, par rapport au poids total de la composition.

Parmi les composés 4,4-diarylbutadiènes conformes à l'invention préférés, on peut choisir les composés répondant à la formule (VIII) suivante : 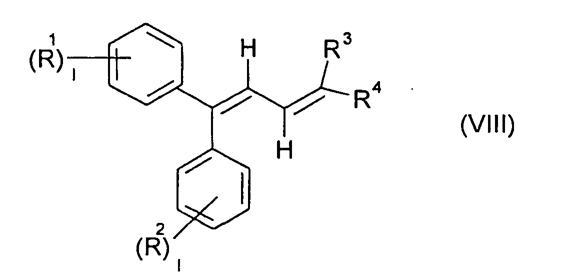 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₂₀, linéaire ou ramifié ; un radical alcényle en C₂- C₁₀ ; un radical alcoxy en C₁-C₁₂ un radical cycloalkyle en C₃-C₁₀ ; un radical cycloalcényle en C₃-C₁₀ ; un radical alcoxycarbonyle en C₁-C₂₀ linéaire ou ramifié ; un radical monoalkylamino en C₁-C₁₂, linéaire ou ramifié ; un radical dialkylamino en C₁-C₁₂ , linéaire ou ramifié ; un aryle ; un hétéroaryle ou un substituant hydrosolubilisant choisi parmi un groupe carboxylate, un groupe sulfonate ou un reste ammonium ;
- R³ désigne un groupe COOR⁵ ; COR⁵ ; CONR⁵R⁶ ; CN ; un radical alkyle en C₁-C₂₀, linéaire ou ramifié ; un radical alcényle en C₂- C₁₀ ; un radical cycloalkyle en C₃-C₁₀; un radical bicycloalkyle en C₇-C₁₀; un radical cycloalcényle en C₃-C₁₀; un radical bicycloalcényle en C₇-C₁₀ ; un aryle en C₆-C₁₈ ; un hétéroaryle en C₃-C₇;
- R⁴ désigne un groupe COOR⁶; COR⁶ ; CONR⁵R⁶ ; CN ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀; un radical cycloalcényle en C₃-C₁₀ ; ; un radical bicycloalcényle en C₇-C₁₀ ; un aryle ; un hétéroaryle ;
- R⁵ et R⁶, identiques ou différents, désignent hydrogène ; [V]ₒ-R⁷, C₁-C₆-alkylène-SO₃U; C₁-C₆-alkylène-PO₃U ; C₁-C₆-alkylène-N(R⁸)₃⁺ D⁻; un radical alkyle en C₁-C₂₀, linéaire ou ramifié ; un radical alcényle en C₂-C₁₀ ; un radical cycloalkyle en C₃-C₁₀; un radical bicycloalkyle en C₇-C₁₀ ; un radical cycloalcényle en C₃-C₁₀ ; un radical bicycloalcényle en C₇-C₁₀ ; un aryle ; un hétéroaryle ;
- V désigne un groupe -CH₂-CH₂-W-, -CH₂CH₂CH₂W-, -CH(CH₃)-CH₂-W-,
- CH₂-CH₂-CH₂-CH₂-W-, -CH₂-CH(CH₂CH₃)-W- ;
- D désigne Cl, Br, I, SO₄R⁹ ;
- U désigne hydrogène, Na⁺, K⁺, Mg²⁺, Ca²⁺, Li⁺, Al³⁺, -N(R⁸)₄ +
- W désigne O ou NH ;
- R⁷ et R identiques ou différents, désignent hydrogène, un radical alkyle en C₁-C₆, linéaire ou ramifié ; un radical alcényle en C₂- C₆, linéaire ou ramifié ; un radical acyle en C₁-C₆ linéaire ou ramifié ;
- R⁹ désigne hydrogène, un radical alkyle en C₁-C₆. linéaire ou ramifié ; un radical alcényle en C₂- C₆ ;
- l varie de 1 à 3;
- o varie de 0 à 150.

Comme radicaux alkyle, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : éthènyle, n-propènyle, 1-méthyléthènyle, n-butènyle, 1-méthylpropènyle, 2-méthylpropènyle, 1,1-diméthyléthènyle, n-pentènyle, 1-méthylbutènyle, 2-méthylbutènyle, 3-méthylbutènyle, 2,2-diméthylpropènyle, 1-éthylpropènyle, n-hexènyle, 1,1-diméthylpropènyle, 1,2-diméthylpropènyle, 1-méthylpentènyle, 2-méthylpentènyle, 3-méthylpentènyle, 4-méthylpentènyle,1,1-diméthylbutènyle,1,2-diméthylbutènyle,1,3-diméthylbutèn yle,2,2-diméthylbutènyle, 2,3-diméthylbutènyle, 3,3-diméthylbutènyle, 1-éthylbutènyle, 2-éthylbutènyle, 1,1,2-triméthytpropènyle, 1,2,2-triméthylpropènyle, 1-éthyl-1-méthylpropènyle, 1-éthyl-2-méthylpropènyle, n-heptènyle, n-octènyle, n-nonènyle, n-décènyle.

Comme radicaux alcoxy en C₁-C₁₂ pour les radicaux R¹ et R², on peut citer : méthoxy, n-propoxy, 1-méthyléthoxy, 1-méthylpropoxy, n-pentoxy, 3-méthylbutoxy, 2,2-diméthylpropoxy, 1 -méthyl- 1-éthylpropoxy, octoxy, éthoxy, n-propoxy, n-butoxy, 2-méthylpropoxy, 1,1-diméthylpropoxy, hexoxy, heptoxy, 2-éthylhexoxy.

Comme radicaux cycloalkyles en C₃-C₁₀ pour les radicaux R⁶ et R⁷, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, pour les radicaux R⁶ et R⁷, on peut citer : cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino; C₁-C₄alkyle; C₁-C₄-alcoxy; hydroxy; ils peuvent également comporter de 1 à 3 hétéroatomes comme souffre, oxygène ou azote dont les valences libres peuvent être saturées par un hydrogène ou un radical alkyle en C₁-C₄.

Comme radicaux acyle, on peut citer par exemple formyle, acétyle, propionyle, ou n-butyryle.

Les groupes bicycloalkyles ou bicycloalcènyles sont choisis par exemple parmi les terpènes bicycliques comme les dérivés de pinane, de bornane, de pinène ou de camphre ou d'adamantane.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro; amino ; C₁-C₄-alkylamino ; C₁-C₄ dialkylamino; C₁-C₄alkyle ; C₁-C₄-alcoxy; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Les composés de formule (VIII) préférentiels sont choisis parmi ceux de formule (Vllla) suivante : 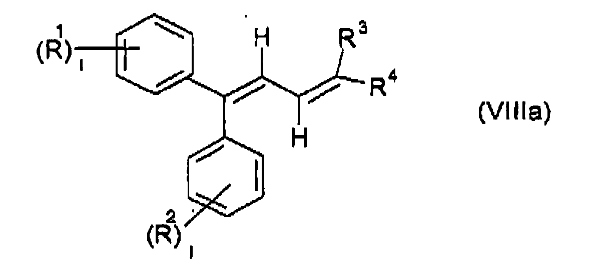 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ; un substituant hydrosolubilisant choisi parmi un groupe carboxylate, un groupe sulfonate ou un reste ammonium :

- R³ désigne un groupe COOR⁵ ; CONR⁵R⁶; CN ;
- R⁴ désigne un groupe COOR⁶ ; CONR⁵R⁶ ;
- R⁵ désigne hydrogène ; [V]ₒ-R⁷ ; C₁-C₈-alkylène-SO₃U ; C₁-C₆-alkylène-N(R⁸)₃⁺D⁻
- R⁶ désigne [V]ₒ-R⁷ ; C₁-C₆-alkylène-SO₃U ; C₁-C₆-alkylène-N(R⁸)₃⁺ D⁻ ;
- V désigne un groupe -CH₂-CH₂-O-, -CH₂CH₂CH₂O-, -CH(CH₃)-CH₂-O-,
- D désigne Cl, Br, 1, SO₄R⁶ ;
- U désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié:
   - ℓ varie de 1 à 3
   - o varie de 0 à 50 .

Les composés de formule (VIII) encore plus préférentiels sont choisis parmi ceux répondant à la formule (VIIIb) suivante; 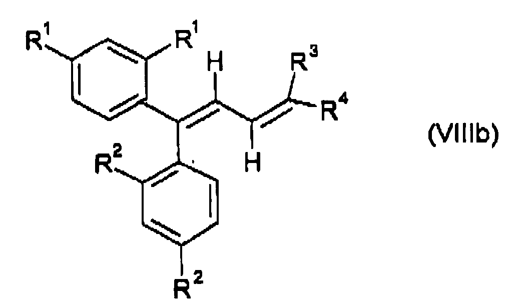 dans laquelle le système diène est de configuration Z,Z; Z,E; E,Z ou E,E ou des mélanges desdites configurations et où:
- R¹ et R², identiques ou différents, désignent hydrogène, un radical alkyle en C₁-C₆; un radical alcoxy en C₁-C₈;
- R³ désigne un groupe COOR⁵; CONR⁶R⁶; CN;
- R⁴ désigne un groupe COOR⁶; CONR⁵R⁶ ;
- R⁵ désigne hydrogène; [V]ₒ-R⁷ ; C₁-C₆-alylène-SO₃U ;C₁-C₆-alylène-N(R⁸)₃⁺ D⁻
- R⁶ désigne [V]ₒ-R⁷; C₁-C₆-alkylène-SO₃U ; C₁-C₆-alkylène-N(R⁸)₃⁺ D⁻ ;
- V désigne un groupe -CH₂-CH₂-O-, -CH₂CH₂CH₂O-, -CH(CH₃)-CH₂-O-,
- D désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié ;
- o varie de 0 à 50.

Les composés de formule (VIII) encore plus préférentiels sont choisis parmi ceux répondant à la formule (VIIIc) suivante : 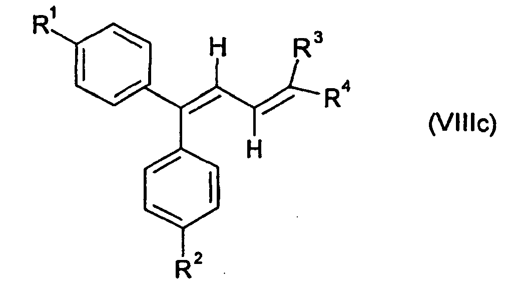 dans laquelle le système diène est de configuration Z,Z ; Z,E ; E,Z ou E,E ou des mélanges desdites configurations et où:
- R¹ et R² identiques ou différents, désignent hydrogène, un radical alkyle en C₁-C₈; un radical alcoxy en C₁-C₈;
- R³ désigne un groupe COOR⁵; CONR⁵R⁶; CN;
- R⁴ désigne un groupe COOR⁶; CONR⁵R⁶;
- R⁵ désigne hydrogène ; [V]ₒ-R⁷; C₁-C₆-alkylène-SO₃Y; C₁-C₆-alkylène-N(R⁸)₃+D⁻;
- R⁶ désigne [V]ₒ-R⁷; C₁-C₆-alkylène-.SO₃U; C₁-C₆-alkylène-N(R⁸)₃⁺ D⁻;
- V désigne un groupe -CH₂-CH₂-O-, -CH₂CH₂CH₂O-, -CH(CH₃)-CH₂-O-,
- D désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié;
- o varie de 0 à 50.

Les composés de formule (VIII) encore plus particulièrement préférés sont choisis parmi les composés suivants :

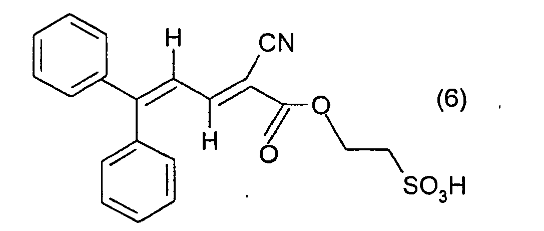

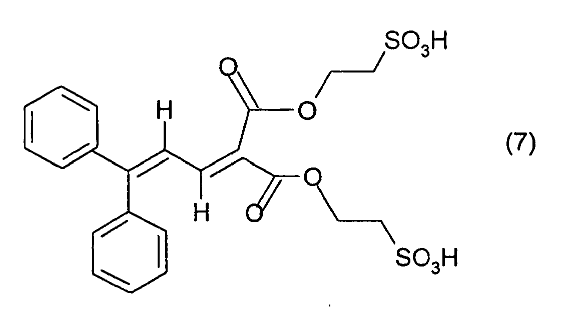

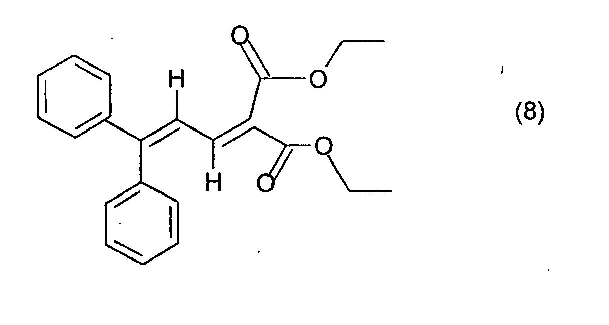

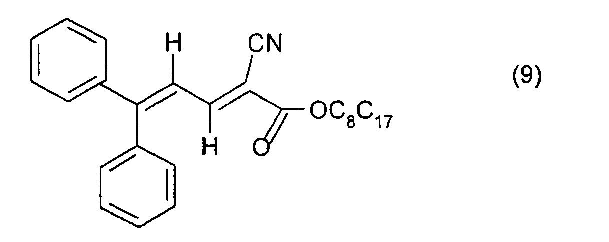

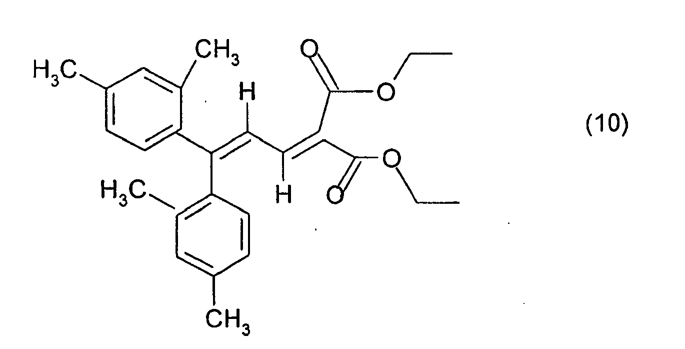

Les composés de formule (VIII) tels que définis ci-dessus sont connus en eux-mêmes et leurs structures et leurs synthèses sont décrites dans les demandes de brevet EP0967200 , DE 19746654 et DE19755649 (faisant partie intégrante du contenu de la description).

Parmi les composés 4,4-diarylbutadiènes conformes à l'invention préférés, on peut également citer les oligomères répondant à la formule (IX) suivante : 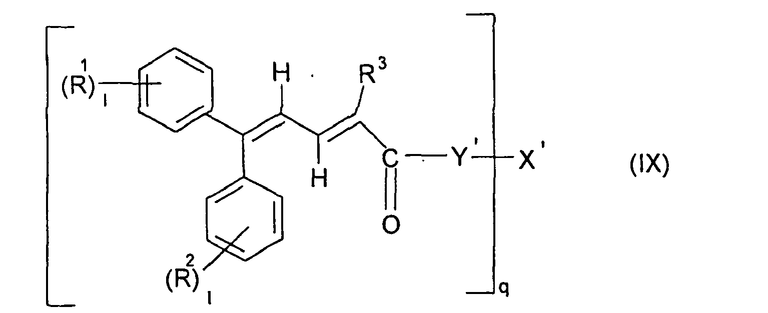 dans laquelle le système diène est de configuration Z,Z ; Z,E ; E,Z ou E,E ou des mélanges desdites configurations et où :
- R¹, R², R³ et 1 ont les mêmes significations indiquées dans la formule (VIII) précédente ;
- Y' désigne un groupe -O- ou -NR¹⁰-
- R¹⁰ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀; un radical bicycloalcényle en C₇-C₁₀ ; un aryle ; un hétéroaryle ;
- X' désigne un reste de polyol linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- 1 varie de 2 à 10.

X' est un reste polyol contenant de 2 à 10 groupes hydroxyles et notamment :

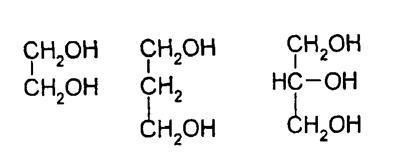

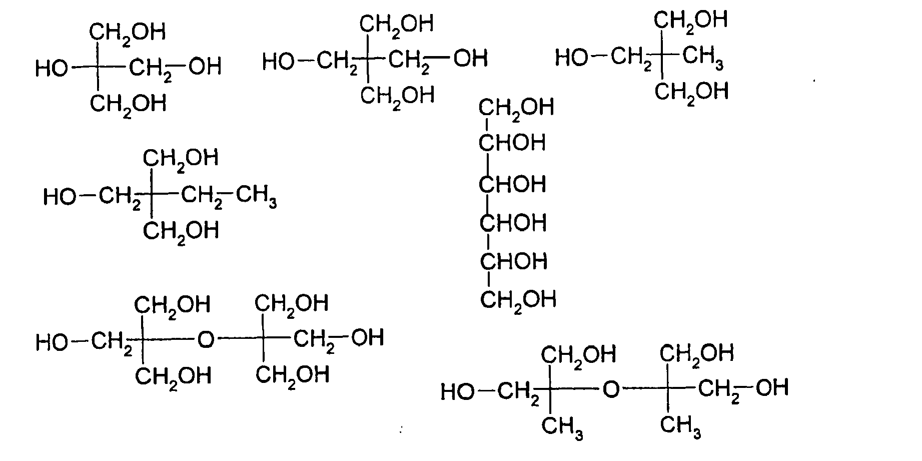

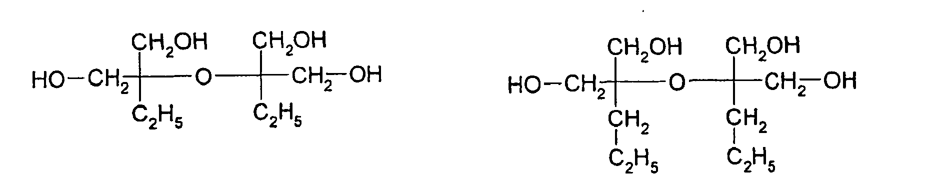

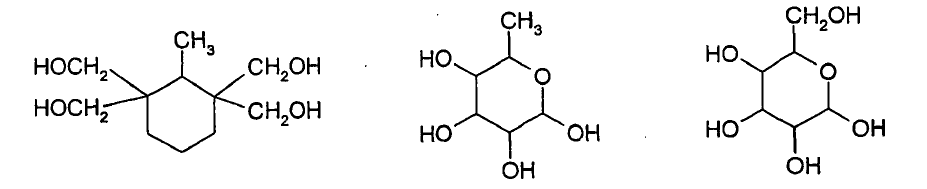

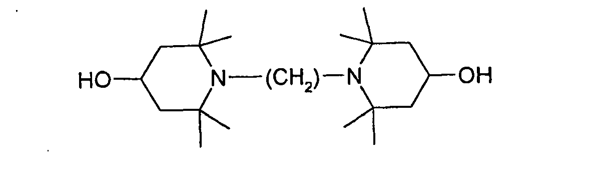

Les composés plus préférentiels de formule (IX) sont ceux pour lesquels :
- ¹ et R², identiques ou différents, désignent hydrogène, un radical alkyle en C₁-C₁₂; un radical alcoxy en C₁-C₈; un substituant hydrosolubilisant choisi parmi un groupe carboxylate, un groupe sulfonate ou un reste ammonium;
- R³ désigne un groupe COOR⁵; CONR⁵R⁶; CN ; un radical cycloalkyle en C₃-C₁₀; un radical bicycloalkyle en C₇-C₁₀;
- R⁵ et R⁶ identiques ou différents, désignent un radical alkyle en C₁-C₂₀, linéaire ou ramifié ; un radical cycloalkyle en C₃-C₁₀ ; un radical bicycloalkyle en C₇-C₁₀ ; naphtyle ou phényle éventuellement substitué ;
- X' désigne un reste de polyol comprenant de 2 à 6 groupes hydroxy et plus particulièrement de 2 à 4.

Les composés encore plus préférentiels de formule (IX) sont ceux pour lesquels :
- X' désigne un reste d'éthanol ou de pentaérythrol.

Les composés de formule (IX) encore plus particulièrement préférés sont choisis parmi les composés suivants :

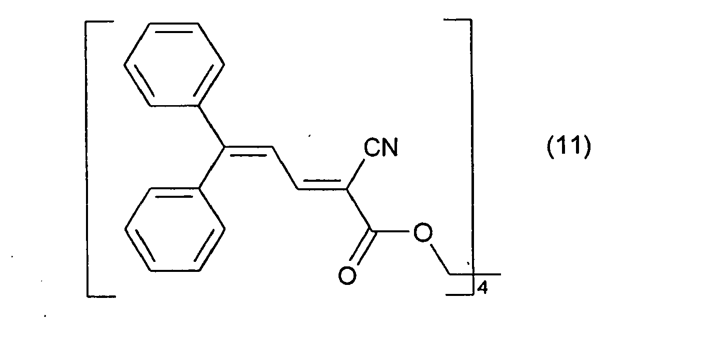

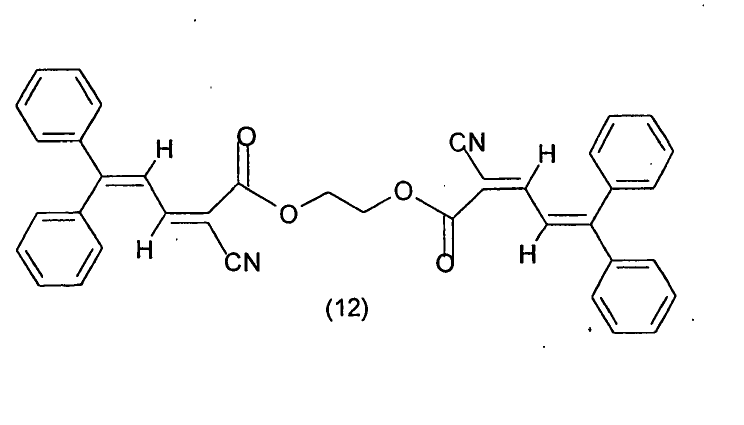

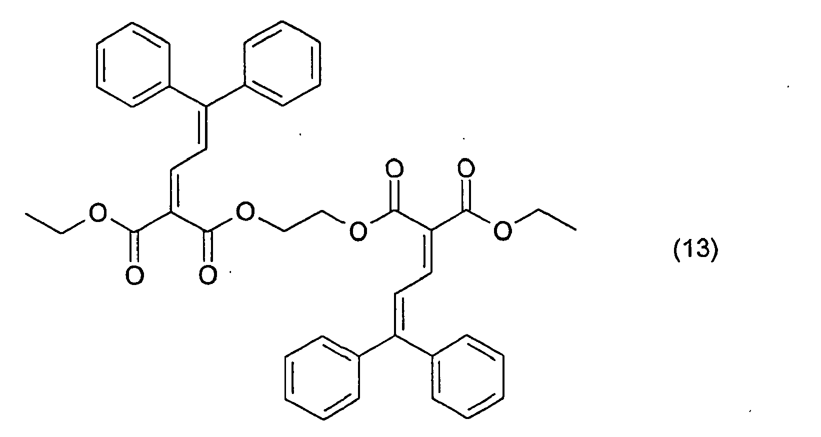

Les composés de formule (IX) tels que définis ci-dessus sont connus en eux-mêmes et leurs structures et leurs synthèses sont décrites dans la demande de brevet EP-A-1008586 (faisant partie intégrante du contenu de la description).

Les composés 4,4-diarylbutadiène conformes à l'invention sont présents dans la composition de l'invention à des teneurs qui de préférence varient de 0,5 à 15% en poids et plus préférentiellement de 1 % à 10 % en poids, par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates ; les dérivés salicyliques, les dérivés du camphre ; les dérivés de la benzophénone ; les dérivés de β,β'-diphénylacrylate, les dérivés de benzotriazole ; les dérivés de triazine non-photosensibles ; les dérivés de benzalmalonate ; les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649.

Comme exemples de filtres organiques complémentaires actifs dans l'UV-A et/ou l'UV-B, on peut citer désignés ci-dessus sous leur nom INCI :

### Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

### Dérivés de β,β'-diphénylacrylate:

- Octocrylene vendu notamment sous le nom commercial « UVINUL N539 » par BASF,
- Etocrylene, vendu notamment sous le nom commercial « UVINUL N35 » par BASF,

### Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

### Dérivé du benzylidène camphre :

- 3-Benzylidene camphor fabriqué sous le nom « MEXORYL SD» par CHIMEX,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

### Dérivés de benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial commercial « NEO HELIOPAN AP » par HAARMANN et REIMER,

### Dérivés de triazine :

- Anisotriazine vendu sous le nom commercial «TINOSORB S » par CIBA SPECIALTY CHEMICALS
- la 2,4,6-tris-(4'-amino benzalmalonate de düsobutyle)-s-triazine.

### Dérivés de benzotriazole :

- Drometrizole Trisiloxane vendu sous le nom « SILATRIZOLE » par RHODIA CHIMIE,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques:

- Menthyl anthranilate vendu sous le nom commercial commercial « NEO HELIOPAN MA » par HAARMANN et REIMER,

### Dérivés d'imidazolines:

- Ethylhexyl Dimethoxybenzylidene Dioxoimidazoline Propionate,

### Dérivés de benzalmalonate:

- Polyorganosiloxane à fonction benzalmalonate vendu sous la dénomination commerciale « PARSOL SLX » par HOFFMANN LA ROCHE et leurs mélanges.

Les filtres UV organiques plus particulièrement préférés sont choisis parmi les composés suivants :
- Ethylhexyl Salicylate,
- Octocrylene,
- Phenylbenzimidazole Sulfonic Acid,
- Benzophenone-3,
- Benzophenone-4,
- Benzophenone-5,
- 4-Methylbenzylidene camphor,
- Terephthalylidene Dicamphor Sulfonic Acid,
- Disodium Phenyl Dibenzimidazole Tetra-sulfonate,
- la 2,4,6-tris-(4'-amino benzalmalonate de diisobutyle)-s-triazine
- Anisotriazine,
- Methylène bis-Benzotriazolyl Tetramethylbutylphénol
- Drometrizole Trisiloxane et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la photostabilité du dérivé de triazine, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et . FR2416008)..

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara du ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un objet de l'invention est l'utilisation d'une composition telle que définie précédemment pour la fabrication d'une composition cosmétique ou dermatologique destinées à la protection de la peau et/ou des cheveux contre le rayonnement ultraviolet, en particulier le rayonnement solaire.

Des exemples concrets, mais nullement limitatifs, illustrant l'invention, vont maintenant être donnés.

| **COMPOSITION** | | **EX 1** |
|---|---|---|
| Mélange mono /distéarate de glycerol /stéarate de polyéthylène | | 2 |
| glycol (100 OE) (ARLACEL 165 FL- ICI) | | |
| Alcool stéarylique (LANETTE 18 - HENKEL) | | 1 |
| Acide stéarique d'huile de palme | | 2.5 |
| (STEARINE TP - STEARINERIE DUBOIS) | | |
| Poly diméthylsiloxane | | 0.5 |
| (DOW CORNING 200 FLUID- DOW CORNING) | | |
| Benzoate d'alcools en C12/C15 | | 20 |
| (WITCONOL TN -WITCO) | | |
| Triéthanolamine | | 0.5 |
| Butyl Methoxydibenzoylmethane | | 2 |
| (PARSOL 1789 HOFFMANN LA ROCHE) | | |
| Ethylhexyl triazone | | 5 |
| (UVINUL T150, BASF) | | |
| Composé de formule (6) | | 8 |
| Glycérine | | 4 |
| Triéthanolamine | | 0.3 |
| Acide polyacrylique | | 0.4 |
| (SYNTHALEN K - 3V) | | |
| Conservateurs | | qs |
| Eau démineralisée | qsp | 100 g |

| **COMPOSITION** | **EX 2** |
|---|---|
| Mélange d'alcool cétylstéarylique et d'alcool cétylstéarylique oxyéthyléné (33 OE) 80/20 (SINNOWAX AO -HENKEL) | 7 |
| Mélange de mono et distéarate de glycérol (CERASYNT SD-V ISP) | 2 |
| Alcool cétylique | 1.5 |
| Polydiméthyl siloxane (DOW CORNING 200 FLUID -DOW CORNING) | 1.5 |
| Huile de vaseline | 15 |
| Butyl Methoxydibenzoylmethane (PARSOL 1789 HOFFMANN LA ROCHE) | 2 |
| Composé de formule (11) | 6 |
| Ethylhexyl triazone (UVINUL T150, BASF) | 5 |
| glycérine | 15 |
| Conservateurs | qs |
| Eau déminéralisée qsp | 100 g |

## Revendications (Revendications pour l'(les) Etat(s) contractant(s) suivant(s): AT, BE, CH, CY, DK, ES, FI, FR, GB, GR, IE, IT, LI, LU, MC, NL, PT, SE, TR)

1. Composition cosmétique ou dermatologique, à usage topique, en particulier pour la photoprotection de la peau et des cheveux, **caractérisée par le fait qu'**elle comprend, dans un support cosmétiquement acceptable :
(a) au moins un dérivé du dibenzoylméthane et
(b) au moins un dérivé de 1,3,5-triazine photosensible en présence d'un dérivé du dibenzoylméthane et
(c) au moins un composé 4,4-diarylbutadiène; le rapport en poids du composé 4,4-diarylbutadiène sur le dérivé de dibenzoylméthane étant supérieur à 2,5 et ladite composition ne contenant pas de dérivé de cinnamate.

2. Composition selon la revendication 1, où le dérivé de 1,3,5-triazine répond à la formule (I) suivante: 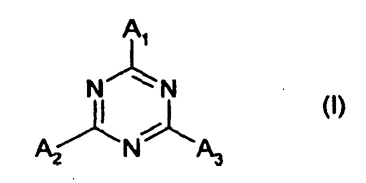 dans laquelle les radicaux A₁, A₂ et A₃, identiques ou différents sont choisis parmi les groupes de formule (II): 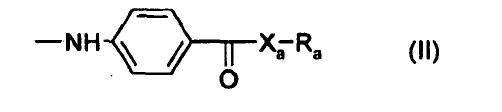 dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈ ; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (III), (IV) ou (V) suivantes : 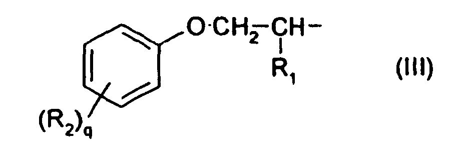 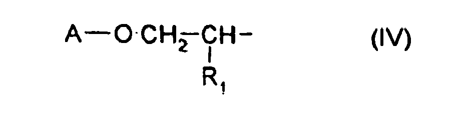 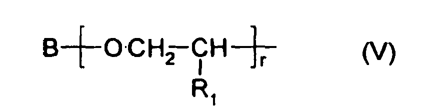
dans lesquelles :
- R₁ est l'hydrogène ou un radical méthyle;
- R₂ est un radical alkyle en C₁-C₉;
- q est un nombre entier allant de 0 à 3;
- r est un nombre entier allant de 1 à 10;
- A est un radical alkyle en C₄-C₈ ou un radical cycloalkyle en C₅-C₈;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.

3. Composition selon la revendication 2, **caractérisée par le fait que** le dérivé de 1,3,5-triazine de formule (1) est choisi parmi ceux où les A₁, A₂ et A₃ sont de formule (II) et présentent les caractéristiques suivantes :
- un Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄ ;
- R₂ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

4. Composition selon la revendication 2, **caractérisée par le fait que** le dérivé de 1,3,5-triazine est choisi parmi ceux où les A₁, A₂ et A₃ sont de formule (II) et présentant l'ensemble des caractéristiques suivantes :
- un ou deux Xₐ-Rₐ représente le radical -NH-Rₐ, avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles:
- B est un radical alkyle en C₁-C₄;
- R₂ est le radical méthyle.

5. Composition selon la revendication 4, **caractérisée par le fait que** le dérivé de 1,3,5-triazine est celui répondant à la formule suivante : 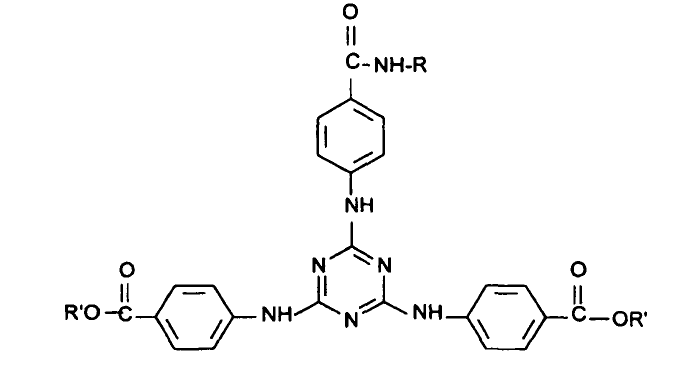 dans laquelle R' désigne un radical éthyl-2 hexyle et R désigne un radical tertio-butyle.

6. Composition selon la revendication 2, **caractérisée par le fait que** le dérivé de 1,3,5-triazine répondant à la formule (I) est choisi parmi ceux où les A₁, A₂ et A₃ sont de formule (II) et présentent les caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

7. Composition selon la revendication 6, **caractérisée par le fait que** le dérivé de 1,3,5-triazine est celui répondant à la formule suivante : 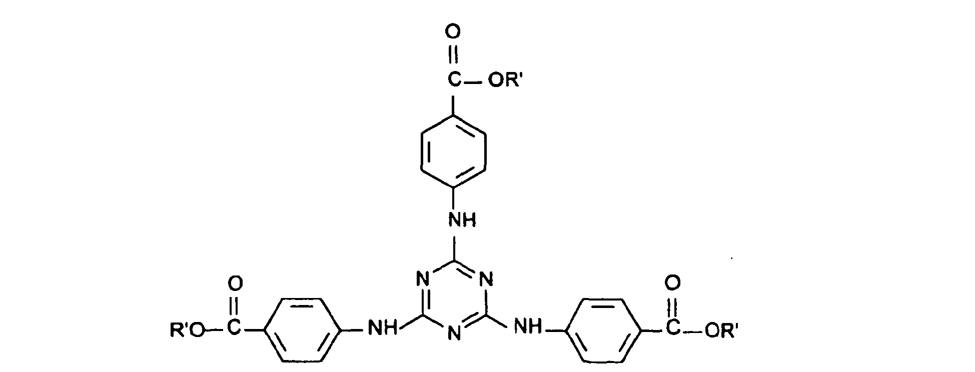 dans laquelle R' désigne un radical 2-éthyl hexyle.

8. Composition selon l'une quelconque des revendications 1 à 7, **caractérisée par le fait que** le dérivé de 1,3,5-triazine est présent à des teneurs allant de 0,5 à 15% en poids et de préférence de 1 % à 10 % en poids, par rapport au poids total de la composition.

9. Composition selon l'une quelconque des revendications 1 à 8, **caractérisée par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

10. Composition selon la revendication 9, **caractérisée par le fait que** le dérivé de dibenzoylméthane est le 4-(ter.-butyl) 4'-méthoxy dibenzoylméthane.

11. Composition selon la revendication 9, **caractérisée par le fait que** le dérivé de dibenzoylméthane est le 4-isopropyl-dibenzoylméthane.

12. Composition selon l'une quelconque des revendications 1 à 11, où le dérivé du dibenzoylméthane est présent à des teneurs allant de 0,5 à 15% en poids et de préférence de 1 % à 10 % en poids, par rapport au poids total de la composition.

13. Composition l'une quelconque des revendications 1 à 12, où le composé 4,4-diarylbutadiène répond à la formule (VIII) suivante : 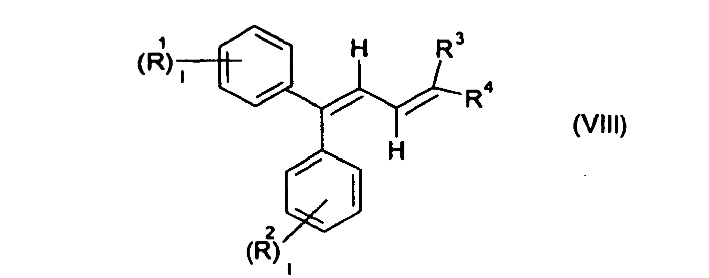 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₂₀, linéaire ou ramifié ; un radical alcényle en C₂- C₁₀ ; un radical alcoxy en C₁-C₁₂; un radical cycloalkyle en C₃-C₁₀ ; un radical cycloalcényle en C₃-C₁₀ ; un radical alcoxycarbonyle en C₁-C₂₀ linéaire ou ramifié ; un radical monoalkylamino en C₁-C₁₂, linéaire ou ramifié ; un radical dialkylamino en C₁-C₁₂, linéaire ou ramifié ; un aryle ; un hétéroaryle ou un substituant hydrosolubilisant choisi parmi un groupe carboxylate, un groupe sulfonate ou un reste ammonium ;
- R³ désigne un groupe COOR⁵; COR⁵; CONR⁵R⁶; CN ; un radical alkyle en C₁-C₂₀, linéaire ou ramifié ; un radical alcényle en C₂- C₁₀; un radical cycloalkyle en C₃-C₁₀ ; un radical bicycloalkyle en C₇-C₁₀ ; un radical cycloalcényle en C₃-C₁₀; un radical bicycloalcényle en C₇-C₁₀ ; un aryle en C₆-C₁₈ ; un hétéroaryle en C₃-C₇;
- R⁴ désigne un groupe COOR⁶ ; COR⁶ ; CONR⁵R⁶ ; CN ; un radical alkyle en C₁-C₂₀, linéaire ou ramifié ; un radical alcényle en C₂- C₁₀ ; un radical cycloalkyle en C₃-C₁₀; un radical bicycloalkyle en C₇-C₁₀; un radical cycloalcényle en C₃-C₁₀ ;; un radical bicycloalcényle en C₇-C₁₀ ; un aryle ; un hétéroaryle ;
- R⁵ et R⁶ identiques ou différents, désignent hydrogène ; [V]ₒ-R⁷, C₁-C₆-alkylène-SO₃U; C₁-C₆-alkylène-PO₃U ; C₁-C₆-alkylène-N(R⁸)₃⁺ D⁻ ; un radical alkyle en C₁-C₂₀, linéaire ou ramifié ; un radical alcényle en C₂-C₁₀ ; un radical cycloalkyle en C₃-C₁₀; un radical bicycloalkyle en C₇-C₁₀ ; un radical cycloalcényle en C₃-C₁₀ ; un radical bicycloalcényle en C₇-C₁₀ ; un aryle; un hétéroaryle ;
- V désigne un groupe -CH₂-CH₂-W-, -CH₂CH₂CH₂W-, -CH(CH₃)-CH₂-W-,
- CH₂-CH₂-CH₂-CH₂-W-, -CH₂-CH(CH₂CH₃)-W- ;
- D désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- W désigne O ou NH ;
- R⁷ et R identiques ou différents, désignent hydrogène, un radical alkyle en C₁-C₆, linéaire ou ramifié ; un radical alcényle en C₂- C_{6.} linéaire ou ramifié ; un radical acyle en C₁-C₆ linéaire ou ramifié ;
- R⁹ désigne hydrogène, un radical alkyle en C₁-C₆, linéaire ou ramifié ; un radical alcényle en C₂- C₆ ;
- 1 varie de 1 à 3 ;
- o varie de 0 à 150.

14. Composition selon la revendication 13, où le composé de formule (VIII) est choisi parmi ceux de formule (VIIIa) suivante : 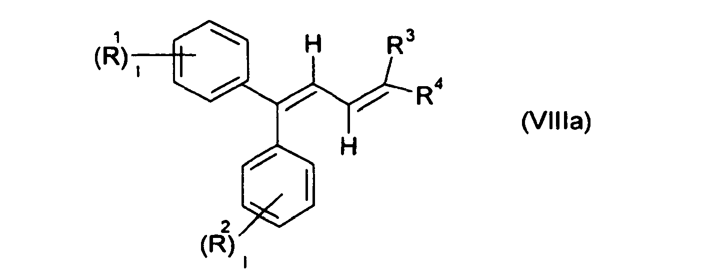 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈; un radical alcoxy en C₁-C₈; un substituant hydrosolubilisant choisi parmi un groupe carboxylate, un groupe sulfonate ou un reste ammonium ;
- R³ désigne un groupe COOR⁵ ; CONR⁵R⁶ ; CN ;
- R⁴ désigne un groupe COOR⁶ ; CONR⁵R⁶ ;
- R⁵ désigne hydrogène ; [V]ₒ-R⁷ ; C₁-C₆-alkylène-SO₃U ; C₁-C₆-alkylène-N(R⁸)₃⁺ D⁻
- R⁶ désigne [V]ₒ-R⁷ ; C₁-C₆-alkylène-SO₃U ; C₁-C₆-alkylène-N(R⁸)₃⁺ D' ;
- V désigne un groupe -CH₂-CH₂-O-, -CH₂CH₂CH₂O-, -CH(CH₃)-CH₂-O-,
- D désigne CI, Br, l, SO₄R⁹ ;
- U désigne hydrogène, Na⁺, K⁺, Mg²⁺, Ca²⁺, Li⁺, Al³⁺, -N(R⁸)₄⁺
- R⁷ , R⁸ et R⁹ identiques ou différents, désignent hydrogène, un radical alkyle en C₁-C₃, linéaire ou ramifié ;
- l varie de 1 à 3
- o varie de 0 à 50.

15. Composition selon la revendication 14, où le composé de formule (VIII) est choisi parmi ceux répondant à la formule (VIIIb) suivante : 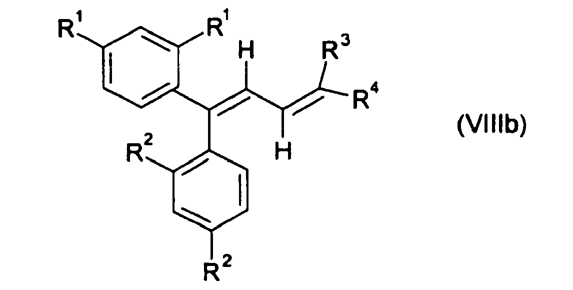 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈ ; un radical alcoxy en C₁-C₈ ;
- R³ désigne un groupe COOR⁵ ; CONR⁵R⁶ ; CN ;
- R⁴ désigne un groupe COOR⁶ ; CONR⁵R⁶;
- R⁵ désigne hydrogène : [V]ₒ-R⁷; C₁-C₆-alkylène-SO₃U; C₁-C₆-alkylène-N(R⁸)₃⁺D⁻
- R⁶ désigne [V]ₒ-R⁷; C₁-C₆-alkylène-SO₃U ; C₁-C₆-alkylène-N(R⁸)₃⁺ + D⁻ ;
- V désigne un groupe -CH₂-CH₂-O-, -CH₂CH₂CH₂O-, -CH(CH₃)-CH₂-O-,
- D désigne Cl, Br, I, SO₄R⁹ ;
- U désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C₃, linéaire ou ramifié ;
- o varie de 0 à 50.

16. Composition selon la revendication 15, où le composé de formule (VIII) est choisi parmi ceux répondant à la formule (VIIIc) suivante : 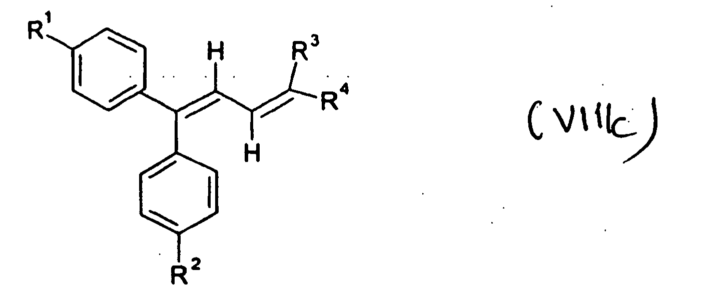 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₈; un radical alcoxy en C₁-C₈ :
- R³ désigne un groupe COOR⁵; CONR⁵R⁶ ; CN ;
- R⁴ désigne un groupe COOR⁶ ; CONR⁵R⁶ ;
- R⁵ désigne hydrogène R⁷ C₁-C₆-alkylène-SO₃Y :C₁-C₆-alkylène-N(R⁸)₃⁺D
- R⁶ désigne [V]ₒ-R⁷ ; C₁-C₆-alkylène-SO₃U ; C₁-C₆-alkylène-N(R⁸)₃⁺ D⁻ ;
- V désigne un groupe -CH₂-CH₂-O-, -CH₂CH₂CH₂O-, -CH(CH₃)=CH₂-O-,
- D désigne CI, Br, l, SO₄R⁹;
- U désigne hydrogène, Na⁺, K⁺, Mg²⁺, Ca²⁺, Li⁺, Al³⁺, -N(R⁸)₄⁺
- R⁷, R⁸ et R⁹ identiques ou différents, désignent hydrogène, un radical alkyle en C₁-C_{3.} linéaire ou ramifié :
- o varie de 0 à 50.

17. Composition selon l'une quelconque des revendications 13 à 16, où le composé de formule (VIII) est choisi parmi les composés suivants :

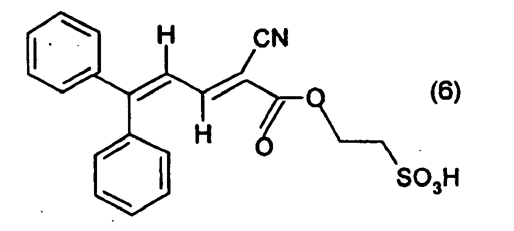

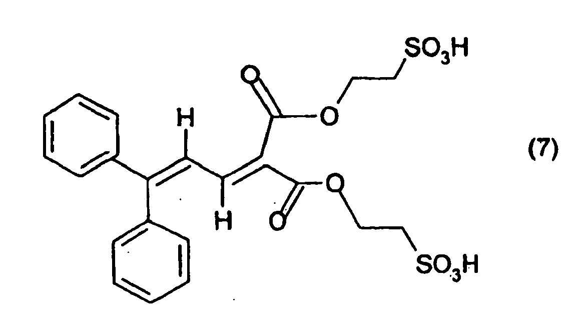

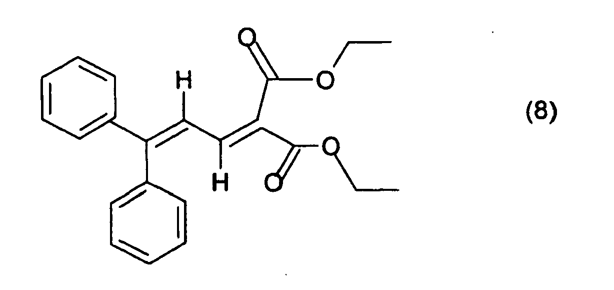

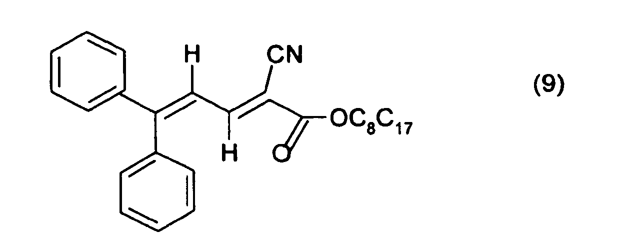

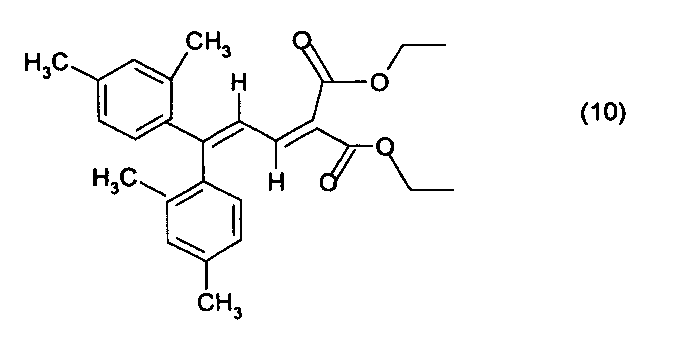

18. Composition selon l'une quelconque des revendication 1 à 12, où le composé 4,4-diarylbutadiène est un oligomère répondant à la formule (IX) suivante : 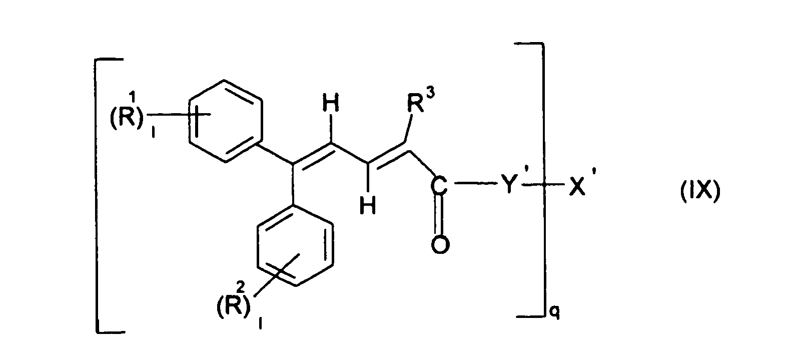 dans laquelle le système diène est de configuration Z,Z ; Z,E ; E,Z ou E,E ou des mélanges desdites configurations et où :
- R¹, R² R³ et l ont les mêmes significations indiquées dans la formule (VIII) dans la revendication 17 ;
- Y' désigne un groupe -0- ou -NR¹⁰-
- R¹⁰ désigne hydrogène ; un radical alkyle en C₁-C₂₀, linéaire ou ramifié ; un radical alcényle en C₂- C₁₀; un radical cycloalkyle en C₃-C₁₀ ; un radical bicycloalkyle en C₇-C₁₀ ; un radical cycloalcényle en C₃-C₁₀ ;; un radical bicycloalcényle en C₇-C₁₀ ; un aryle ; un hétéroaryle ;
- X' désigne un reste de polyol linéaire ou ramifié, aliphatique ou cycloaliphatique comprenant de 2 à 10 groupes hydroxy et de valence q ; la chaîne carbonée dudit reste pouvant être interrompue par un ou plusieurs atomes de souffre ou d'oxygène ; un ou plusieurs groupes imines ou un ou plusieurs alkylimino en C₁-C₄ ;
- q varie de 2 à 10.

19. Composition selon la revendication 18, où le composé de formule (IX) est choisi parmi ceux pour lesquels :
- R¹ et R², identiques ou différents, désignent hydrogène, un radical alkyle en C₁-C₁₂ ; un radical alcoxy en C₁-C₈ ; un substituant hydrosolubilisant choisi parmi un groupe carboxylate, un groupe sulfonate ou un reste ammonium ;
- R³ désigne un groupe COOR⁵; CONR⁵R⁶; CN ; un radical cycloalkyle en C₃-C₁₀; un radical bicycloalkyle en C₇-C₁₀;
- R⁵ et R⁶ , identiques ou différents, désignent un radical alkyle en C₁-C₂₀, linéaire ou ramifié ; un radical cycloalkyle en C₃-C₁₀; un radical bicycloalkyle en C₇-C₁₀; naphtyle ou phényle éventuellement substitué ;
- X' désigne un reste de polyol comprenant de 2 à 6 groupes hydroxy et plus particulièrement de 2 à 4.

20. Composition selon la revendication 19, où le composé de formule (IX) est choisi parmi ceux pour lesquels :
- X' désigne un reste d'éthanol ou de pentaérythrol.

21. Composition selon la revendication 20, où le composé de formule (IX) est choisi parmi les composés suivants :

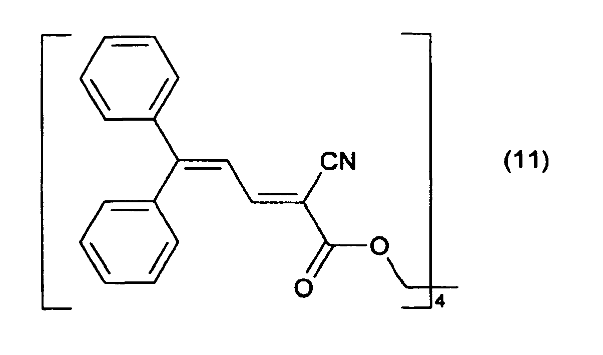

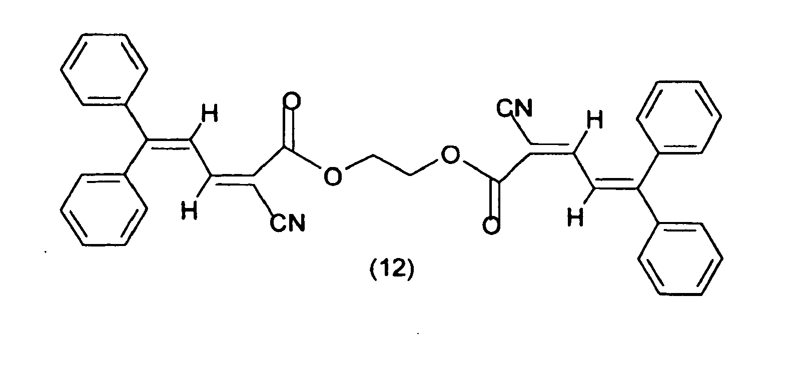

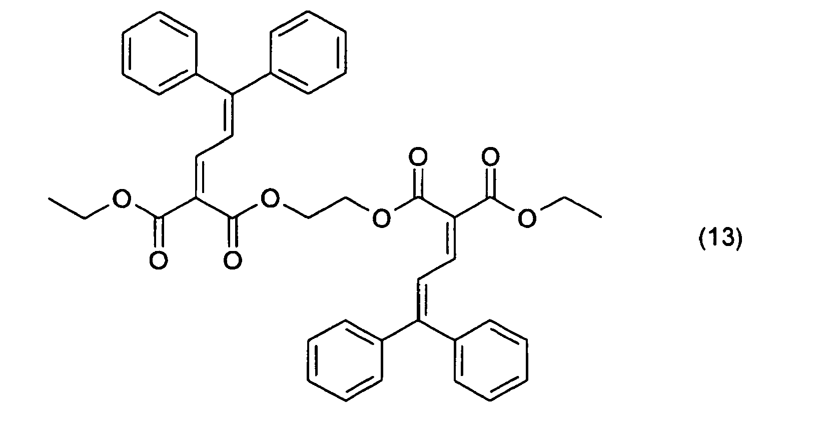

22. Composition selon l'une quelconque des revendications 1 à 21, **caractérisée par le fait qu'**elle contient en plus d'autres filtres organiques actifs dans l'UV-A et/ou l'UV-B.

23. Composition selon la revendication 22, où le ou les filtres UV organiques complémentaires sont choisis parmi les anthranilates; les dérivés salicyliques, les dérivés du camphre ; les dérivés de la benzophénone ; les dérivés de β,β'-diphénylacrylate, les dérivés de benzotriazole ; les dérivés de triazine non-photosensibles; les dérivés de benzalmalonate; les dérivés de benzimidazole ; les imadazolines; les dérivés bis-benzoazolyle ; les dérivés de l'acide p-aminobenzoïque (PABA); les dérivés de méthylène bis-(hydroxyphényl benzotriazole) ; les polymères filtres et silicones ; les dimères dérivés d'α-alkylstyrène.

24. Composition selon la revendication 23, **caractérisée par le fait que** le ou les filtres UV organiques sont choisis parmi les composés suivants :
- Ethylhexyl Salicylate,
- Octocrylene,
- Phenylbenzimidazole Sulfonic Acid,
- Benzophenone-3,
- Benzophenone-4,
- Benzophenone-5,
- 4-Methylbenzylidene camphor,
- Terephthalylidene Dicamphor Sulfonic Acid,
- Disodium Phenyl Dibenzimidazole Tetra-sulfonate,
- la 2,4,6-tris-(4'-amino benzalmalonate de düsobutyle)-s-triazine
- Anisotriazine,
- Methylène bis-Benzotriazolyl Tetramethylbutylphénol
- Drometrizole Trisiloxane
et leurs mélanges.

25. Composition selon l'une quelconque des revendications 1 à 24, **caractérisée par le fait qu'**elle comprend en outre, des pigments ou des nanopigments d'oxydes métalliques, enrobés ou non.

26. Composition selon la revendication 25, **caractérisée par le fait que** lesdits pigments ou nanopigments sont choisis parmi les oxydes de titane, de zinc, de fer, de zirconium, de cérium et leurs mélanges, enrobés ou non.

27. Composition selon l'une quelconque des revendications 1 à 26, **caractérisée par le fait qu'**elle comprend en outre au moins un agent de bronzage et/ou de brunissage artificiel de la peau.

28. Composition selon l'une quelconque des revendications 1 à 27, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les a-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants.

29. Composition selon l'une quelconque des revendications 1 à 28, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

30. Composition selon l'une quelconque des revendications 1 à 29, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

31. Composition selon l'une quelconque des revendications 1 à 29, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

32. Utilisation d'un composé 4;4-diarylbutadiène tel que défini dans les revendications précédentes dans des compositions cosmétiques contenant au moins un dérivé de dibenzoylméthane en association avec au moins un dérivé de 1,3,5-triazine photosensible tels que définis dans les revendications précédentes en vue d'améliorer dans lesdites compositions la stabilité au rayonnement UV (photostabilité) dudit dérivé de 1,3,5-triazine ; le rapport en poids du composé 4,4-diarylbutadiène sur le dérivé de dibenzoylméthane étant supérieur à 2,5 et lesdites compositions ne contenant pas de dérivé de cinnamate.

## Revendications (Revendications pour l'(les) Etat(s) contractant(s) suivant(s): DE)

1. Composition cosmétique ou dermatologique, à usage topique, pour la protection de l'épiderme humain contre les rayons UV, **caractérisée par le fait qu'**elle comprend, dans un support cosmétiquement acceptable :
(a) au moins un dérivé du dibenzoylméthane et
(b) au moins un dérivé de 1,3,5-triazine photosensible en présence d'un dérivé du dibenzoylméthane et
(c) au moins un composé 4,4-diarylbutadiène ; le rapport en poids du composé 4,4-diarylbutadiène sur le dérivé de dibenzoylméthane étant supérieur à 2,5 et ladite composition ne contenant pas de dérivé de cinnamate
et qu'elle se présente sous forme de dispersion vésiculaire non ionique ; sous forme d'émulsion huile/eau ; sous forme de gel ; sous forme de poudre ; sous forme de bâtonnet solide ; sous forme de mousse aérosol ou de spray.

2. Composition cosmétique ou dermatologique, à usage topique, pour la protection des cheveux contre les rayons UV, **caractérisée par le fait qu'**elle comprend, dans un support cosmétiquement acceptable :
(a) au moins un dérivé du dibenzoylméthane et
(b) au moins un dérivé de 1,3,5-triazine photosensible en présence d'un dérivé du dibenzoylméthane et
(c) au moins un composé 4,4-diarylbutadiène ; le rapport en poids du composé 4,4-diarylbutadiène sur le dérivé de dibenzoylméthane étant supérieur à 2,5 et ladite composition ne contenant pas de dérivé de cinnamate
et qu'elle se présente sous forme de shampooing, de gel ou de dispersion vésiculaire non ionique.

3. Composition de maquillage des cils, des sourcils ou de la peau, **caractérisée par le fait qu'**elle comprend, dans un support cosmétiquement acceptable :
(a) au moins un dérivé du dibenzoylméthane et
(b) au moins un dérivé de 1,3,5-triazine photosensible en présence d'un dérivé du dibenzoylméthane et
(c) au moins un composé 4,4-diarylbutadiène ; le rapport en poids du composé 4,4-diarylbutadiène sur le dérivé de dibenzoylméthane étant supérieur à 2,5 et ladite composition ne contenant pas de dérivé de cinnamate
et qu'elle se présente sous forme solide ou pâteuse, anhydre ou aqueuse sous forme d'émulsion huile/eau ou de dispersion vésiculaire non-ionique.

4. Composition selon l'une quelconque des revendications 1 à 3, où le dérivé de 1.3.5-triazine répond à la formule (I) suivante : 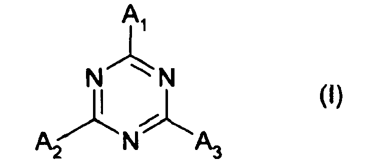 dans laquelle les radicaux A₁, A₂ et A₃, identiques ou différents sont choisis parmi les groupes de formules (11) : 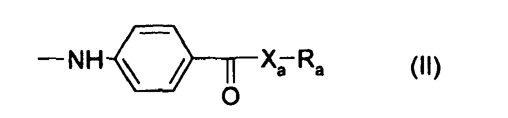 dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé; un radical de formule (III), (IV) ou (V) suivantes : 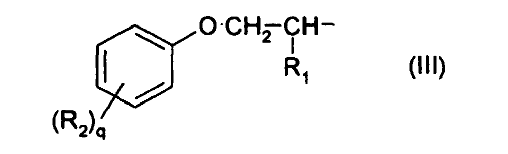 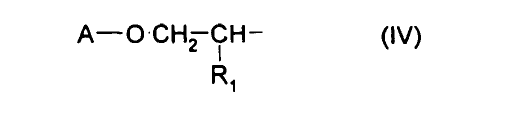 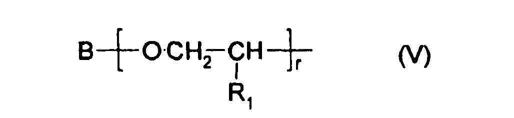
dans lesquelles:
- R₁ est l'hydrogène ou un radical méthyle;
- R₂ est un radical alkyle en C₁-C₉;
- q est un nombre entier allant de 0 à 3;
- r est un nombre entier allant de 1 à 10;
- A est un radical alkyle en C₄-C₈ ou un radical cycloalkyle en C₅-C₈;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.

5. Composition selon la revendication **4, caractérisée par le fait que** le dérivé de 1,3,5-triazine de formule (I) est choisi parmi ceux où les A₁, A₂ et A₃ sont de formule (II) et présentent les caractéristiques suivantes :
- un Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄ ;
- R₂ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

6. Composition selon la revendication **4, caractérisée par le fait que** le dérivé de 1,3,5-triazine est choisi parmi ceux où les A₁, A₂ et A₃ sont de formule (II) et présentant l'ensemble des caractéristiques suivantes :
- un ou deux Xₐ-Rₐ représente le radical -NH-R_{A} , avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (III), (IV) ou (V) ci-dessus dans lesquelles :
- B est un radical alkyle en C₁-C₄ ;
- R₂ est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (III), (IV) ou (V) ci-dessus dans lesquelles :
- B est un radical alkyle en C₁-C₄;
- R₂ est le radical méthyle.

7. Composition selon la revendication **6, caractérisée par le fait que** le dérivé de 1,3,5-triazine est celui répondant à la formule suivante : 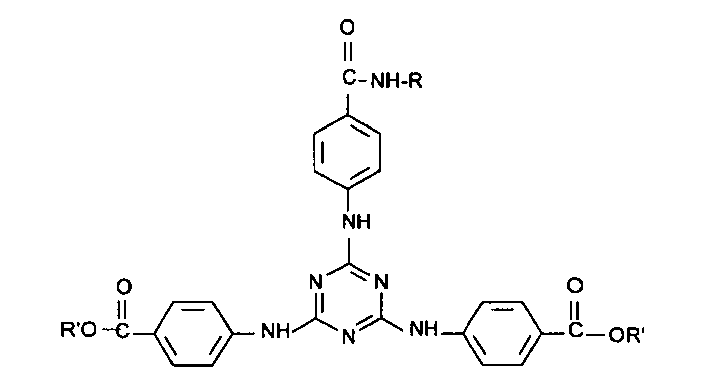 dans laquelle R' désigne un radical éthyl-2 hexyle et R désigne un radical tertio-butyle.

8. Composition selon la revendication **4**, **caractérisée par le fait que** le dérivé de 1,3,5-triazine répondant à la formule (1) est choisi parmi ceux où les A₁, A₂ et A₃ sont de formule (II) et présentent les caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

9. Composition selon la revendication **8**, **caractérisée par le fait que** le dérivé de 1,3,5-triazine est celui répondant à la formule suivante : 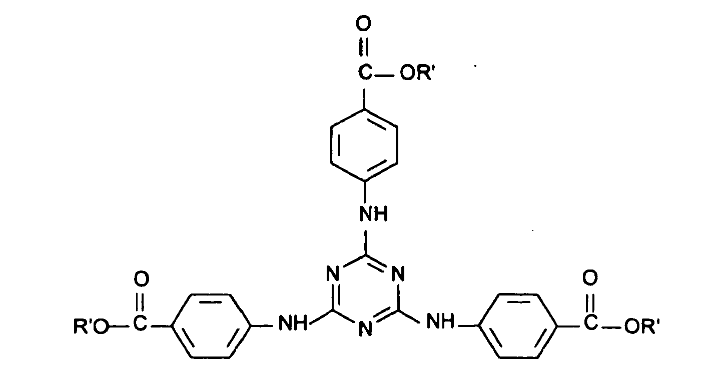 dans laquelle R' désigne un radical 2-éthyl hexyle.

10. Composition selon l'une quelconque des revendications **1** à **9**, **caractérisée par le fait que** le dérivé de 1,3,5-triazine est présent à des teneurs allant de 0,5 à 15% en poids et de préférence de 1 % à 10 % en poids, par rapport au poids total de la composition.

11. Composition selon l'une quelconque des revendications **1** à **10, caractérisée par le fait que** le dérivé de dibenzoylméthane est choisi parmi :
- le 2-méthyldibenzoylméthane
- le 4-méthyldibenzoylméthane
- le 4-isopropyldibénzoylméthane
- le 4-tert.-butyldibenzoylméthane
- le 2,4-diméthyldibenzoylméthane
- le 2 5-diméthyidibenzoylméthane
- le 4,4'-düsopropyldibenzoylméthane .
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoyméthane
- le 2,6-diméthyl-4-tert.-butyl-4'-méthoxydibenzoylméthane..

12. Composition selon la revendication **11**, **caractérisée par le fait que** le dérivé de dibenzoylméthane est le 4-(ter.-butyl) 4'-méthoxy dibenzoylméthane.

13. Composition selon la revendication **11 caractérisée par le fait que** le dérivé de dibenzoylméthane est le 4-isopropyl-dibenzoylméthane.

14. Composition selon l'une quelconque des revendications **1** à **13**, où le dérivé du dibenzoylméthane est présent à des teneurs allant de 0,5 à 15% en poids et de préférence de 1 % à 10 % en poids, par rapport au poids total de la composition.

15. Composition l'une quelconque des revendications **1** à **14**, où le composé 4,4-diarylbutadiène répond à la formule (VIII) suivante : 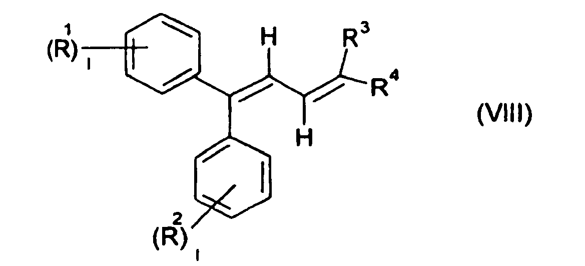 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₂₀, linéaire ou ramifié; un radical alcényle en C₂- C₁₀; un radical alcoxy en C₁-C₁₂; un radical cycloalkyle en C₃-C₁₀; un radical cycloalcényle en C₃-C₁₀; un radical alcoxycarbonyle en C₁-C₂₀ linéaire ou ramifié ; un radical monoalkylamino en C₁-C_{12,} linéaire ou ramifié ; un radical dialkylamino en C₁-C₁₂, linéaire ou ramifié ; un aryle ; un hétéroaryle ou un substituant hydrosolubilisant choisi parmi un groupe carboxylate, un groupe sulfonate ou un reste ammonium ;
- R³ désigne un groupe COOR⁵ ; COR⁵; CONR⁵R⁶; CN ; un radical alkyle en C₁-C₂₀, linéaire ou ramifié ; un radical alcényle en C₂- C₁₀ ; un radical cycloalkyle en C₃-C₁₀, un radical bicycloalkyle en C₇-C₁₀; un radical cycloalcényle en C₃-C₁₀ ; un radical bicycloalcényle en C₇-C₁₀ : un aryle en C₆-C₁₈ ; un hétéroaryle en C₃-C₇ ;
- R⁴ désigne un groupe COOR⁶ ; COR⁶; CONR⁵R⁶ ; CN ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle ; un hétéroaryle ;
- R⁵ et R⁶, identiques ou différents, désignent hydrogène ; [V]ₒ-R⁷, C₁-C₆-alkylène-SO₃U; C₁-C₆-alkylène-PO₃U ; C₁-C₆-alkylène-N(R⁸)₃⁺ D⁻ ; un radical alkyle en C₁-C₂₀, linéaire ou ramifié ; un radical alcényle en C₂-C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle ; un hétéroaryle ;
- V désigne un groupe -CH₂-CH₂-W-, -CH₂CH₂CH₂W-, -CH(CH₃)-CH₂-W-,
- CH₂-CH₂-CH₂-CH₂-W-, -CH₂-CH(CH₂CH₃)-W- ;
- D désigne CI, Br, I, SO₄R⁹ ;
- U désigne hydrogène, Na⁺, K⁺, Mg²⁺, Ca²⁺, Li⁺, Al³⁺, -N(R⁸)₄⁺
- W désigne O ou NH ;
- R⁷ et R⁸ identiques ou différents, désignent hydrogène, un radical alkyle en C₁-C₆, linéaire ou ramifié ; un radical alcényle en C₂- C₆, linéaire ou ramifié ; un radical acyle en C₁-C₆ linéaire ou ramifié ;
- R⁹ désigne hydrogène, un radical alkyle en C₁-C₆, linéaire ou ramifié ; un radical alcényle en C₂- C₆;
- I varie de 1 à 3;
- o varie de 0 à 150.

16. Composition selon la revendication **15**, où le composé de formule (VIII) est choisi parmi ceux de formule (VIIIa) suivante : 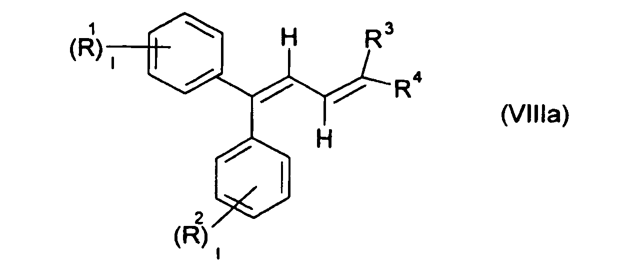 dans laquelle le système diène est de configuration Z,Z ; Z,E ; E,Z ou E,E ou des mélanges desdites configurations et où :
- R¹ et R ² identiques ou différents, désignent hydrogène, un radical alkyle en C₁-C₈; un radical alcoxy en C₁-C₈; un substituant hydrosolubilisant choisi parmi un groupe carboxylate, un groupe sulfonate ou un reste ammonium ;
- R³ désigne un groupe COOR⁵ ; CONR⁵R⁶ ; CN ;
- R⁴ désigne un groupe COOR⁶ ; CONR⁵R⁶ ;
- R⁵ désigne hydrogène : [V]ₒ-R⁷ ; C₁-C₆-alkylène-SO₃U; C₁-C₆-alkylène-N(R⁸)₃⁺ D⁻
- R⁶ désigne [V]ₒ-R⁷ ; C₁-C₆-alkylène-SO₃U ; C₁-C₆-alkylène-N(R⁸)3⁺ D- ;
- V désigne un groupe -CH₂-CH₂-O-, -CH₂CH₂CH₂O-, -CH(CH₃)-CH₂-O-,
- D désigne CI, Br, 1, SO₄R⁹ ;
- U désigne hydrogène, Na⁺, K⁺, Mg²⁺, Ca²⁺, Li⁺, Al³⁺, -N(R⁸)₄⁺
- R⁷, R⁸et R⁹ identiques ou différents, désignent hydrogène, un radical alkyle en C₁-C_{3.} linéaire ou ramifié ;
- I varie de 1 à 3
- o varie de 0 à 50.

17. Composition selon la revendication **16**, où le composé de formule (VIII) est choisi parmi ceux répondant à la formule (VIIIb) suivante : 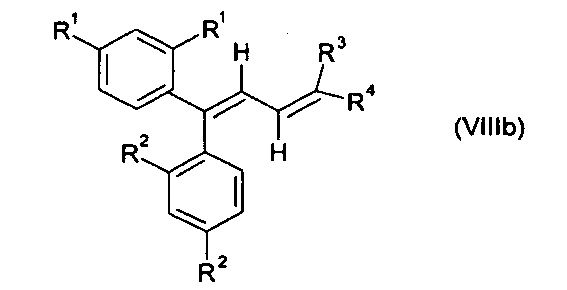 dans laquelle le système diène est de configuration Z,Z ; Z,E ; E,Z ou E,E ou des mélanges desdites configurations et où :
- R¹ et R² identiques ou différents, désignent hydrogène, un radical alkyle en C₁-C₈; un radical alcoxy en C₁-C₈:
- R³ désigne un groupe COOR⁵ CONR⁵R⁶ ; CN ;
- R⁴ désigne un groupe COOR⁶ ; CONR⁵R⁶ ;
- R⁵ désigne hydrogène ; [V]ₒ-R⁷; C₁-C₆-alkylène-SO₃U;C₁-C₆-alkylène-N(R⁸)₃⁺D⁻
- R⁶ désigne [V]ₒ-R⁷ ; C₁-C₆-alkylène-SO₃U ; C₁-C₆-alkylène-N(R⁸)₃⁺ D⁻ ;
- V désigne un groupe -CH₂-CH₂-O-, -CH₂CH₂CH₂O-, -CH(CH₃)-CH₂-O-,
- D désigne CI, Br, I, SO₄R⁹ ;
- U désigne hydrogène, Na⁺, K⁺, Mg²⁺, Ca²⁺, Li⁺ Al³⁺, -N(R⁸)₄⁺
- R⁷, R⁸ et R⁹ identiques ou différents, désignent hydrogène, un radical alkyle en C₁-C₃. linéaire ou ramifié;
- o varie de 0 à 50.

18. Composition selon la revendication **17,** où le composé de formule (VIII) est choisi parmi ceux répondant à la formule (VIIIc) suivante : 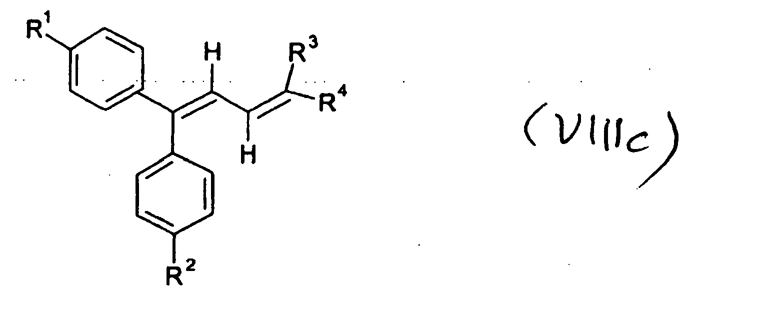 dans laquelle le système diène est de configuration Z,Z ; Z,E; E,Z ou E,E ou des mélanges desdites configurations et où:
- R¹ et R² identiques ou différents, désignent hydrogène, un radical alkyle en C₁-C₈; un radical alcoxy en C₁-C₈;
- R³ désigne un groupe COOR⁵; CONR⁵R⁶; CN;
- R⁴ désigne un groupe COOR⁶; CONR⁵R⁶;
- R⁵ désigne hydrogène, [V]ₒ-R⁷ ;C₁-C₆-alkylène-SO₃Y; C₁-C₆-alkylène-N(R⁸)₃⁺D⁻
- R⁶ désigne [V]ₒ-R⁷; C₁-C₆-alkylène-SO₃U; C₁-C₆-alkylène-N(R⁸)₃⁺ D⁻;
- V désigne un groupe -CH₂-CH₂-O-, -CH₂CH₂CH₂O-, -CH(CH₃)-CH₂-O-,
- D désigne CI, Br, I, SO₄R⁹;
- U désigne hydrogène, Na⁺, K⁺, Mg²⁺, Ca²⁺, Li⁺, Al³⁺, -N(R⁸)₄⁺
- R⁷ , R⁸ et R⁹ identiques ou différents, désignent hydrogène, un radical alkyle en C₁-C₃, linéaire ou ramifié ;
- o varie de 0 à 50.

19. Composition selon l'une quelconque des revendications **15 à 17**, où le composé de formule (VIII) est choisi parmi les composés suivants :

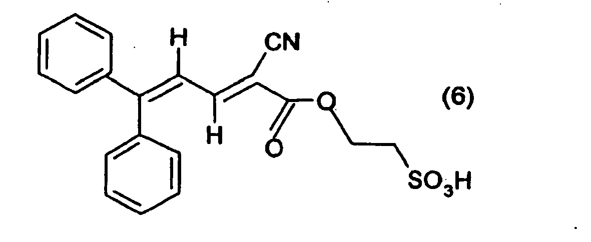

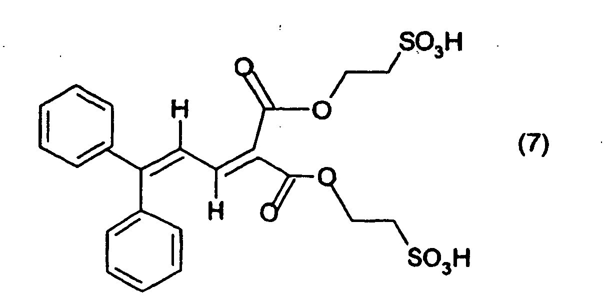

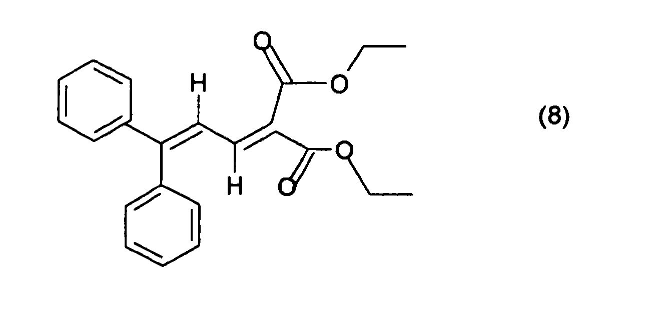

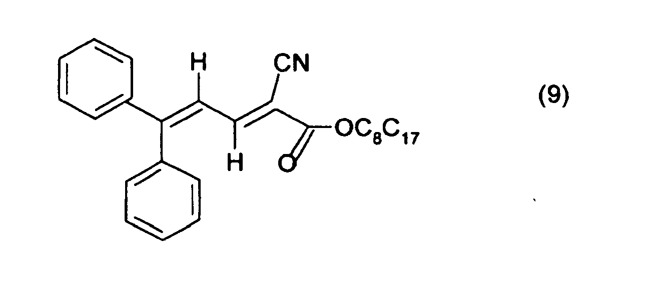

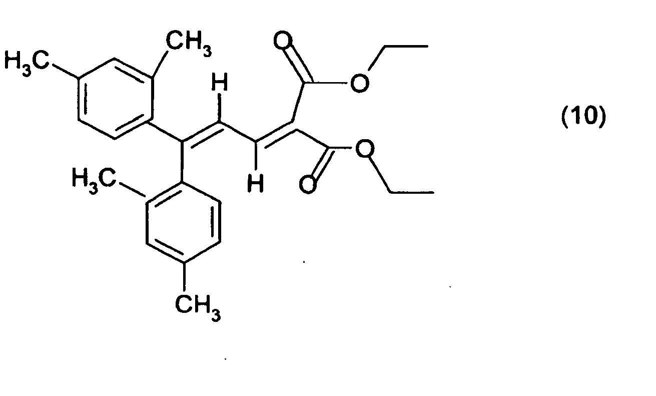

20. Composition selon l'une quelconque des revendication **1 à 14,** où le composé 4,4-diarylbutadiène est un oligomère répondant à la formule (IX) suivante : 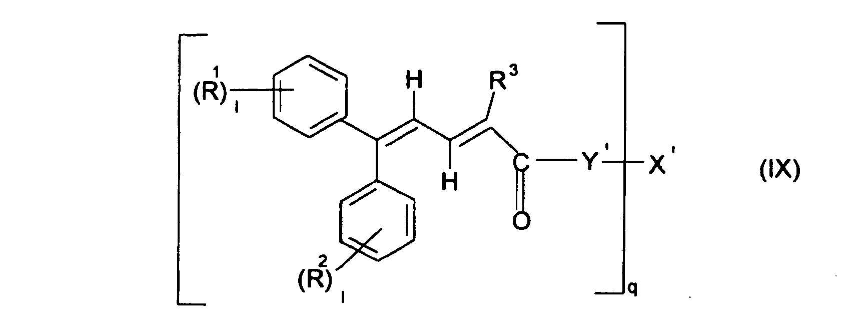 dans laquelle le système diène est de configuration Z,Z; Z,E ; E,Z ou E,E ou des mélanges desdites configurations et où:
- R¹, R² R et 1 ont les mêmes significations indiquées dans la formule (VIII) dans la revendication 17 ;
- Y' désigne un groupe -O- ou -NR¹⁰-
- R¹⁰ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle ; un hétéroaryle ;
- X' désigne un reste de polyol linéaire ou ramifié, aliphatique ou cycloaliphatique comprenant de 2 à 10 groupes hydroxy et de valence q ; la chaîne carbonée dudit reste pouvant être interrompue par un ou plusieurs atomes de souffre ou d'oxygène ; un ou plusieurs groupes imines ou un ou plusieurs alkylimino en C₁-C₄ ;
- q varie de 2 à 10.

21. Composition selon la revendication **20,** où le composé de formule (IX) est choisi parmi ceux pour lesquels :
- R¹ et R², identiques ou différents, désignent hydrogène, un radical alkyle en C₁-C₁₂ ; un radical alcoxy en C₁-C₈; un substituant hydrosolubilisant choisi parmi un groupe carboxylate, un groupe sulfonate ou un reste ammonium ;
- R³ désigne un groupe COOR⁵ ; CONR⁵R⁶ ; CN ; un radical cycloalkyle en C₃-C₁₀ ; un radical bicycloalkyle en C₇-C₁₀ ;
- R⁵ et R⁶, identiques ou différents, désignent un radical alkyle en C₁-C₂₀, linéaire ou ramifié ; un radical cycloalkyle en C₃-C₁₀; un radical bicycloalkyle en C₇-C₁₀ ; naphtyle ou phényle éventuellement substitué ;
- X' désigne un reste de polyol comprenant de 2 à 6 groupes hydroxy et plus particulièrement de 2 à 4.

22. Composition selon la revendication **21,** où le composé de formule (IX) est choisi parmi ceux pour lesquels :
- X' désigne un reste d'éthanol ou de pentaérythrol.

23. Composition selon la revendication **22,** où le composé de formule (IX) est choisi parmi les composés suivants :

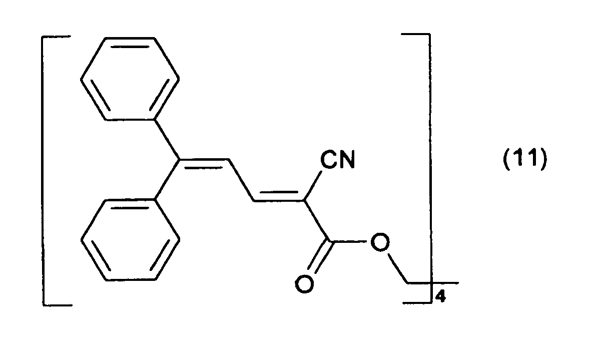

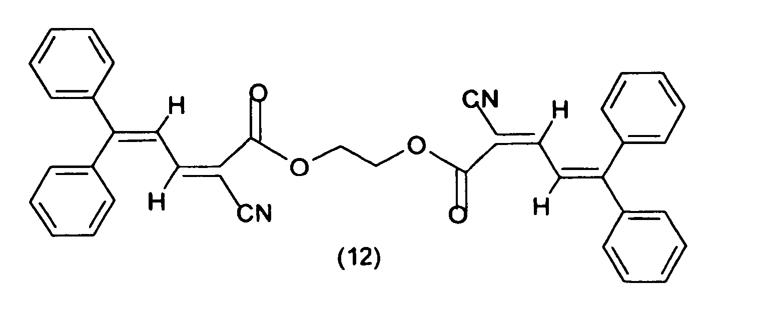

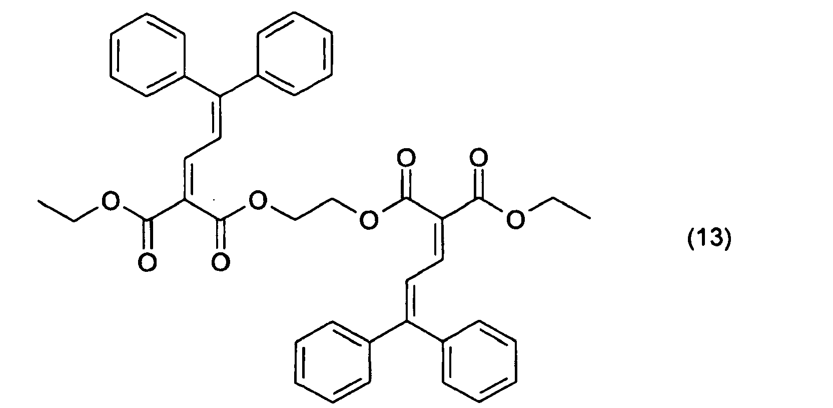

24. Composition selon l'une quelconque des revendications 1 à **23, caractérisée par le fait qu'**elle contient en plus d'autres filtres organiques actifs dans l'UV-A et/ou l'UV-B.

25. Composition selon la revendication **24,** où le ou les filtres UV organiques complémentaires sont choisis parmi les anthranilates ; les dérivés salicyliques, les dérivés du camphre ; les dérivés de la benzophénone ; les dérivés de β,β'-diphénylacrylate, les dérivés de benzotriazole ; les dérivés de triazine non-photosensibles ; les dérivés de benzalmalonate ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones ; les dimères dérivés d'α-alkylstyrène .

26. Composition selon la revendication **25, caractérisée par le fait que** le ou les filtres UV organiques sont choisis parmi les composés suivants :
- Ethylhexyl Salicylate,
- Octocrylene,
- Phenylbenzimidazole Sulfonic Acid,
- Benzophenone-3,
- Benzophenone-4,
- Benzophenone-5,
- 4-Methylbenzylidene camphor,
- Terephthalylidene Dicamphor Sulfonic Acid,
- Disodium Phenyl Dibenzimidazole Tetra-sulfonate,
- la 2,4,6-tris-(4'-amino benzalmalonate de diisobutyle)-s-triazine
- Anisotriazine,
- Methylène bis-Benzotriazolyl Tetramethylbutylphénol
- Drometrizole Trisiloxane
et leurs mélanges.

27. Composition selon l'une quelconque des revendications 1 à **26, caractérisée par le fait qu'**elle comprend en outre, des pigments ou des nanopigments d'oxydes métalliques, enrobés ou non.

28. Composition selon la revendication **27, caractérisée par le fait que** lesdits pigments ou nanopigments sont choisis parmi les oxydes de titane, de zinc, de fer, de zirconium, de cérium et leurs mélanges, enrobés ou non.

29. Composition selon l'une quelconque des revendications 1 à **28, caractérisée par le fait qu'**elle comprend en outre au moins un agent de bronzage et/ou de brunissage artificiel de la peau.

30. Composition selon l'une quelconque des revendications 1 à **29, caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants.

31. Utilisation d'un composé 4;4-diarylbutadiène tel que défini dans les revendications précédentes dans des compositions cosmétiques contenant au moins un dérivé de dibenzoylméthane en association avec au moins un dérivé de 1,3,5-triazine photosensible tels que définis dans les revendications précédentes en vue d'améliorer dans lesdites compositions la stabilité au rayonnement UV (photostabilité) dudit dérivé de 1,3,5-triazine ; le rapport en poids du composé 4,4-diarylbutadiène sur le dérivé de dibenzoylméthane étant supérieur à 2,5 et lesdites compositions ne contenant pas de dérivé de cinnamate.

## Claims (Claims for the following Contracting State(s): AT, BE, CH, CY, DK, ES, FI, FR, GB, GR, IE, IT, LI, LU, MC, NL, PT, SE, TR)

1. Cosmetic or dermatological composition for topical use, in particular for photoprotecting the skin and the hair, **characterized in that** it comprises, in a cosmetically acceptable support:
(a) at least one dibenzoylmethane derivative, and
(b) at least one 1,3,5-triazine derivative that is photosensitive in the presence of a dibenzoylmethane derivative, and
(c) at least one 4,4-diarylbutadiene compound; the weight ratio of the 4,4-diarylbutadiene compound to the dibenzoylmethane derivative being greater than 2.5 and the said composition containing no cinnamate derivative.

2. Composition according to Claim 1, in which the 1,3,5-triazine derivative corresponds to formula (I) below: 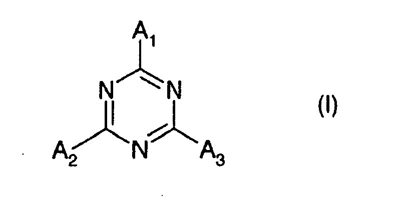 in which the radicals A₁, A₂ and A₃, which may be identical or different, are chosen from the groups of formula (II): 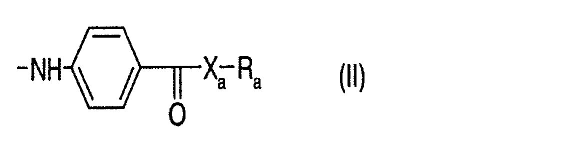 in which:
- Xₐ, which may be identical or different, represent oxygen or a radical -NH-;
- Rₐ, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a polyoxyethylenated radical comprising from 1 to 6 ethylene oxide units, the terminal OH group of which is methylated; a radical of formula (III), (IV) or (V) below: 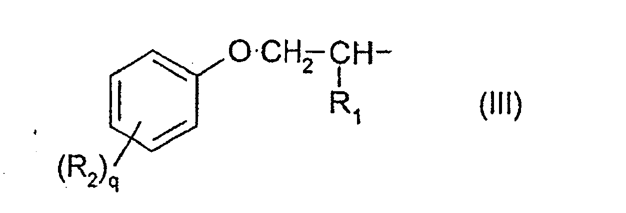 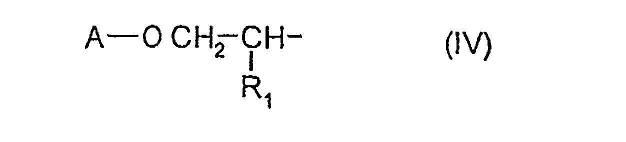 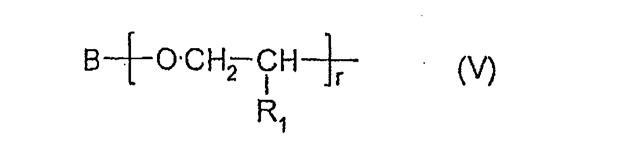
in which:
- R₁ is hydrogen or a methyl radical;
- R₂ is a C₁-C₉ alkyl radical;
- q is an integer ranging from 0 to 3;
- r is an integer ranging from 1 to 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals.

3. Composition according to Claim 2, **characterized in that** the 1,3,5-triazine derivative of formula (I) is chosen from those in which A₁, A₂ and A₃ are of formula (II) and have the following characteristics:
- one of the groups Xₐ-Rₐ represents a radical - NH-Rₐ with Rₐ chosen from: a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical;
- the other two groups Xₐ-Rₐ represent a radical
- 0-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical.

4. Composition according to Claim 2, **characterized in that** the 1,3,5-triazine derivative is chosen from those in which A₁, A₂ and A₃ are of formula (II) and have all of the following characteristics:
- one or two groups Xₐ-Rₐ represent a radical - NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical; the other or the other two group (s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical.

5. Composition according to Claim 4, **characterized in that** the 1,3,5-triazine derivative is that corresponding to the following formula: 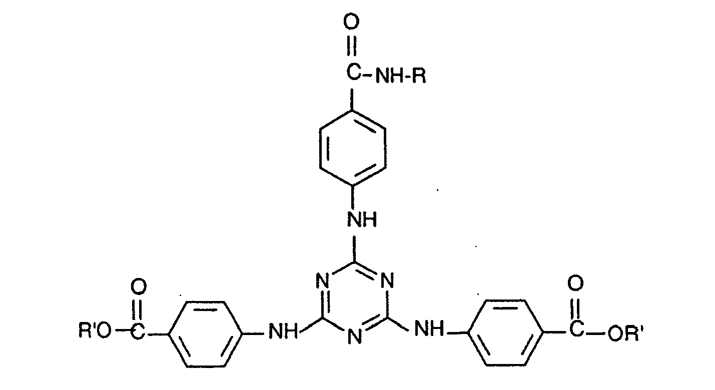 in which R' denotes a 2-ethylhexyl radical and R denotes a tert-butyl radical.

6. Composition according to Claim 2, **characterized in that** the 1,3,5-triazine derivative corresponding to formula (I) is chosen from those in which A₁, A₂ and A₃ are of formula (II) and have the following characteristics:
- Xₐ are identical and represent oxygen;
- Rₐ, which may be identical or different, represent a C₆-C₁₂ alkyl radical or a polyoxyethylenated radical comprising from 1 to 6 ethylene oxide units and in which the terminal OH group is methylated.

7. Composition according to Claim 6, **characterized in that** the 1,3,5-triazine derivative is that corresponding to the following formula: 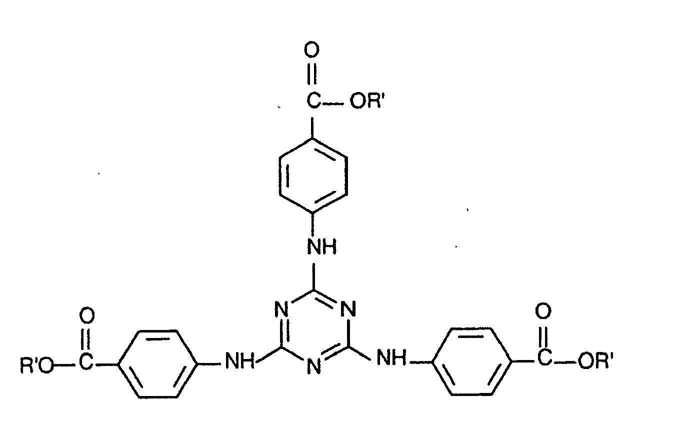 in which R' denotes a 2-ethylhexyl radical.

8. Composition according to any one of Claims 1 to 7, **characterized in that** the 1,3,5-triazine derivative is present in contents ranging from 0.5% to 15% by weight and preferably from 1% to 10% by weight, relative to the total weight of the composition.

9. Composition according to any one of Claims 1 to 8, **characterized in that** the dibenzoylmethane derivative is chosen from:
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4,4'-dimethoxydibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

10. Composition according to Claim 9, **characterized in that** the dibenzoylmethane derivative is 4-tert-butyl-4'-methoxydibenzoylmethane.

11. Composition according to Claim 9, **characterized in that** the dibenzoylmethane derivative is 4-isopropyldibenzoylmethane.

12. Composition according to any one of Claims 1 to 11, in which the dibenzoylmethane derivative is present in contents ranging from 0.5% to 15% by weight and preferably from 1% to 10% by weight, relative to the total weight of the composition.

13. Composition according to any one of Claims 1 to 12, in which the 4,4-diarylbutadiene compound corresponds to formula (VIII) below: 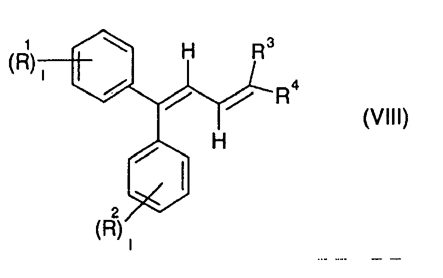 in which the diene system is of Z,Z; Z,E; E,Z or E,E configuration or mixtures of the said configurations, and in which:
- R¹ and R², which may be identical or different, denote hydrogen, a linear or branched C₁-C₂₀ alkyl radical; a C₂-C₁₀ alkenyl radical; a C₁-C₁₂ alkoxy radical; a C₃-C₁₀ cycloalkyl radical; a C₃-C₁₀ cycloalkenyl radical; a linear or branched C₁-C₂₀ alkoxycarbonyl radical; a linear or branched C₁-C₁₂ monoalkylamino radical; a linear or branched C₁-C₁₂ dialkylamino radical; an aryl; a heteroaryl or a hydro-solubilizing substituent chosen from a carboxylate group, a sulfonate group and an ammonium residue;
- R³ denotes a group COOR⁵; COR⁵; CONR⁵R⁶; CN; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; a C₆-C₁₈ aryl; a C₃-C₇ heteroaryl;
- R⁴ denotes a group COOR⁶; COR⁶; CONR⁵R⁶; CN; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- R⁵ and R⁶, which may be identical or different, denote hydrogen; [V]ₒ-R⁷; C₁-C₆-alkylene-SO₃U; C₁-C₆-alkylene-PO₃U; C₁-C₆-alkylene-N(R⁸)₃⁺ D⁻; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- V denotes a group -CH₂-CH₂-W-, -CH₂CH₂CH₂W-,
- CH(CH₃)-CH₂-W-, -CH₂-CH₂-CH₂-CH₂-W- or -CH₂-CH(CH₂CH₃)-W-;
- D denotes Cl, Br, I or SO₄R⁹;
- U denotes hydrogen, Na⁺, K⁺, Mg²⁺, Ca²⁺, Li⁺, Al³⁺ or -N(R⁸)₄⁺;
- W denotes 0 or NH;
- R⁷ and R⁸, which may be identical or different, denote hydrogen, a linear or branched C₁-C₆ alkyl radical; a linear or branched C₂-C₆ alkenyl radical; a linear or branched C₁-C₆ acyl radical;
- R⁹ denotes hydrogen, a linear or branched C₁-C₆ alkyl radical; a C₂-C₆ alkenyl radical;
- I ranges from 1 to 3;
- o ranges from 0 to 150.

14. Composition according to Claim 13, in which the compound of formula (VIII) is chosen from those of formula (VIIIa) below: 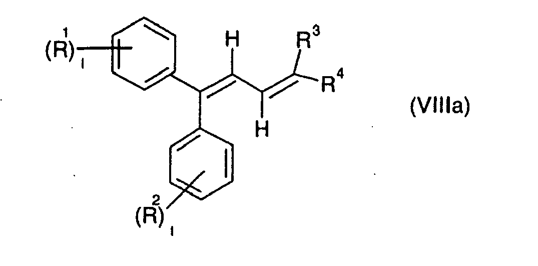 in which the diene system is of Z,Z; Z,E; E,Z or E,E configuration or mixtures of the said configurations, and in which:
- R¹ and R², which may be identical or different, denote hydrogen, a C₁-C₈ alkyl radical; a C₁-C₈ alkoxy radical; a hydro-solubilizing substituent chosen from a carboxylate group, a sulfonate group and an ammonium residue;
- R³ denotes a group COOR⁵; CONR⁵R⁶; CN;
- R⁴ denotes a group COOR⁶; CONR⁵R⁶;
- R⁵ denotes hydrogen; [V]ₒ-R⁷; C₁-C₆-alkylene-SO₃U; C₁-C₆-alkylene-N(R⁸)₃⁺ D⁻;
- R⁶ denotes [V]ₒ-R⁷; C₁-C₆-alkylene-SO₃U; C₁-C₆-alkylene-N (R⁸)₃⁺ D⁻;
- V denotes a -CH₂-CH₂-O-, -CH₂CH₂CH₂O-, -CH (CH₃) - CH₂-O- group;
- D denotes Cl, Br, I or SO₄R⁹;
- U denotes hydrogen, Na⁺, K⁺, Mg²⁺, Ca²⁺, Li⁺, Al³⁺ or -N (R⁸)₄⁺;
- R⁷, R⁸ and R⁹, which may be identical or different, denote hydrogen or a linear or branched C₁-C₃ alkyl radical;
- I ranges from 1 to 3;
- o ranges from 0 to 50.

15. Composition according to Claim 14, in which the compound of formula (VIII) is chosen from those corresponding to formula (VIIIb) below: 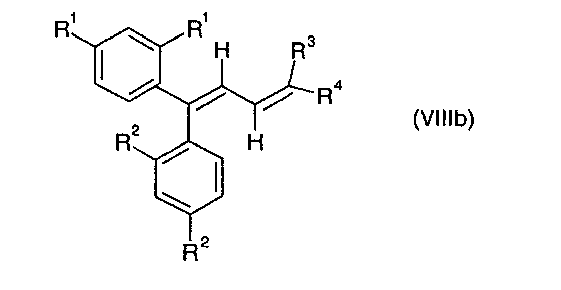 in which the diene system is of Z,Z; Z,E; E,Z or E,E configuration or mixtures of the said configurations, and in which:
- R¹ and R², which may be identical or different, denote hydrogen, a C₁-C₈ alkyl radical; a C₁-C₈ alkoxy radical;
- R³ denotes a group COOR⁵; CONR⁵R⁶; CN;
- R⁴ denotes a group COOR⁶; CONR⁵R⁶;
- R⁵ denotes hydrogen; [V]ₒ-R⁷; C₁-C₆-alkylene-SO₃U; C₁-C₆-alkylene-N (R⁸) ₃⁺ D⁻;
- R⁶ denotes [V]ₒ-R⁷; C₁-C₆-alkylene-SO₃U; C₁-C₆-alkylene-N(R⁸)₃⁺ D⁻;
- V denotes a -CH₂-CH₂-O-, -CH₂CH₂CH₂O-, -CH(CH₃)-CH₂-O- group;
- D denotes Cl, Br, I or SO₄R⁹;
- U denotes hydrogen, Na⁺, K⁺, Mg²⁺, Ca²⁺, Li⁺, Al³⁺ or -N(R⁸)₄⁺;
- R⁷, R⁸ and R⁹, which may be identical or different, denote hydrogen or a linear or branched C₁-C₃ alkyl radical;
- o ranges from 0 to 50.

16. Composition according to Claim 15, in which the compound of formula (VIII) is chosen from those corresponding to formula (VIIIc) below: 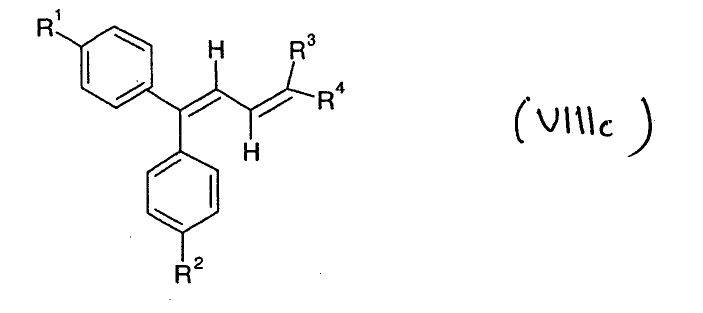 in which the diene system is of Z,Z; Z,E; E,Z or E,E configuration or mixtures of the said configurations, and in which:
- R¹ and R², which may be identical or different, denote hydrogen, a C₁-C₈ alkyl radical; a C₁-C₈ alkoxy radical;
- R³ denotes a group COOR⁵; CONR⁵R⁶; CN;
- R⁴ denotes a group COOR⁶ ; CONR⁵R⁶ ;
- R⁵ denotes hydrogen; [V]ₒ-R⁷; C₁-C₆-alkylene-S0₃Y; C₁-C₆-alkylene-N (R⁸)₃⁺ D⁻;
- R⁶ denotes [V]ₒ-R⁷; C₁-C₆-alkylene-SO₃U ; C₁-C₆-alkylene-N (R⁸)₃⁺ D⁻;
- V denotes a -CH₂-CH₂-O-, -CH₂CH₂CH₂O-, -CH (CH₃) - CH₂-O- group;
- D denotes Cl, Br, I or SO₄R⁹;
- U denotes hydrogen, Na⁺, K⁺, Mg²⁺, Ca²⁺, Li⁺, Al³⁺ or -N(R⁸)₄⁺;
- R⁷, R⁸ and R⁹, which may be identical or different, denote hydrogen or a linear or branched C₁-C₃ alkyl radical;
- o ranges from 0 to 50.

17. Composition according to any one of Claims 13 to 16, in which the compound of formula (VIII) is chosen from the following compounds:

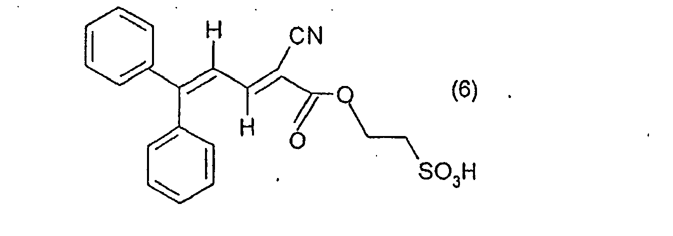

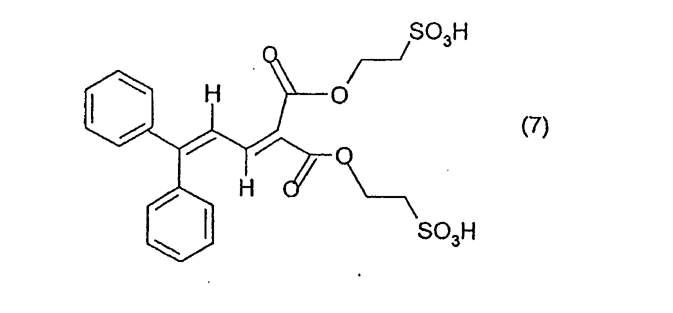

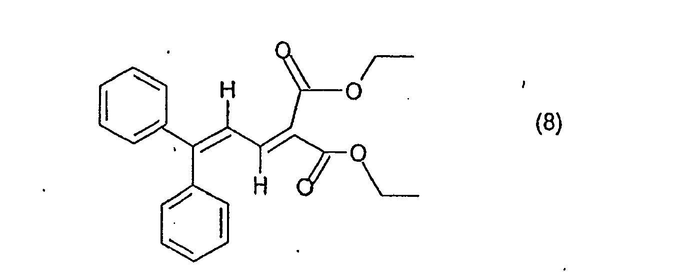

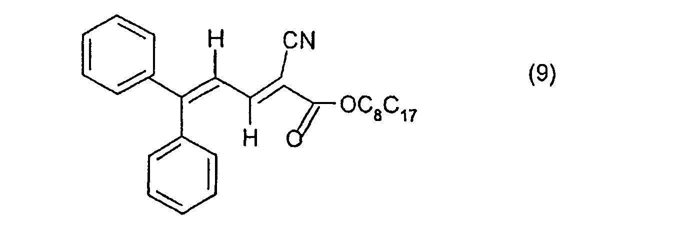

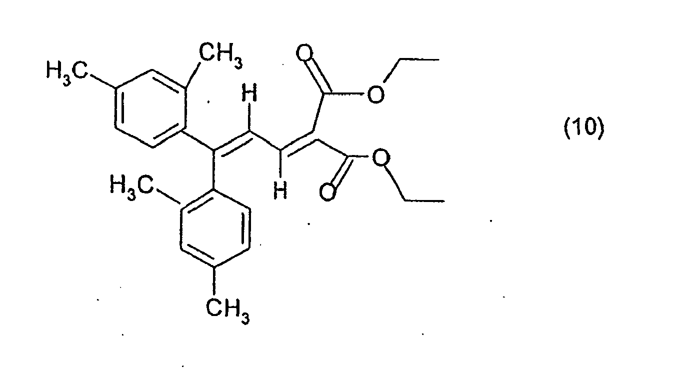

18. Composition according to any one of Claims 1 to 12, in which the 4,4-diarylbutadiene compound is an oligomer corresponding to formula (IX) below: 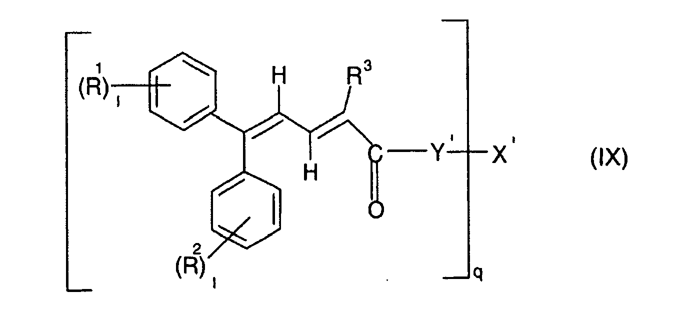 in which the diene system is of Z,Z; Z,E; E,Z or E,E configuration or mixtures of the said configurations, and in which:
- R¹, R², R³ and I have the same definitions as indicated in formula (VIII) in Claim 17;
- Y' denotes a group -O- or -NR¹⁰-;
- R¹⁰ denotes hydrogen; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- X' denotes an aliphatic or cycloaliphatic linear or branched polyol residue comprising from 2 to 10 hydroxyl groups and of valency q; the carbon-based chain of the said residue possibly being interrupted with one or more sulfur or oxygen atoms; one or more imine groups; or one or more C₁-C₄ alkylimino groups;
- q ranges from 2 to 10.

19. Composition according to Claim 18, in which the compound of formula (IX) is chosen from those for which:
- R¹ and R², which may be identical or different, denote hydrogen, a C₁-C₁₂ alkyl radical; a C₁-C₈ alkoxy radical; a hydro-solubilizing substituent chosen from a carboxylate group, a sulfonate group or an ammonium residue;
- R³ denotes a group COOR⁵; CONR⁵R⁶; CN; a C₃-C₁₀ cycloalkyl radical; a C₇-C₁₀ bicycloalkyl radical;
- R⁵ and R⁶, which may be identical or different, denote a linear or branched C₁-C₂₀ alkyl radical; a C₃-C₁₀ cycloalkyl radical; a C₇-C₁₀ bicycloalkyl radical; optionally substituted naphthyl or phenyl;
- X' denotes a polyol residue comprising from 2 to 6 and more particularly from 2 to 4 hydroxyl groups.

20. Composition according to Claim 19, in which the compound of formula (IX) is chosen from those for which:
- X' denotes an ethanol or pentaerythrol residue.

21. Composition according to Claim 20, in which the compound of formula (IX) is chosen from the following compounds:

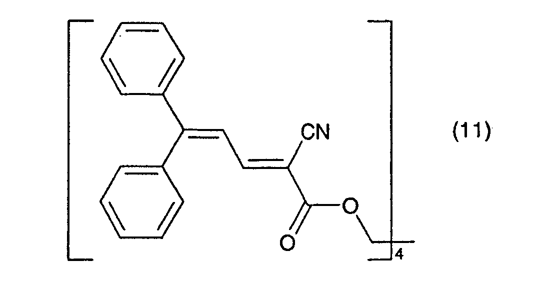

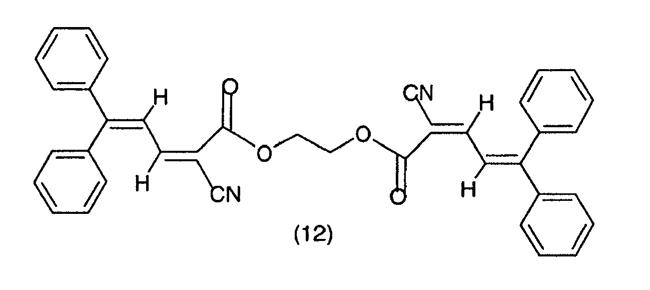

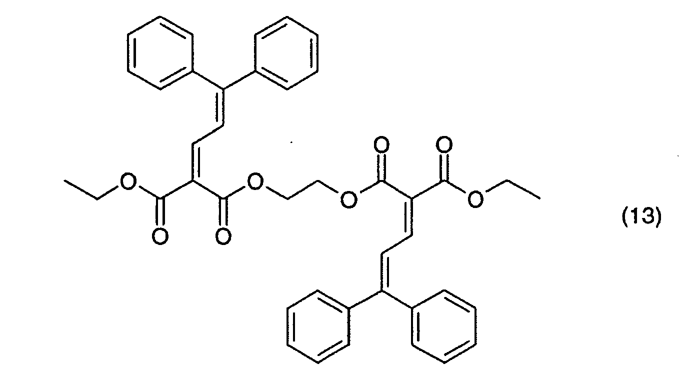

22. Composition according to any one of Claims 1 to 21, **characterized in that** it also contains other UV-A-active and/or UV-B-active organic screening agents.

23. Composition according to Claim 22, in which the additional organic UV-screening agent(s) is (are) chosen from anthranilates; salicylic derivatives; camphor derivatives; benzophenone derivatives; β,β'-diphenylacrylate derivatives; benzotriazole derivatives; non-photosensitive triazine derivatives; benzalmalonate derivatives; benzimidazole derivatives; imidazolines; bis-benzazolyl derivatives; p-aminobenzoic acid (PABA) derivatives; methylenebis(hydroxyphenyl)-benzotriazole derivatives; screening polymers and screening silicones; dimers derived from α-alkylstyrene.

24. Composition according to Claim 23, **characterized in that** the organic UV-screening agent(s) is (are) chosen from the following compounds:
- Ethylhexyl salicylate,
- Octocrylene,
- Phenylbenzimidazolesulfonic acid,
- Benzophenone-3,
- Benzophenone-4,
- Benzophenone-5,
- 4-Methylbenzylidenecamphor,
- Terephthalylidenedicamphorsulfonic acid,
- Disodium phenyl dibenzimidazole tetrasulfonate,
- 2, 4, 6-tris (Diisobutyl 4'-aminobenzalmalonate)-s-triazine,
- Anisotriazine,
- Methylenebis(benzotriazolyl)tetramethylbutylphenol,
- Drometrizole trisiloxane, and mixtures thereof.

25. Composition according to any one of Claims 1 to 24, **characterized in that** it also comprises coated or uncoated metal oxide pigments or nanopigments.

26. Composition according to Claim 25, **characterized in that** the said pigments or nanopigments are chosen from titanium oxide, zinc oxide, iron oxide, zirconium oxide and cerium oxide, and mixtures thereof, which may be coated or uncoated.

27. Composition according to any one of Claims 1 to 26, **characterized in that** it also comprises at least one agent for artificially tanning and/or browning the skin.

28. Composition according to any one of Claims 1 to 27, **characterized in that** it also comprises at least one adjuvant chosen from fatty substances, organic solvents, ionic or nonionic thickeners, softeners, antioxidants, free-radical scavengers, opacifiers, stabilizers, emollients, silicones, α-hydroxy acids, antifoams, moisturizers, vitamins, insect repellants, fragrances, preserving agents, surfactants, antiinflammatories, substance P antagonists, fillers, polymers, propellants, acidifying or basifying agents and colorants.

29. Composition according to any one of Claims 1 to 28, **characterized in that** it is a composition for protecting the human epidermis or an antisun composition and **in that** it is in the form of a nonionic vesicular dispersion, an emulsion, in particular an emulsion of oil-in-water type, a cream, a milk, a gel, a cream-gel, a suspension, a dispersion, a powder, a solid tube, a mousse or a spray.

30. Composition according to any one of Claims 1 to 29, **characterized in that** it is a makeup composition for the eyelashes, the eyebrows or the skin and **in that** it is in solid or pasty, anhydrous or aqueous form, or in the form of an emulsion, a suspension or a dispersion.

31. Composition according to any one of Claims 1 to 29, **characterized in that** it is a composition for protecting the hair against ultraviolet rays and **in that** it is in the form of a shampoo, a lotion, a gel, an emulsion or a nonionic vesicular dispersion.

32. Use of a 4,4-diarylbutadiene compound as defined in the preceding claims in cosmetic compositions containing at least one dibenzoylmethane derivative in combination with at least one photosensitive 1,3,5-triazine derivative as defined in the preceding claims, in order to improve the stability towards UV radiation (photostability) of the said 1,3,5-triazine derivative in the said compositions; the weight ratio of the 4,4-diarylbutadiene compound to the dibenzoylmethane derivative being greater than 2.5 and the said compositions containing no cinnamate derivative.

## Claims (Claims for the following Contracting State(s): DE)

1. Cosmetic or dermatological composition for topical use, for protecting the human epidermis against UV radiation, **characterized in that** it comprises, in a cosmetically acceptable support:
(a) at least one dibenzoylmethane derivative, and
(b) at least one 1,3,5-triazine derivative that is photosensitive in the presence of a dibenzoylmethane derivative, and
(c) at least one 4,4-diarylbutadiene compound; the weight ratio of the 4,4-diarylbutadiene compound to the dibenzoylmethane derivative being greater than 2.5 and the said composition containing no cinnamate derivative,
and **in that** it is in the form of a nonionic vesicular dispersion; in the form of an oil/water emulsion; in the form of gel; in the form of powder; in the form of a solid stick; in the form of an aerosol mousse or a spray.

2. Cosmetic or dermatological composition for topical use, for protecting the hair against UV rays, **characterized in that** it comprises, in a cosmetically acceptable support:
(a) at least one dibenzoylmethane derivative, and
(b) at least one 1,3,5-triazine derivative that is photosensitive in the presence of a dibenzoylmethane derivative, and
(c) at least one 4,4-diarylbutadiene diarylbutadiene compound; the weight ratio of the 4,4-diarylbutadiene compound to the dibenzoylmethane derivative being greater than 2.5 and the said composition containing no cinnamate derivative,
and **in that** it is in the form of a shampoo, a gel or a nonionic vesicular dispersion.

3. Composition for making up the eyelashes, the eyebrows or the skin, **characterized in that** it comprises, in a cosmetically acceptable support:
(a) at least one dibenzoylmethane derivative, and
(b) at least one 1,3,5-triazine derivative that is photosensitive in the presence of a dibenzoylmethane derivative, and
(c) at least one 4,4-diarylbutadiene compound; the weight ratio of the 4,4-diarylbutadiene compound to the dibenzoylmethane derivative being greater than 2.5 and the said composition containing no cinnamate derivative, and **in that** it is in solid or pasty, anhydrous or aqueous form or in the form of an oil/water emulsion or a nonionic vesicular dispersion.

4. Composition according to any one of Claims 1 to 3, in which the 1,3,5-triazine derivative corresponds to formula (I) below: 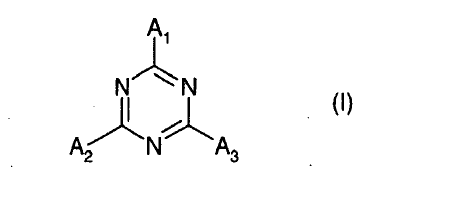 in which the radicals A₁, A₂ and A₃, which may be identical or different, are chosen from the groups of formula (II): 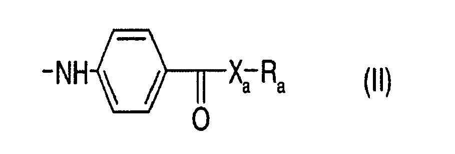 in which:
- Xₐ, which may be identical or different, represent oxygen or a radical -NH-;
- Rₐ, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a polyoxyethylenated radical comprising from 1 to 6 ethylene oxide units, the terminal OH group of which is methylated; a radical of formula (III), (IV) or (V) below: 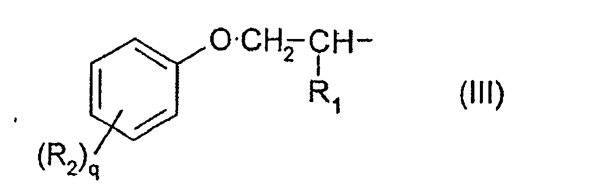 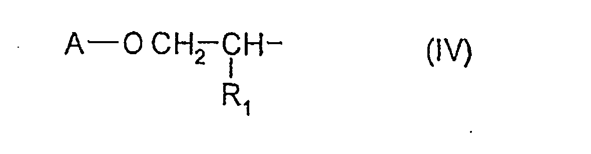 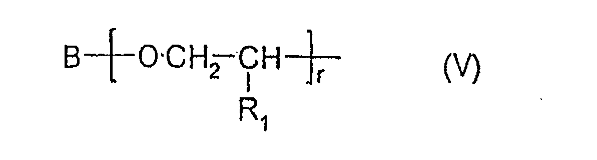
in which:
- R₁ is hydrogen or a methyl radical;
- R2 is a C₁-C₉ alkyl radical;
- q is an integer ranging from 0 to 3;
- r is an integer ranging from 1 to 10;
- A is a C₄-C₈ alkyl radical or a C₅-C₈ cycloalkyl radical;
- B is chosen from: a linear or branched C1-C8 alkyl radical; a C₅-C₈ cycloalkyl radical; an aryl radical optionally substituted with one or more C1-C4 alkyl radicals.

5. Composition according to Claim 4, **characterized in that** the 1,3,5-triazine derivative of formula (I) is chosen from those in which A₁, A₂ and A₃ are of formula (II) and have the following characteristics:
- one of the groups Xₐ-Rₐ represents a radical - NH-Rₐ with Rₐ chosen from: a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical;
- the other two groups Xₐ-Rₐ represent a radical
- O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical.

6. Composition according to Claim 4, **characterized in that** the 1,3,5-triazine derivative is chosen from those in which A₁, A₂ and A₃ are of formula (II) and have all of the following characteristics:
- one or two groups Xₐ-Rₐ represent a radical - NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical; the other or the other two group(s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (III), (IV) or (V) above in which:
- B is a C₁-C₄ alkyl radical;
- R₂ is a methyl radical.

7. Composition according to Claim 6, **characterized in that** the 1,3,5-triazine derivative is that corresponding to the following formula: 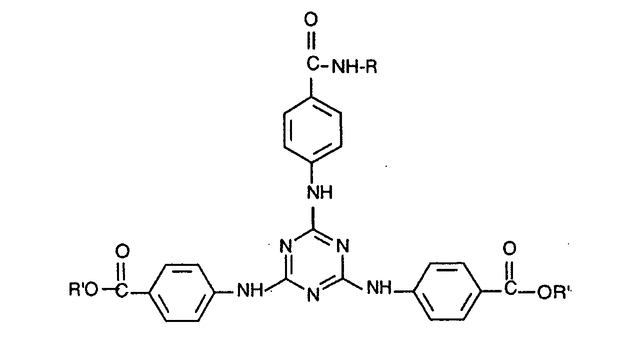 in which R' denotes a 2-ethylhexyl radical and R denotes a tert-butyl radical.

8. Composition according to Claim 4, **characterized in that** the 1,3,5-triazine derivative corresponding to formula (I) is chosen from those in which A₁, A₂ and A₃ are of formula (II) and have the following characteristics:
- Xₐ are identical and represent oxygen;
- Rₐ, which may be identical or different, represent a C₆-C₁₂ alkyl radical or a polyoxyethylenated radical comprising from 1 to 6 ethylene oxide units and in which the terminal OH group is methylated.

9. Composition according to Claim 8, **characterized in that** the 1,3,5-triazine derivative is that corresponding to the following formula: 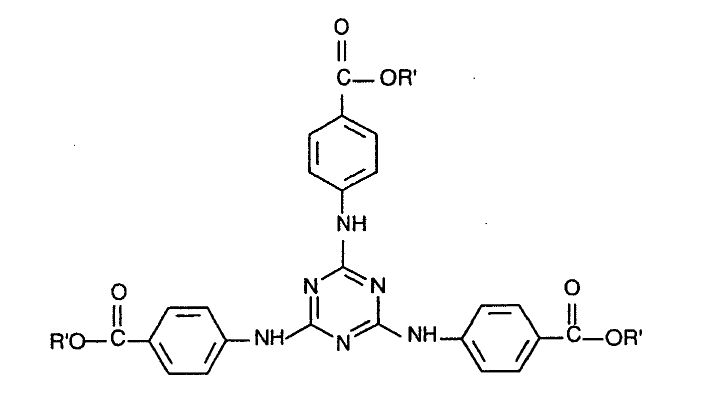 in which R' denotes a 2-ethylhexyl radical.

10. Composition according to any one of Claims 1 to 9, **characterized in that** the 1,3,5-triazine derivative is present in contents ranging from 0.5% to 15% by weight and preferably from 1% to 10% by weight, relative to the total weight of the composition.

11. Composition according to any one of Claims 1 to 10, **characterized in that** the dibenzoylmethane derivative is chosen from:
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4,4'-dimethoxydibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

12. Composition according to Claim 11, **characterized in that** the dibenzoylmethane derivative is 4-tert-butyl-4'-methoxydibenzoylmethane.

13. Composition according to Claim 11, **characterized in that** the dibenzoylmethane derivative is 4-isopropyldibenzoylmethane.

14. Composition according to any one of Claims 1 to 13, in which the dibenzoylmethane derivative is present in contents ranging from 0.5% to 15% by weight and preferably from 1% to 10% by weight, relative to the total weight of the composition.

15. Composition according to any one of Claims 1 to 14, in which the 4,4-diarylbutadiene compound corresponds to formula (VIII) below: 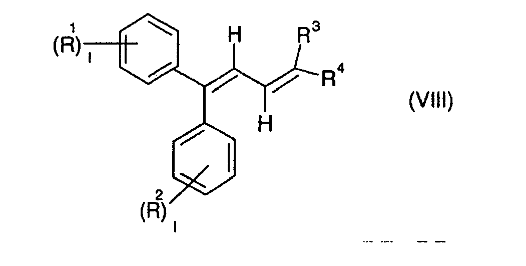 in which the diene system is of Z,Z; Z,E; E,Z or E,E configuration or mixtures of the said configurations, and in which:
- R¹ and R², which may be identical or different, denote hydrogen, a linear or branched C₁-C₂₀ alkyl radical; a C₂-C₁₀ alkenyl radical; a C₁-C₁₂ alkoxy radical; a C₃-C₁₀ cycloalkyl radical; a C₃-C₁₀ cycloalkenyl radical; a linear or branched C₁-C₁₀ alkoxycarbonyl radical; a linear or branched C₁-C₁₂ monoalkylamino radical; a linear or branched C₁-C₁₂ dialkylamino radical; an aryl; a heteroaryl or a hydro-solubilizing substituent chosen from a carboxylate group, a sulphonate group and an ammonium residue;
- R³ denotes a group COOR⁵; COR⁵; CONR⁵R⁶; CN; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; a C₆-C₁₈ aryl; a C₃-C₇ heteroaryl;
- R⁴ denotes a group COOR⁶; COR⁶; CONR⁵R⁶; CN; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- R⁵ and R⁶, which may be identical or different, denote hydrogen; [V]ₒ-R⁷; C₁-C₆-alkylene-SO₃U; C₁-C₆-alkylene-PO₃U; C₁-C₆-alkylene-N(R⁸)₃+ D⁻; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- V denotes a group -CH₂-CH₂-W-, -CH₂CH₂CH₂W-,
- CH(CH₃)-CH₂-W-, -CH₂-CH₂-CH₂-CH₂-W- or -CH₂-CH(CH₂CH₃)-W-;
- D denotes Cl, Br, I or SO₄R⁹;
- U denotes hydrogen, Na⁺, K⁺, Mg²⁺, Ca²⁺, Li⁺, Al³⁺ or -N(R⁸)₄+;
- W denotes 0 or NH;
- R ⁷ and R⁸, which may be identical or different, denote hydrogen, a linear or branched C₁-C₆ alkyl radical; a linear or branched C₂-C₆ alkenyl radical; a linear or branched C₁-C₆ acyl radical;
- R⁹ denotes hydrogen, a linear or branched C₁-C₆ alkyl radical; a C₂-C₆alkenyl radical;
- I ranges from 1 to 3;
- o ranges from 0 to 150.

16. Composition according to Claim 15, in which the compound of formula (VIII) is chosen from those of formula (VIIIa) below: 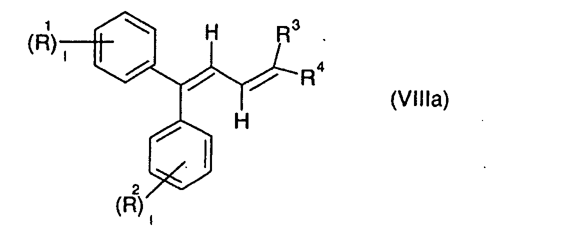 in which the diene system is of Z,Z; Z,E; E,Z or E,E configuration or mixtures of the said configurations, and in which:
- R¹ and R², which may be identical or different, denote hydrogen, a C₁-C₈ alkyl radical; a C₁-C₈ alkoxy radical; a hydro-solubilizing substituent chosen from a carboxylate group, a sulphonate group and an ammonium residue;
- R³ denotes a group COOR⁵; CONR⁵R⁶; CN;
- R⁴ denotes a group. COOR⁶; _{CONR}⁵_{R}⁶;
- R⁵ denotes hydrogen; [V]ₒ-R⁷; C₁-C₆-alkylene-SO₃U ; C₁-C₆-alkylene-N(R⁸)/ D⁻;
- R⁶ denotes [V]ₒ-R⁷; C₁-C₆-alkylene-SO₃U; C₁-C₆-alkylene-N (R⁸) ₃⁺ D⁻;
- V denotes a -CH₂-CH₂-O-, -CH₂CH₂CH₂O-, -CH (CH₃) - CH₂-O- group;
- D denotes Cl, Br, I or SO₄R⁹;
- U denotes hydrogen, Na⁺, K⁺, Mg²⁺, Ca²⁺, Li⁺, Al³⁺ or -N (R⁸)₄⁺;
- R⁷ R⁸ and R⁹, which may be identical or different, denote hydrogen or a linear or branched C₁-C₃ alkyl radical;
- I ranges from 1 to 3;
- o ranges from 0 to 50.

17. Composition according to Claim 16, in which the compound of formula (VIII) is chosen from those corresponding to formula (VIIIb) below: 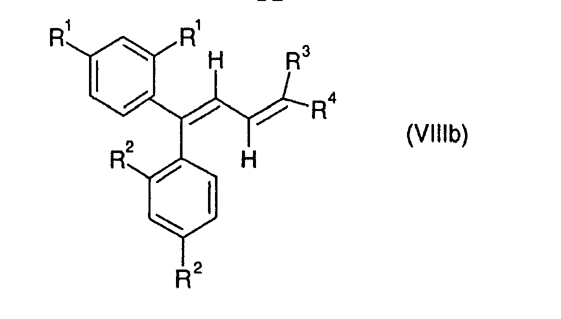 in which the diene system is of Z,Z; Z,E; E,Z or E,E configuration or mixtures of the said configurations, and in which:
- R¹ and R², which may be identical or different, denote hydrogen, a C₁-C₈ alkyl radical; a C₁-C₈ alkoxy radical;
- R³ denotes a group COOR⁵; CONR⁵R⁶; CN;
- R⁴ denotes a group COOR⁶; CONR⁵R⁶;
- R⁵ denotes hydrogen; [V]ₒ-R⁷; C₁-C₆-alkylene-SO₃U; C₁-C₆-alkylene-N (R⁸)₃⁺ D⁻ ;
- R⁶ denotes [V] ₒ-R⁷; C₁-C₆-alkylene-SO₃U; C₁-C₆-alkylene-N (R⁸) ₃⁺ D⁻;
- V denotes a -CH₂-CH₂-O-, -CH₂CH₂CH₂O-, -CH (CH₃) - CH₂-O- group;
- D denotes Cl, Br, I or SO₄R⁹;
- U denotes hydrogen, Na⁺, K⁺, Mg²⁺, Ca²⁺, Li⁺, Al³⁺ or -N (R⁸)₄⁺;
- R⁷, R⁸ and R⁹, which may be identical or different, denote hydrogen or a linear or branched C₁-C₃ alkyl radical;
- o ranges from 0 to 50.

18. Composition according to Claim 17, in which the compound of formula (VIII) is chosen from those corresponding to formula (VIIIc) below: 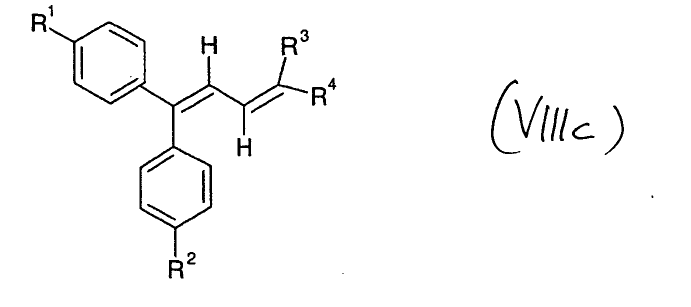 in which the diene system is of Z,Z; Z,E; E,Z or E,E configuration or mixtures of the said configurations, and in which:
- R¹ and R², which may be identical or different, denote hydrogen, a C₁-C₈ alkyl radical; a C₁-C₈ alkoxy radical;
- R³ denotes a group COOR⁵; CONR⁵R⁶; CN;
- R⁴ denotes a group COOR⁶; CONR⁵R⁶;
- R⁵ denotes hydrogen; [V]ₒ-R⁷; C₁-C₆-alkylene-SO₃Y; C₁-C₆-alkylene-N (R⁸)₃⁺ D⁻;
- R⁶ denotes [V]ₒ-R⁷; C₁-C₆-alkylene-SO₃U; C₁-C₆-alkylene-N(R⁸)₃⁺ D⁻;
- V denotes a -CH₂-CH₂-O-, -CH₂CH₂CH₂O-, -CH(CH₃)-CH₂-O- group;
- D denotes Cl, Br, I or SO₄R⁹;
- U denotes hydrogen, Na⁺, K⁺, Mg²⁺, Ca²⁺, Li⁺, Al³⁺ or -N(R⁸)₄⁺;
- R⁷, R⁸ and R⁹, which may be identical or different, denote hydrogen or a linear or branched C₁-C₃ alkyl radical;
- o ranges from 0 to 50.

19. Composition according to any one of Claims 15 to 17, in which the compound of formula (VIII) is chosen from the following compounds:

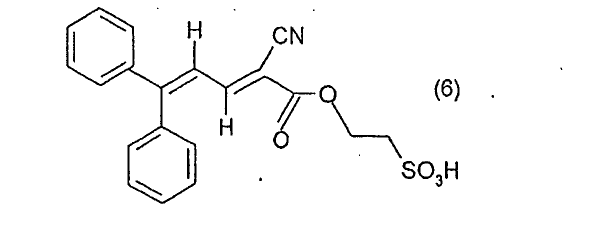

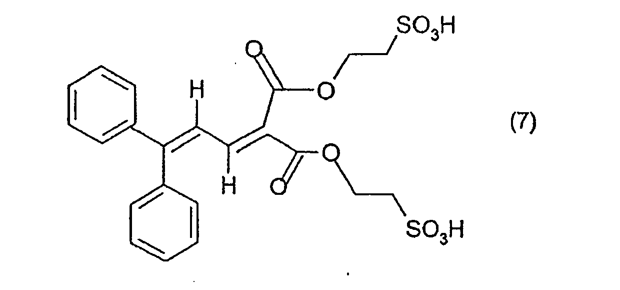

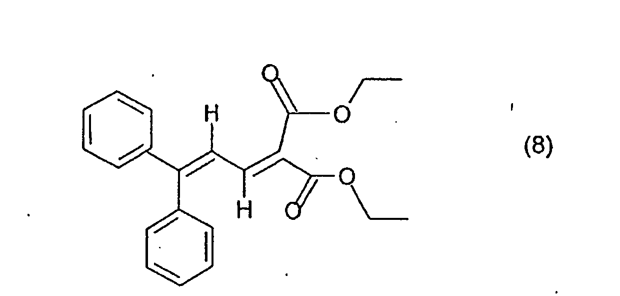

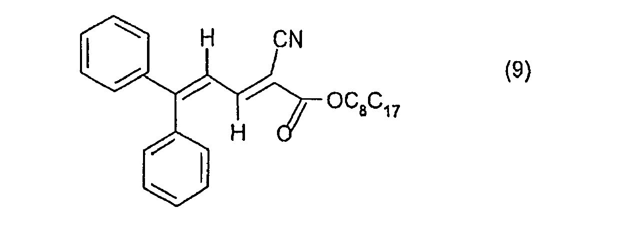

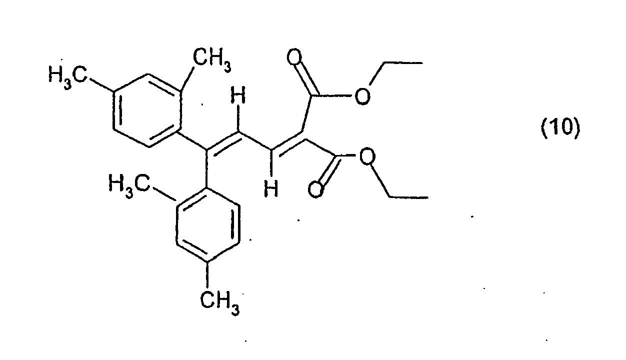

20. Composition according to any one of Claims 1 to 14, in which the 4,4-diarylbutadiene compound is an oligomer corresponding to formula (IX) below: 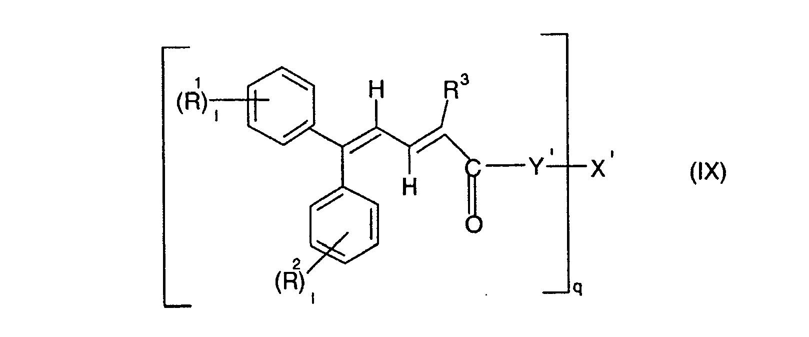 in which the diene system is of Z,Z; Z,E; E,Z or E,E configuration or mixtures of the said configurations, and in which:
- R¹, R², R³ and I have the same definitions as indicated in formula (VIII) in Claim 17;
- Y' denotes a group -0- or -NR¹⁰-;
- R¹⁰ denotes hydrogen; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- X' denotes an aliphatic or cycloaliphatic linear or branched polyol residue comprising from 2 to 10 hydroxyl groups and of valency q; the carbon-based chain of the said residue possibly being interrupted with one or more sulphur or oxygen atoms; one or more imine groups or one or more C₁-C₄ alkylimino groups;
- q ranges from 2 to 10.

21. Composition according to Claim 20, in which the compound of formula (IX) is chosen from those for which:
- R¹ and R², which may be identical or different, denote hydrogen, a C₁-C₁₂ alkyl radical; a C₁-C₈ alkoxy radical; a hydro-solubilizing substituent chosen from a carboxylate group, a sulphonate group or an ammonium residue;
- R³ denotes a group COOR⁵; CONR⁵R⁶; CN; a C₃-C₁₀ cycloalkyl radical; a C₇-C₁₀ bicycloalkyl radical;
- R⁵ and R⁶, which may be identical or different, denote a linear or branched C₁-C₂₀ alkyl radical; a C₃-C₁₀ cycloalkyl radical; a C₇-C₁₀ bicycloalkyl radical; optionally substituted naphthyl or phenyl;
- X' denotes a polyol residue comprising from 2 to 6 and more particularly from 2 to 4 hydroxyl groups.

22. Composition according to Claim 21, in which the compound of formula (IX) is chosen from those for which:
- X' denotes an ethanol or pentaerythrol residue.

23. Composition according to Claim 22, in which the compound of formula (IX) is chosen from the following compounds:

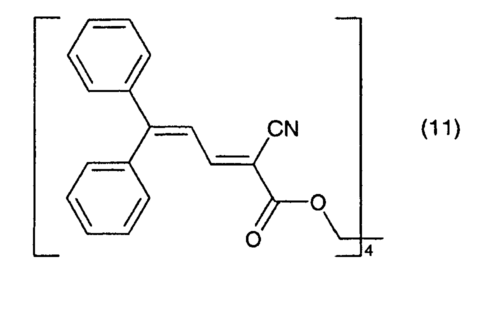

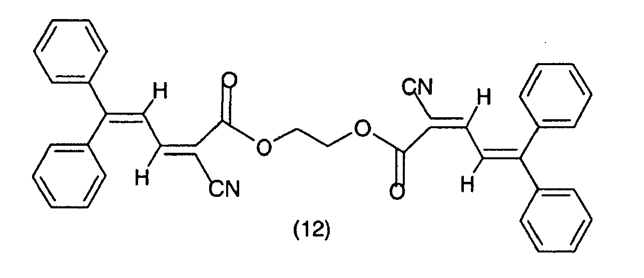

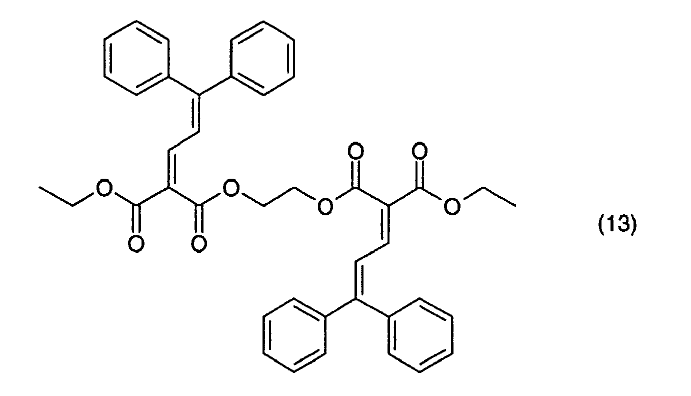

24. Composition according to any one of Claims 1 to 23, **characterized in that** it also contains other UV-A-active and/or UV-B-active organic screening agents.

25. Composition according to Claim 24, in which the additional organic UV-screening agent(s) is (are) chosen from anthranilates; salicylic derivatives; camphor derivatives; benzophenone derivatives; β,β'-diphenylacrylate derivatives; benzotriazole derivatives; non-photosensitive triazine derivatives; benzalmalonate derivatives; benzimidazole derivatives; imidazolines; bis-benzazolyl derivatives; p-aminobenzoic acid (PABA) derivatives; methylenebis(hydroxyphenyl)-benzotriazole derivatives; screening polymers and screening silicones; dimers derived from α-alkylstyrene.

26. Composition according to Claim 25, **characterized in that** the organic UV-screening agent(s) is (are) chosen from the following compounds:
- Ethylhexyl salicylate,
- Octocrylene,
- Phenylbenzimidazolesulphonic acid,
- Benzophenone-3,
- Benzophenone-4,
- Benzophenone-5,
- 4-Methylbenzylidenecamphor,
- Terephthalylidenedicamphorsulphonic acid,
- Disodium phenyl dibenzimidazole tetrasulphonate,
- 2, 4, 6-tris (Diisobutyl 4'-aminobenzalmalonate)-s-triazine,
- Anisotriazine,
- Methylenebis(benzotriazolyl)tetramethylbutylphenol,
- Drometrizole trisiloxane,
and mixtures thereof.

27. Composition according to any one of Claims 1 to 26, **characterized in that** it also comprises coated or uncoated metal oxide pigments or nanopigments.

28. Composition according to Claim 27, **characterized in that** the said pigments or nanopigments are chosen from titanium oxide, zinc oxide, iron oxide, zirconium oxide and cerium oxide, and mixtures thereof, which may be coated or uncoated.

29. Composition according to any one of Claims 1 to 28, **characterized in that** it also comprises at least one agent for artificially tanning and/or browning the skin.

30. Composition according to any one of Claims 1 to 29, **characterized in that** it also comprises at least one adjuvant chosen from fatty substances, organic solvents, ionic or nonionic thickeners, softeners, antioxidants, free-radical scavengers, opacifiers, stabilizers, emollients, silicones, α-hydroxy acids, antifoams, moisturizers, vitamins, insect repellants, fragrances, preserving agents, surfactants, antiinflammatories, substance P antagonists, fillers, polymers, propellants, acidifying or basifying agents and colorants.

31. Use of a 4,4-diarylbutadiene compound as defined in the preceding claims in cosmetic compositions containing at least one dibenzoylmethane derivative in combination with at least one photosensitive 1,3,5-triazine derivative as defined in the preceding claims, in order to improve the stability towards UV radiation (photostability) of the said 1,3,5-triazine derivative in the said compositions; the weight ratio of the 4,4-diarylbutadiene compound to the dibenzoylmethane derivative being greater than 2.5 and the said compositions containing no cinnamate derivative.

## Patentansprüche (Patentansprüche für folgende(n) Vertragsstaat(en): AT, BE, CH, CY, DK, ES, FI, FR, GB, GR, IE, IT, LI, LU, MC, NL, PT, SE, TR)

1. Kosmetische oder dermatologische Zusammensetzung zur topischen Anwendung, insbesondere zum Lichtschutz der Haut und der Haare, **dadurch gekennzeichnet, dass** sie in einem kosmetisch akzeptablen Träger enthält:
(a) mindestens ein Dibenzoylmethan-Derivat und
(b) mindestens ein in Gegenwart eines Dibenzoylmethan-Derivats lichtempfindliches 1,3,5-Triazin-Derivat sowie
(c) mindestens eine 4,4-Diarylbutadien-Verbindung,
wobei das Gewichtsverhältnis von 4,4-Diarylbutadien-Verbindung zu Dibenzoylmethan-Derivat größer als 2,5 ist und die Zusammensetzung kein Cinnamat-Derivat enthält.

2. Zusammensetzung nach Anspruch 1, in der das 1,3,5-Triazin-Derivat die nachstehende Formel (I) aufweist: 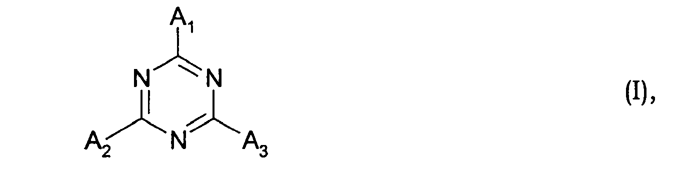 in der die Gruppen A₁, A₂ und A₃, die gleich oder verschieden sind, unter den Gruppen der Formel (II) ausgewählt sind: 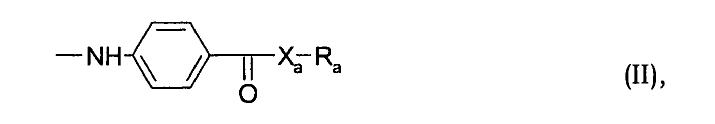 worin
- Xₐ, die gleich oder verschieden sein können, Sauerstoff oder die Gruppe -NH- bedeuten;
- Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁-₁₈-Alkylgruppe; einer C₅₋₁₂-Cycloalkylgruppe, die gegebenenfalls mit einer oder mehreren C₁₋₄-Alkylgruppen substituiert ist; einer Polyoxyethylen-Gruppe, die 1 bis 6 Ethylenoxideinheiten aufweist und deren endständige OH - Gruppe methyliert ist; einer Gruppe der nachstehenden Formeln (III), (IV) oder (V): 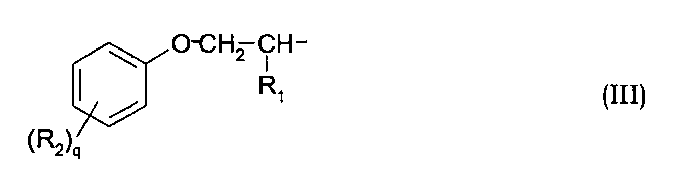 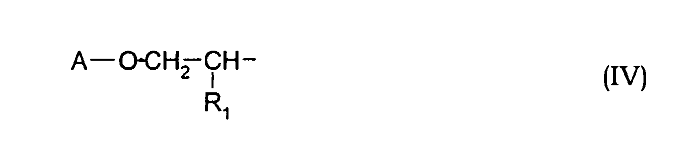 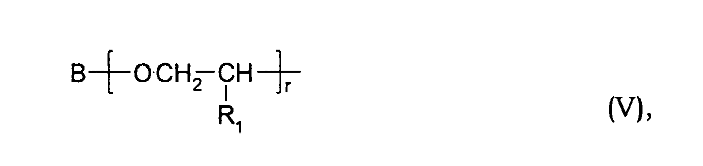
worin:
- R₁ Wasserstoff oder Methyl bedeutet;
- R₂ eine C₁₋₉-Alkylgruppe ist;
- q eine ganze Zahl von 0 bis 3 bedeutet;
- r eine ganze Zahl von 1 bis 10 bedeutet;
- A eine C₄₋₈-Alkylgruppe oder eine C₅₋₈-Cycloalkylgruppe bedeutet;
- B ausgewählt ist unter: einer geradkettigen oder verzweigten C₁₋₈-Alkylgruppe; einer C₅₋₈-Cycloalkylgruppe; einer Arylgruppe, die gegebenenfalls mit einer oder mehreren C₁₋₄-Alkylgruppen substituiert ist.

3. Zusammensetzung nach Anspruch 2, **dadurch gekennzeichnet, dass** das 1,3,5-Triazin-Derivat der Formel (I) unter solchen Verbindungen ausgewählt ist, bei denen A₁, A₂ und A₃ die Formel (II) besitzen und bei denen folgende Merkmale vorliegen:
- eine Gruppe Xₐ-Rₐ bedeutet die Gruppe -NH-Rₐ, wobei Rₐ ausgewählt ist unter: einer C₅₋₁₂-Cycloalkylgruppe, die gegebenenfalls mit einer oder mehreren C₁₋₄-Alkylgruppen substituiert ist; einer Gruppe der obigen Formel (III), (IV) oder (V), worin bedeuten:
- B eine C₁₋₄-Alkylgruppe;
- R₂ Methyl;
- die 2 übrigen Gruppen Xₐ-Rₐ bedeuten die Gruppe -O-Rₐ,
wobei Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der Formel (III), (IV) oder (V) wie oben, worin bedeuten:
- B eine C₁₋₄-Alkylgruppe;
- R₂ Methyl.

4. Zusammensetzung nach Anspruch 2, **dadurch gekennzeichnet, dass** das 1,3,5-Triazin-Derivat unter Verbindungen ausgewählt ist, bei denen A₁, A₂ und A₃ die Formel (II) besitzen und bei denen die Gesamtheit folgender Merkmale vorliegt:
- eine oder zwei Gruppen Xₐ-Rₐ bedeuten die Gruppe -NH-Rₐ, wobei Rₐ ausgewählt ist unter: einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der obigen Formel (III), (IV) oder (V), worin bedeuten:
- B eine C₁₋₄-Alkylgruppe;
- R₂ Methyl;
- die übrige Gruppe Xₐ-Rₐ oder die beiden übrigen Gruppen Xₐ-Rₐ bedeuten die Gruppe -O-Rₐ, wobei Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der Formel (III), (IV) oder (V) wie oben, worin bedeuten:
- B eine C₁₋₄-Alkylgruppe;
- R₂ Methyl.

5. Zusammensetzung nach Anspruch 4, **dadurch gekennzeichnet, dass** das 1,3,5-Triazin-Derivat das Derivat der nachstehenden Formel ist: 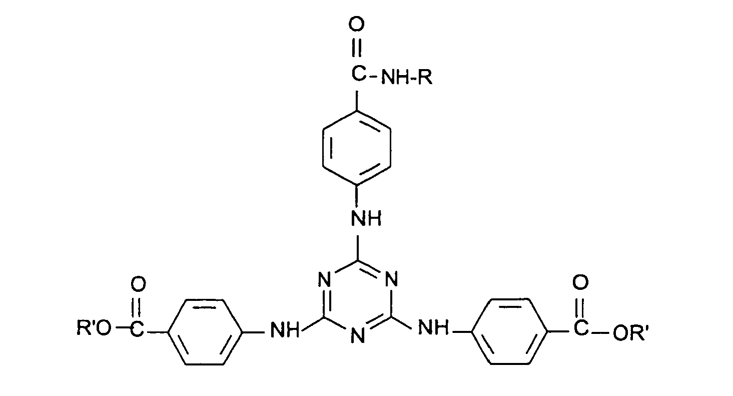 in der R' eine 2-Ethylhexyl-Gruppe und R eine tert.-ButylGruppe bezeichnen.

6. Zusammensetzung nach Anspruch 2, **dadurch gekennzeichnet, dass** das 1,3,5-Triazin-Derivat der Formel (I) unter Verbindungen ausgewählt ist, bei denen A₁, A₂ und A₃ die Formel (II) besitzen und bei denen folgende Merkmale vorliegen:
- Xₐ sind gleich und bedeuten Sauerstoff;
- Rₐ, die gleich oder verschieden sind, bedeuten eine C₆₋₁₂-Alkylgruppe oder eine Polyoxyethylen-Gruppe, die 1 bis 6 Ethylenoxideinheiten aufweist und deren endständige OH - Gruppe methyliert ist.

7. Zusammensetzung nach Anspruch 6, **dadurch gekennzeichnet, dass** das 1,3,5-Triazin-Derivat das Derivat der nachstehenden Formel ist: 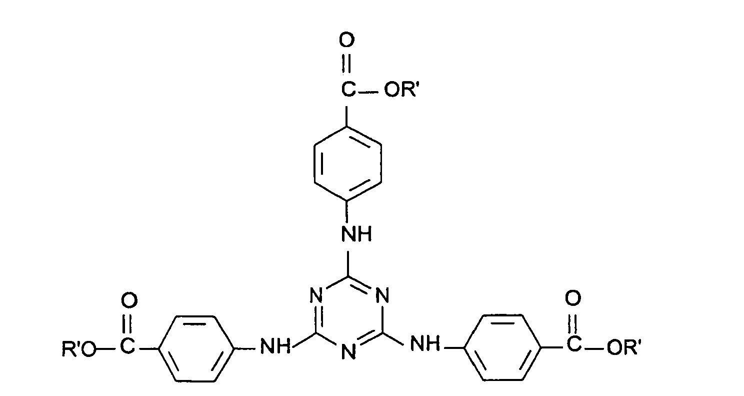 in der R' eine 2-Ethylhexyl-Gruppe bezeichnet.

8. Zusammensetzung nach einem der Ansprüche 1 bis 7, **dadurch gekennzeichnet, dass** das 1,3,5-Triazin-Derivat in Mengenanteilen von 0,5 bis 15 Gew.-% und bevorzugt in Mengenanteilen von 1 bis 10 Gew.-%, bezogen auf das Gesamtgewicht der Zusammensetzung, vorliegt.

9. Zusammensetzung nach einem der Ansprüche 1 bis 8, **dadurch gekennzeichnet, dass** das Dibenzoylmethan-Derivat ausgewählt ist unter:
- 2-Methyldibenzoylmethan
- 4-Methyldibenzoylmethan
- 4-Isopropyldibenzoylmethan
- 4-tert.-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-tert.-Butyl-4'-methoxydibenzoylmethan
- 2-Methyl-5-isopropyl-4'-methoxydibenzoylmethan
- 2-Methyl-5-tert.-butyl-4'-methoxydibenzoylmethan
- 2,4-Dimethyl-4'-methoxydibenzoylmethan
- 2,6-Dimethyl-4-tert.-butyl-4'-methoxydibenzoylmethan.

10. Zusammensetzung nach Anspruch 9, **dadurch gekennzeichnet, dass** das Dibenzoylmethan-Derivat 4-tert.-Butyl-4'-methoxy-dibenzoylmethan ist.

11. Zusammensetzung nach Anspruch 9, **dadurch gekennzeichnet, dass** das Dibenzoylmethan-Derivat 4-Isopropyldibenzoylmethan ist.

12. Zusammensetzung nach einem der Ansprüche 1 bis 11, in der das Dibenzoylmethan-Derivat in Mengenanteilen von 0,5 bis 15 Gew.-% und bevorzugt in Mengenanteilen von 1 bis 10 Gew.-%, bezogen auf das Gesamtgewicht der Zusammensetzung, vorliegt.

13. Zusammensetzung nach einem der Ansprüche 1 bis 12, in der die 4,4-Diarylbutadien-Verbindung die nachstehende Formel (VIII) aufweist: 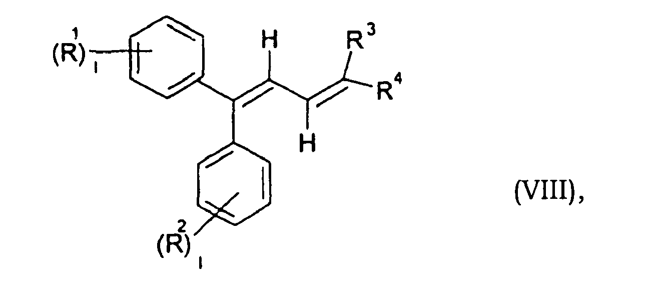 in der das Diensystem die Konfiguration Z,Z; Z,E; E,Z oder E,E aufweist oder Gemische dieser Konfigurationen vorliegen und wobei bedeuten:
- R¹ und R², die gleich oder verschieden sind, Wasserstoff; eine geradkettige oder verzweigte C₁₋₂₀-Alkylgruppe; eine C₂₋₁₀-Alkenylgruppe; eine C₁₋₁₂-Alkoxygruppe; eine C₃₋₁₀-Cycloalkylgruppe; eine C₃₋₁₀-Cycloalkenylgruppe; eine geradkettige oder verzweigte C₁₋₂₀-Alkoxycarbonylgruppe; eine geradkettige oder verzweigte C₁₋₁₂-Monoalkylaminogruppe; eine geradkettige oder verzweigte C₁₋₁₂-Dialkylaminogruppe; eine Arylgruppe, eine Heteroarylgruppe oder einen wasserlöslich machenden Substituenten, der unter einer Carboxylatgruppe, einer Sulfonatgruppe und einem Ammoniumrest ausgewählt ist;
- R³ eine Gruppe COOR⁵; COR⁵; CONR⁵R⁶; CN; eine geradkettige oder verzweigte C₁₋₂₀-Alkylgruppe; eine C₂₋₁₀-Alkenylgruppe, eine C₃₋₁₀-Cycloalkylgruppe; eine C₇₋₁₀-Bicycloalkylgruppe, eine C₃₋₁₀-Cycloalkenylgruppe, eine C₇₋₁₀-Bicycloalkenylgruppe; eine C₆₋₁₈-Arylgruppe; eine C₃₋₇-Heteroarylgruppe;
- R⁴ eine Gruppe COOR⁶; COR⁶; CONR⁵R⁶; CN; eine geradkettige oder verzweigte C₁₋₂₀-Alkylgruppe, eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- R⁵ und R⁶, die gleich oder verschieden sind, Wasserstoff; [V]ₒ-R⁷; C₁₋₆-Alkylen-SO₃U; C₁₋₆-Alkylen-PO₃U; C₁₋₆-Alkylen-N(R⁸)₃⁺ D⁻; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- V eine Gruppe -CH₂-CH₂-W-, -CH₂CH₂CH₂W-, -CH(CH₃)-CH₂-W-, -CH₂-CH₂-CH₂-CH₂-W-, -CH₂-CH(CH₂CH₃)-W-;
- D Cl, Br, I, SO₄R⁹;
- U Wasserstoff, Na⁺, K⁺, Mg²⁺, Ca²⁺, Li⁺, Al³⁺, -N(R8)₄⁺;
- W O oder NH;
- R⁷ und R⁸, die gleich oder verschieden sind, Wasserstoff; eine geradkettige oder verzweigte C₁₋₆-Alkylgruppe; eine geradkettige oder verzweigte C₂₋₆-Alkenylgruppe; eine geradkettige oder verzweigte C₁₋₆-Acylgruppe;
- R⁹ Wasserstoff; eine geradkettige oder verzweigte C₁₋₆-Alkylgruppe; eine C₂₋₆-Alkenylgruppe;
- 1 einen Wert von 1 bis 3;
- o einen Wert von 0 bis 150.

14. Zusammensetzung nach Anspruch 13, in der die Verbindung der Formel (VIII) unter Verbindungen der nachstehenden Formel (VIIIa) ausgewählt ist: 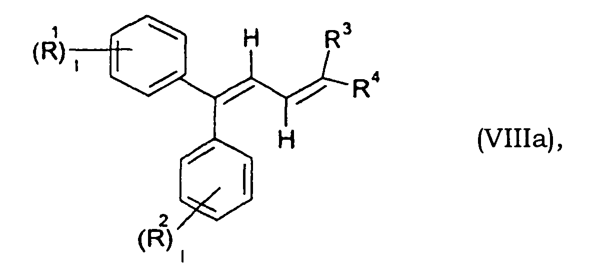 in der das Diensystem die Konfiguration ZZ; ZE; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe, eine C₁₋₈-Alkoxygruppe; einen wasserlöslich machenden Substituenten, der unter einer Carboxylatgruppe, einer Sulfonatgruppe und einem Ammoniumrest ausgewählt ist;
- R³ eine Gruppe COOR⁵; CONR⁵R⁶; CN;
- R⁴ eine Gruppe COOR⁶; CONR⁵R⁶;
- R⁵ Wasserstoff; [V]ₒ-R⁷; C₁₋₆-Alkylen-SO₃U; C₁₋₆-Alkylen-N(R⁸)₃⁺ D⁻;
- R⁶ [V]ₒ-R⁷; C₁₋₆-Alkylen-SO₃U; C₁₋₆-Alkylen-N(R⁸)₃⁺ D⁻;
- V eine Gruppe -CH₂-CH₂-O-, -CH₂CH₂CH₂O-, -CH(CH₃)-CH₂-O-;
- D Cl, Br, I, SO₄R⁹;
- U Wasserstoff, Na⁺, K⁺, Mg²⁺, Ca²⁺, Li⁺, Al³⁺, -N(R⁸)₄+;
- R⁷, R⁸ und R⁹, die gleich oder verschieden sind, Wasserstoff; eine geradkettige oder verzweigte C₁₋₃-Alkylgruppe;
- 1 einen Wert von 1 bis 3;
- o einen Wert von 0 bis 50.

15. Zusammensetzung nach Anspruch 14, in der die Verbindung der Formel (VIII) unter Verbindungen der nachstehenden Formel (VIIIb) ausgewählt ist: 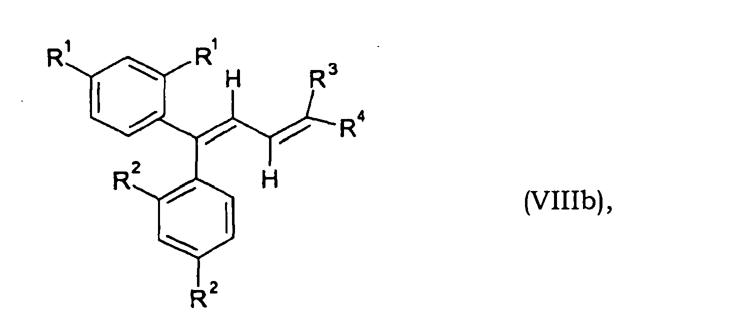 in der das Diensystem die Konfiguration ZZ; Z,E; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe; eine C₁₋₈-Alkoxygruppe;
- R³ eine Gruppe COOR⁵; CONR⁵R⁶; CN;
- R⁴ eine Gruppe COOR⁶; CONR⁵R⁶;
- R⁵ Wasserstoff; [V]ₒ-R⁷; C₁₋₆-Alkylen-SO₃U; C₁₋₆-Alkylen-N(R⁸)₃⁺ D⁻;
- R⁶ [V]ₒ-R⁷; C₁₋₆-Alkylen-SO₃U; C₁₋₆-Alkylen-N(R⁸)₃⁺ D⁻;
- V eine Gruppe -CH₂-CH₂-O-, -CH₂CH₂CH₂O-, -CH(CH₃)-CH₂-O-;
- D Cl, Br, I, SO₄R⁹;
- U Wasserstoff, Na⁺, K⁺, Mg²⁺; Ca²⁺, Li⁺, Al³⁺, -N(R⁸)₄⁺;
- R⁷, R⁸ und R⁹, die gleich oder verschieden sind, Wasserstoff; eine geradkettige oder verzweigte C₁₋₃-Alkylgruppe;
- o einen Wert von 0 bis 50.

16. Zusammensetzung nach Anspruch 15, in der die Verbindung der Formel (VIII) unter Verbindungen der nachstehenden Formel (VIIIc) ausgewählt ist: 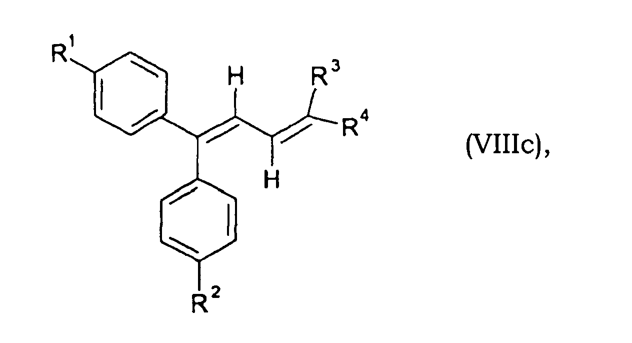 in der das Diensystem die Konfiguration Z,Z; Z,E; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe; eine C₁₋₈-Alkoxygruppe;
- R³ eine Gruppe COOR⁵; CONR⁵R⁶; CN;
- R⁴ eine Gruppe COOR⁶; CONR⁵R⁶;
- R⁵ Wasserstoff; [V]ₒ-R⁷; C₁₋₆-Alkylen-SO₃Y; C₁₋₆-Alkylen-N(R⁸)₃⁺ D⁻;
- R⁶ [V]ₒ-R⁷; C₁₋₆-Alkylen-SO₃U; C₁₋₆-Alkylen-N(R⁸)3⁺ D⁻;
- V eine Gruppe -CH₂-CH₂-O-, -CH₂CH₂CH₂O-, -CH(CH₃)-CH₂-O-;
- D Cl, Br, I, SO₄R⁹;
- U Wasserstoff, Na⁺, K⁺, Mg²⁺, Ca²⁺, Li⁺, A1³⁺, -N(R8)₄⁺;
- R⁷, R⁸ und R⁹, die gleich oder verschieden sind, Wasserstoff; eine geradkettige oder verzweigte C₁₋₃-Alkylgruppe;
- o einen Wert von 0 bis 50.

17. Zusammensetzung nach einem der Ansprüche 13 bis 16, in der die Verbindung der Formel (VIII) unter folgenden Verbindungen ausgewählt ist:

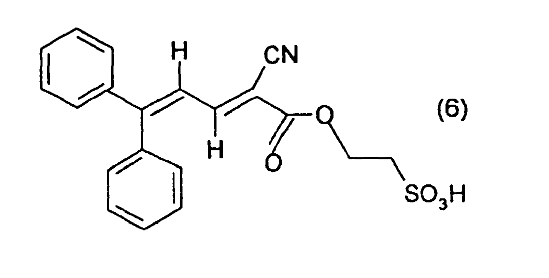

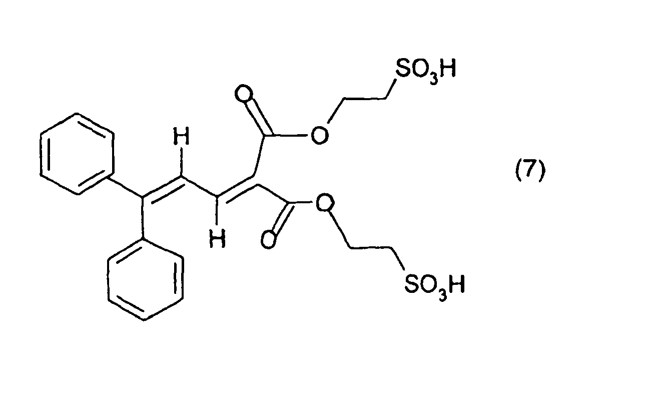

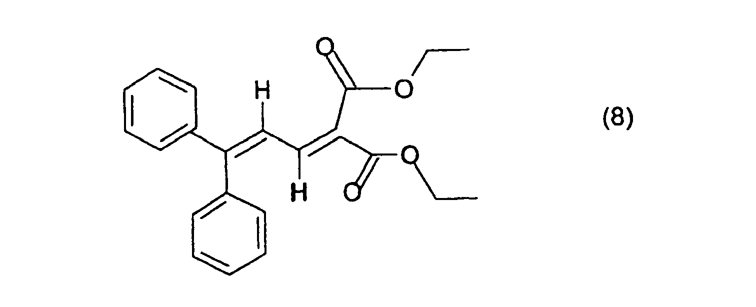

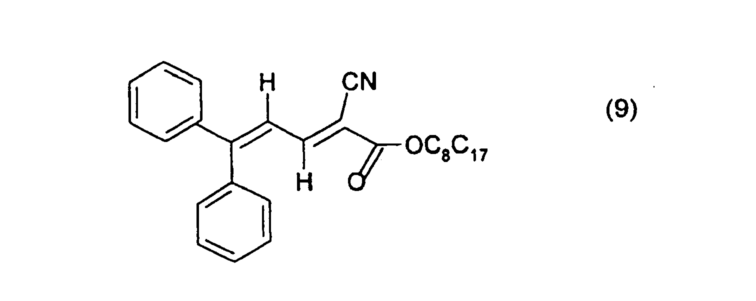

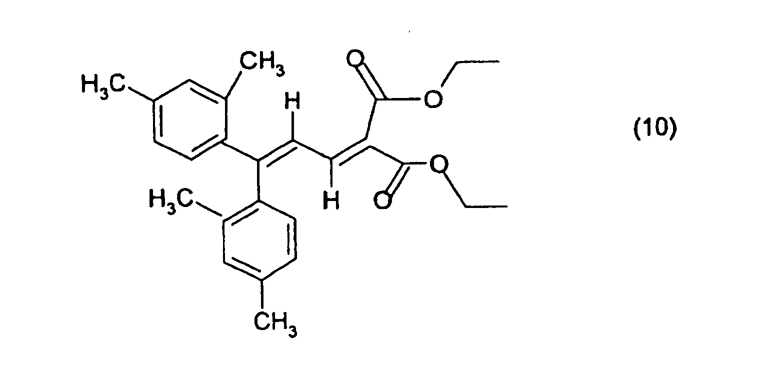

18. Zusammensetzung nach einem der Ansprüche 1 bis 12, in der die 4,4-Diarylbutadien-Verbindung ein Oligomer der nachstehenden Formel (IX) ist: 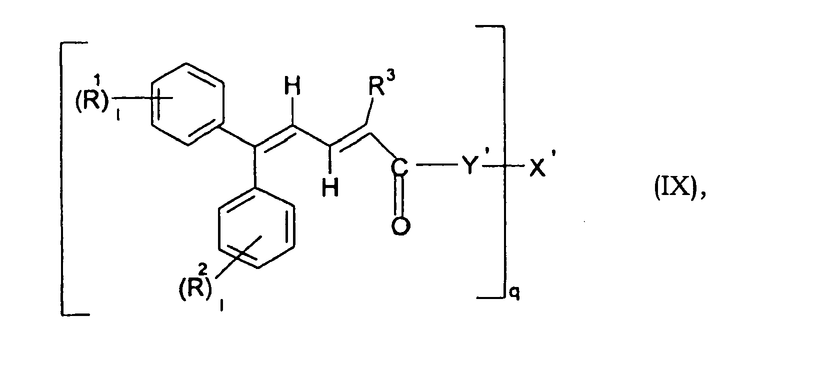 in der das Diensystem die Konfiguration Z,Z; Z,E; E,Z oder E,E aufweist oder Gemische dieser Konfigurationen vorliegen und wobei bedeuten:
- R¹, R², R³ und 1 dasselbe wie in Anspruch 17 für Formel (VIII) angegeben;
- Y' eine Gruppe -O- oder -NR¹⁰-;
- R¹⁰ Wasserstoff; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- X' eine geradkettige oder verzweigte, aliphatische oder cycloaliphatische Polyolgruppe, die 2 bis 10 Hydroxygruppen aufweist und die Wertigkeit q besitzt, wobei die Kohlenstoffkette dieser Gruppe durch ein oder mehrere Schwefelatome oder Sauerstoffatome unterbrochen sein kann; eine oder mehrere Iminogruppen oder eine oder mehrere C₁₋₄-Alkyliminogruppen;
- q einen Wert von 2 bis 10.

19. Zusammensetzung nach Anspruch 18, in der die Verbindung der Formel (IX) ausgewählt ist unter Verbindungen, bei denen bedeuten:
- R¹ und R², die gleich oder verschieden sind, Wasserstoff; eine C₁₋₁₂-Alkylgruppe; eine C₁₋₈-Alkoxygruppe, einen wasserlöslich machenden Substituenten, der unter einer Carboxylatgruppe, einer Sulfonatgruppe oder einer Ammoniumgruppe ausgewählt ist;
- R³ eine Gruppe COOR⁵; CONR⁵R⁶; CN; eine C₃₋₁₀-Cycloalkylgruppe; eine C₇₋₁₀-Bicycloalkylgruppe ;
- R⁵ und R⁶, die gleich oder verschieden sind, eine geradkettige oder verzweigte C₁₋₂₀-Alkylgruppe; eine C₃₋₁₀-Cycloalkylgruppe, eine C₇₋₁₀-Bicycloalkylgruppe, Naphthyl oder ggf. substituiertes Phenyl;
- X' eine Polyolgruppe, die 2 bis 6 Hydroxygruppen und insbesondere 2 bis 4 Hydroxygruppen aufweist.

20. Zusammensetzung nach Anspruch 19, in der die Verbindung der Formel (IX) unter Verbindungen ausgewählt ist, bei denen
- X' einen Ethanolrest oder einen Pentaerythritrest bedeutet.

21. Zusammensetzung nach Anspruch 20, in der die Verbindung der Formel (IX) unter den folgenden Verbindungen ausgewählt ist:

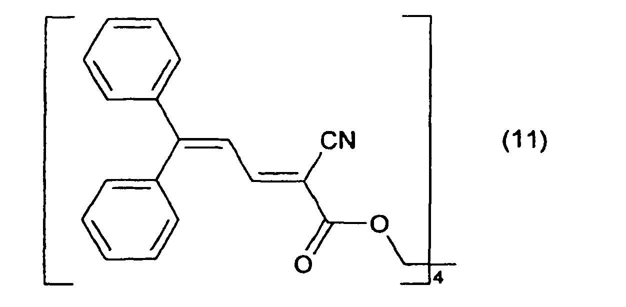

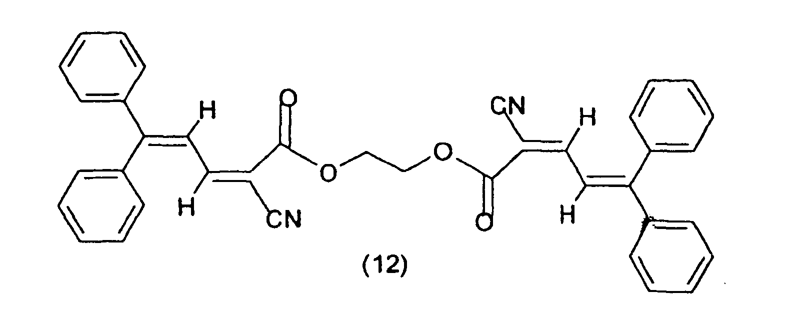

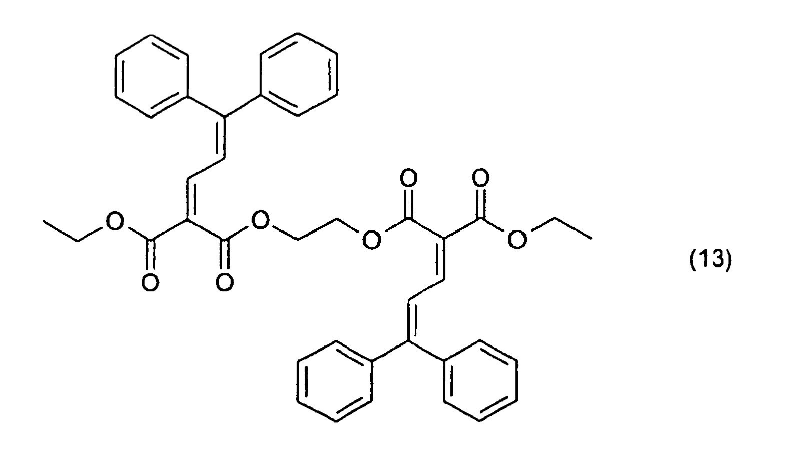

22. Zusammensetzung nach einem der Ansprüche 1 bis 21, **dadurch gekennzeichnet, dass** sie ferner weitere organische Filter enthält, die im UV-A-Bereich und/oder im UV-B-Bereich aktiv sind.

23. Zusammensetzung nach Anspruch 22, bei der das oder die zusätzlichen organischen UV-Filter ausgewählt sind unter den Anthranilaten; den Salicylsäurederivaten; den Campherderivaten; den Benzophenon-Derivaten; den β,β-Diphenylacrylat-Derivaten; den Benzotriazol-Derivaten; den nicht lichtempfindlichen Triazinderivaten; den Benzalmalonat-Derivaten; den Benzimidazol-Derivaten; den Imidazolinen; den Bisbenzoazolyl-Derivaten; den Derivaten der p-Aminobenzoesäure (PABA); den Derivaten von Methylen-bis(hydroxyphenylbenzotriazol); polymeren Filtern und Silicon-Filtern; den von α-Alkylstyrolen abgeleiteten Dimeren.

24. Zusammensetzung nach Anspruch 23, **dadurch gekennzeichnet, dass** das organische UV-Filter oder die organischen UV-Filter unter folgenden Verbindungen ausgewählt sind:
- Ethylhexyl Salicylate,
- Octocrylene,
- Phenylbenzimidazole Sulfonic Acid,
- Benzophenone-3,
- Benzophenone-4,
- Benzophenone-5,
- 4-Methylbenzylidene Camphor,
- Terephthalylidene Dicamphor Sulfonic Acid,
- Disodium Phenyl Dibenzimidazole Tetra-sulfonate,
- 2,4,6-Tris(4'-amino-benzaldiisobutylmalonat)-s-triazin,
- Anisotriazine,
- Methylene Bis-Benzotriazolyl Tetramethylbutylphenol,
- Drometrizole Trisiloxane
sowie Gemischen dieser Verbindungen.

25. Zusammensetzung nach einem der Ansprüche 1 bis 24, **dadurch gekennzeichnet, dass** sie ferner Pigmente oder Nanopigmente von Metalloxiden enthält, die gegebenenfalls ummantelt sind.

26. Zusammensetzung nach Anspruch 25, **dadurch gekennzeichnet, dass** die Pigmente oder Nanopigmente ausgewählt sind unter Titanoxid, Zinkoxid, Eisenoxid, Zirconiumoxid und Ceroxid sowie Gemischen dieser Verbindungen, wobei sie gegebenenfalls ummantelt sind.

27. Zusammensetzung nach einem der Ansprüche 1 bis 26, **dadurch gekennzeichnet, dass** sie ferner mindestens ein Mittel zur künstlichen Bräunung und/oder künstlichen Braunfärbung der Haut enthält.

28. Zusammensetzung nach einem der Ansprüche 1 bis 27, **dadurch gekennzeichnet, dass** sie ferner mindestens einen Hilfsstoff enthält, der ausgewählt ist unter Fettsubstanzen, organischen Lösungsmitteln, ionischen oder nichtionischen Verdickungsmitteln, reizlindernden Mitteln, Antioxidantien, Mitteln gegen freie Radikale, Trübungsmitteln, Stabilisatoren, Emollientien, Siliconen, α-Hydroxysäuren, Antischaummitteln, hydratisierenden Mitteln, Vitaminen, Insektenrepellents, Parfums, Konservierungsmitteln, grenzflächenaktiven Mitteln, entzündungshemmenden Mitteln, Antagonisten der Substanz P, Füllstoffen, Polymeren, Treibmitteln, Mitteln zum Alkalischmachen oder zum Ansäuern, Färbemitteln.

29. Zusammensetzung nach einem der Ansprüche 1 bis 28, **dadurch gekennzeichnet, dass** es sich um eine Zusammensetzung zum Schutz der menschlichen Epidermis oder um eine Sonnenschutzzusammensetzung handelt und die Zusammensetzung in Form einer nichtionischen Vesikeldispersion, einer Emulsion, insbesondere einer Emulsion vom Öl-in-Wasser-Typ, einer Creme, einer Milch, eines Gels, einer Gelcreme, einer Suspension, einer Dispersion, eines Puders, eines festen Stiftes, eines Schaums oder eines Sprays vorliegt.

30. Zusammensetzung nach einem der Ansprüche 1 bis 29, **dadurch gekennzeichnet, dass** es sich um eine Zusammensetzung zum Schminken der Augenbrauen, der Wimpern oder der Haut handelt und die Zusammensetzung in fester oder pastoser Form, wasserfrei oder wasserhaltig, einer Emulsion, einer Suspension oder einer Dispersion vorliegt.

31. Zusammensetzung nach einem der Ansprüche 1 bis 29, **dadurch gekennzeichnet, dass** es sich um eine Zusammensetzung zum Schutz der Haare gegen ultraviolette Strahlung handelt und die Zusammensetzung in Form eines Shampoos, einer Lotion, eines Gels, einer Emulsion oder einer nichtionischen Vesikeldispersion vorliegt.

32. Verwendung einer 4,4-Diarylbutadien-Verbindung wie in den vorhergehenden Ansprüchen definiert in kosmetischen Zusammensetzungen, die mindestens ein Dibenzoylmethan-Derivat in Kombination mit mindestens einem lichtempfindlichen 1,3,5-Triazin-Derivat wie in den vorhergehenden Ansprüchen definiert enthalten, um die Stabilität des 1,3,5-Triazin-Derivats in diesen Zusammensetzungen gegen UV-Strahlung (Photostabilität) zu verbessern, wobei das Gewichtsverhältnis von 4,4-Diarylbutadien-Verbindung zu Dibenzoylmethan-Derivat größer als 2,5 ist und diese Zusammensetzungen kein Cinnamat-Derivat enthalten.

## Patentansprüche (Patentansprüche für folgende(n) Vertragsstaat(en): DE)

1. Kosmetische oder dermatologische Zusammensetzung zur topischen Anwendung zum Schutz der menschlichen Epidermis gegen UV-Strahlung, **dadurch gekennzeichnet, dass** sie in einem kosmetisch akzeptablen Träger enthält:
(a) mindestens ein Dibenzoylmethan-Derivat und
(b) mindestens ein in Gegenwart eines Dibenzoylmethan-Derivats lichtempfindliches 1,3,5-Triazin-Derivat sowie
(c) mindestens eine 4,4-Diarylbutadien-Verbindung,
wobei das Gewichtsverhältnis von 4,4-Diarylbutadien-Verbindung zu Dibenzoylmethan-Derivat größer als 2,5 ist und die Zusammensetzung kein Cinnamat-Derivat enthält, und dass sie in Form einer nichtionischen Versikeldispersion, in Form einer Wasser/Öl-Emulsion, in Form eines Gels, in Form eines Puders, in Form eines festen Stifts, ein Form eines Aerosolschaums oder als Spray vorliegt.

2. Kosmetische oder dermatologische Zusammensetzung zur topischen Anwendung zum Schutz der Haare gegen UV-Strahlung, **dadurch gekennzeichnet, dass** sie in einem kosmetisch akzeptablen Träger enthält:
(a) mindestens ein Dibenzoylmethan-Derivat und
(b) mindestens ein lichtempfindliches 1,3,5-Triazin-Derivat in Gegenwart eines Dibenzoylmethan-Derivats sowie
(c) mindestens eine 4,4-Diarylbutadien-Verbindung,
wobei das Gewichtsverhältnis von 4,4-Diarylbutadien-Verbindung zu Dibenzoylmethan-Derivat größer als 2,5 ist und die Zusammensetzung kein Cinnamat-Derivat enthält, und dass sie in Form eines Shampoos, eines Gels oder einer nichtionischen Versikeldispersion vorliegt.

3. Zusammensetzung zum Schminken der Augenbrauen, der Wimpern oder der Haut,
**dadurch gekennzeichnet, dass** sie in einem kosmetisch akzeptablen Träger enthält
(a) mindestens ein Dibenzoylmethan-Derivat und
(b) mindestens ein lichtempfindliches 1,3,5-Triazin-Derivat in Gegenwart eines Dibenzoylmethan-Derivats sowie
(c) mindestens eine 4,4-Diarylbutadien-Verbindung,
wobei das Gewichtsverhältnis von 4,4-Diarylbutadien-Verbindung zu Dibenzoylmethan-Derivat größer als 2,5 ist und die Zusammensetzung kein Cinnamat-Derivat enthält, und dass sie in fester oder pastoser, wasserfreier oder wässeriger Form, in Form einer Öl/Wasser-Emulsion oder einer nichtionischen Versikeldispersion vorliegt.

4. Zusammensetzung nach einem der Ansprüche 1 bis 3, in der das 1,3,5-Triazin-Derivat die nachstehende Formel (I) aufweist: 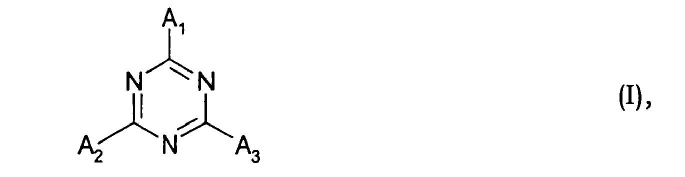 in der die Gruppen A₁, A₂ und A₃, die gleich oder verschieden sind, unter den Gruppen der Formel (II) ausgewählt sind: 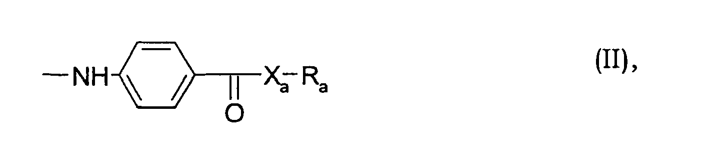 worin
- Xₐ, die gleich oder verschieden sein können, Sauerstoff oder die Gruppe -NH- bedeuten;
- Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Polyoxyethylen-Gruppe, die 1 bis 6 Ethylenoxideinheiten aufweist und deren endständige OH - Gruppe methyliert ist; einer Gruppe der nachstehenden Formeln (III), (IV) oder (V): 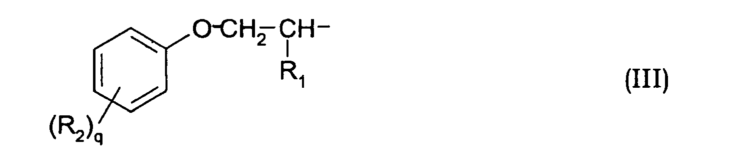 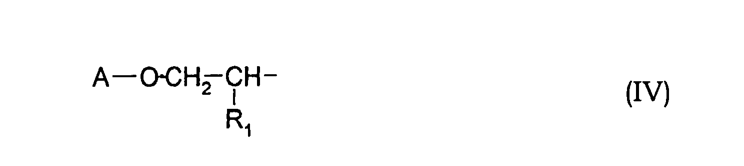 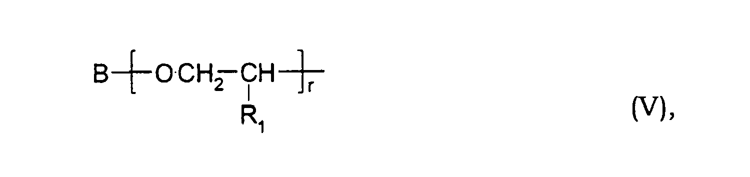
worin:
- R₁ Wasserstoff oder Methyl bedeutet;
- R₂ eine C₁₋₉-Alkylgruppe ist;
- q eine ganze Zahl von 0 bis 3 bedeutet;
- r eine ganze Zahl von 1 bis 10 bedeutet;
- A eine C₄₋₈-Alkylgruppe oder eine C₅₋₈-Cycloalkylgruppe bedeutet;
- B ausgewählt ist unter: einer geradkettigen oder verzweigten C₁₋₈-Alkylgruppe; einer C₅₋₈-Cycloalkylguppe; einer Arylgruppe, die gegebenenfalls mit einer oder mehreren C₁₋₄-Alkylgruppen substituiert ist.

5. Zusammensetzung nach Anspruch 4, **dadurch gekennzeichnet, dass** das 1,3,5-Triazin-Derivat der Formel (I) unter solchen Verbindungen ausgewählt ist, bei denen A₁, A₂ und A₃ die Formel (II) besitzen und bei denen folgende Merkmale vorliegen:
eine Gruppe Xₐ-Rₐ bedeutet die Gruppe -NH-Rₐ, wobei Rₐ ausgewählt ist unter: einer C₅₋₁₂-Cycloalkylgruppe, die gegebenenfalls mit einer oder mehreren C₁₋₄-Alkylgruppen substituiert ist; einer Gruppe der obigen Formel (III), (IV) oder (V), worin bedeuten:
- B eine C₁₋₄-Alkylgruppe;
- R₂ Methyl;
- die 2 übrigen Gruppen Xₐ-Rₐ bedeuten die Gruppe -O-Rₐ,
wobei Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der Formel (III), (IV) oder (V) wie oben, worin bedeuten:
- B eine C₁-₄-Alkylgruppe;
- R₂ Methyl.

6. Zusammensetzung nach Anspruch 4, **dadurch gekennzeichnet, dass** das 1,3,5-Triazin-Derivat unter Verbindungen ausgewählt ist, bei denen A₁, A₂ und A₃ die Formel (II) besitzen und bei denen die Gesamtheit folgender Merkmale vorliegt:
- eine oder zwei Gruppen Xₐ-Rₐ bedeuten die Gruppe -NH-Rₐ, wobei Rₐ ausgewählt ist unter: einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der obigen Formel (III), (IV) oder (V), worin bedeuten:
- B eine C₁₋₄-Alkylgruppe;
- R₂ Methyl;
- die übrige Gruppe Xₐ-Rₐ oder die beiden übrigen Gruppen Xₐ-Rₐ bedeuten die Gruppe -O-Rₐ, wobei Rₐ, die gleich oder verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der Formel (III), (IV) oder (V) wie oben, worin bedeuten:
- B eine C₁₋₄-Alkylgruppe;
- R₂ Methyl.

7. Zusammensetzung nach Anspruch 6, **dadurch gekennzeichnet, dass** das 1,3,5-Triazin-Derivat das Derivat der nachstehenden Formel ist: 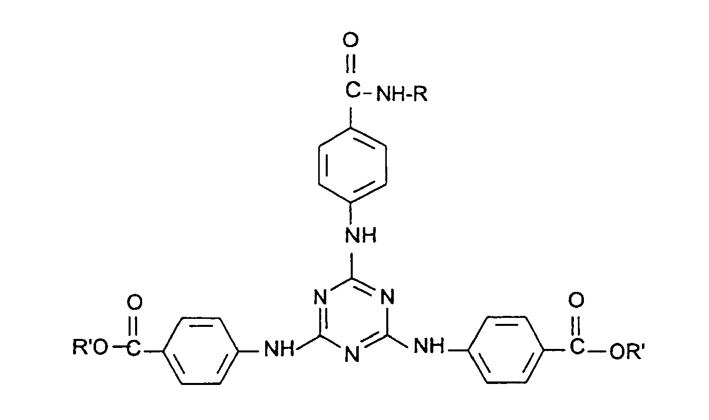 in der R' eine 2-Ethylhexyl-Gruppe und R eine tert.-ButylGruppe bezeichnen.

8. Zusammensetzung nach Anspruch 4, **dadurch gekennzeichnet, dass** das 1,3,5-Triazin-Derivat der Formel (I) unter Verbindungen ausgewählt ist, bei denen A₁, A₂ und A₃ die Formel (II) besitzen und bei denen folgende Merkmale vorliegen:
- Xₐ sind gleich und bedeuten Sauerstoff;
- Rₐ, die gleich oder verschieden sind, bedeuten eine C₆₋₁₂-Alkylgruppe oder eine Polyoxyethylen-Gruppe, die 1 bis 6 Ethylenoxideinheiten aufweist und deren endständige OH - Gruppe methyliert ist.

9. Zusammensetzung nach Anspruch 8, **dadurch gekennzeichnet, dass** das 1,3,5-Triazin-Derivat das Derivat der nachstehenden Formel ist: 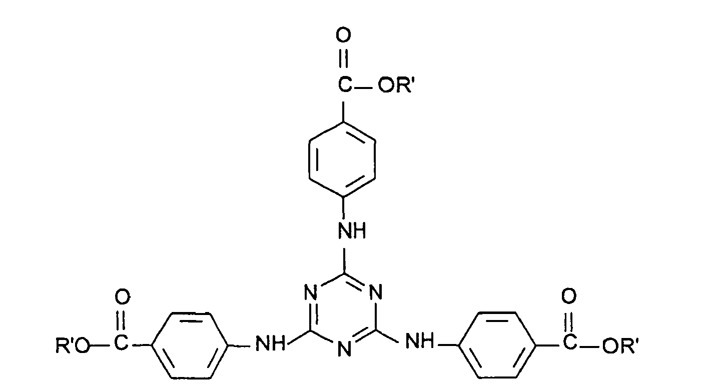 in der R' eine 2-Ethylhexyl-Gruppe bezeichnet.

10. Zusammensetzung nach einem der Ansprüche 1 bis 9, **dadurch gekennzeichnet, dass** das 1,3,5-Triazin-Derivat in Mengenanteilen von 0,5 bis 15 Gew.-% und bevorzugt in Mengenanteilen von 1 bis 10 Gew.-%, bezogen auf das Gesamtgewicht der Zusammensetzung, vorliegt.

11. Zusammensetzung nach einem der Ansprüche 1 bis 10, **dadurch gekennzeichnet, dass** das Dibenzoylmethan-Derivat ausgewählt ist unter:
- 2-Methyldibenzoylmethan
- 4-Methyldibenzoylmethan
- 4-Isopropyldibenzoylmethan
- 4-tert.-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-tert.-Butyl-4'-methoxydibenzoylmethan
- 2-Methyl-5-isopropyl-4'-methoxydibenzoylmethan
- 2-Methyl-5-tert.-butyl-4'-methoxydibenzoylmethan
- 2,4-Dimethyl-4'-methoxydibenzoylmethan
- 2,6-Dimethyl-4-tert.-butyl-4'-methoxydibenzoylmethan.

12. Zusammensetzung nach Anspruch 11, **dadurch gekennzeichnet, dass** das Dibenzoylmethan-Derivat 4-tert.-Butyl-4'-methoxy-dibenzoylmethan ist.

13. Zusammensetzung nach Anspruch 11, **dadurch gekennzeichnet, dass** das Dibenzoylmethan-Derivat 4-Isopropyldibenzoylmethan ist.

14. Zusammensetzung nach einem der Ansprüche 1 bis 13, in der das Dibenzoylmethan-Derivat in Mengenanteilen von 0,5 bis 15 Gew.-% und bevorzugt in Mengenanteilen von 1 bis 10 Gew.-%, bezogen auf das Gesamtgewicht der Zusammensetzung, vorliegt.

15. Zusammensetzung nach einem der Ansprüche 1 bis 14, in der die 4,4-Diarylbutadien-Verbindung die nachstehende Formel (VIII) aufweist: 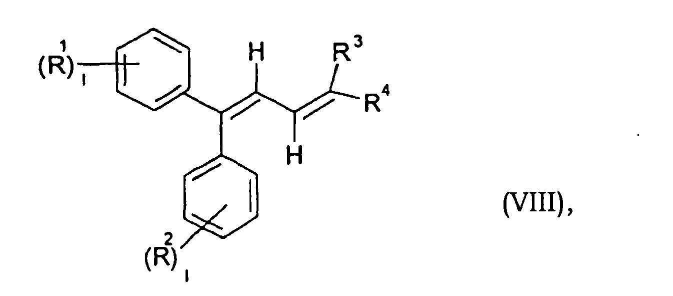 in der das Diensystem die Konfiguration Z,Z; Z,E; E,Z oder E,E aufweist oder Gemische dieser Konfigurationen vorliegen und wobei bedeuten:
- R¹ und R², die gleich oder verschieden sind, Wasserstoff; eine geradkettige oder verzweigte C₁₋₂₀-Alkylgruppe; eine C₂₋₁₀-Alkenylgruppe; eine C₁-₁₂-Alkoxygruppe; eine C₃₋₁₀-Cycloalkylgruppe; eine C₃₋₁₀-Cycloalkenylgruppe; eine geradkettige oder verzweigte C₁₋₂₀-Alkoxycarbonylgruppe; eine geradkettige oder verzweigte C₁₋₁₂-Monoalkylaminogruppe; eine geradkettige oder verzweigte C₁₋₁₂-Dialkylaminogruppe; eine Arylgruppe, eine Heteroarylgruppe oder einen wasserlöslich machenden Substituenten, der unter einer Carboxylatgruppe, einer Sulfonatgruppe und einem Ammoniumrest ausgewählt ist;
- R³ eine Gruppe COOR⁵; COR⁵; CONR⁵R⁶; CN; eine geradkettige oder verzweigte C₁₋₂₀-Alkylgruppe; eine C₂₋₁₀-Alkenylgruppe, eine C₃₋₁₀-Cycloalkylgruppe; eine C₇₋₁₀-Bicycloalkylgruppe, eine C₃₋₁₀-Cycloalkenylgruppe, eine C₇₋₁₀-Bicycloalkenylgruppe; eine C₆₋₁₈-Arylgruppe; eine C₃₋₇-Heteroarylgruppe;
- R⁴ eine Gruppe COOR⁶; COR⁶; CONR⁵R⁶; CN; eine geradkettige oder verzweigte C₁₋₂₀-Alkylgruppe, eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- R⁵ und R⁶, die gleich oder verschieden sind, Wasserstoff; [V]ₒ-R⁷; C₁₋₆-Alkylen-SO₃U; C₁₋₆-Alkylen-PO₃U; C₁₋₆-Alkylen-N(R⁸)₃⁺ D⁻; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- V eine Gruppe -CH₂-CH₂-W-, -CH₂CH₂CH₂W-, -CH(CH₃)-CH₂-W-, -CH₂-CH₂-CH₂-CH₂-W-, -CH₂-CH(CH₂CH₃)-W-;
- D Cl, Br, I, SO₄R⁹;
- U Wasserstoff, Na⁺, K⁺, Mg²⁺, Ca²⁺, Li⁺, Al³⁺, -N(R⁸)₄⁺;
- W O oder NH;
- R⁷ und R⁸, die gleich oder verschieden sind, Wasserstoff; eine geradkettige oder verzweigte C₁₋₆-Alkylgruppe; eine geradkettige oder verzweigte C₂₋₆-Alkenylgruppe; eine geradkettige oder verzweigte C₁₋₆-Acylgruppe;
- R⁹ Wasserstoff; eine geradkettige oder verzweigte C₁₋₆-Alkylgruppe; eine C₂₋₆-Alkenylgruppe;
- 1 einen Wert von 1 bis 3;
- o einen Wert von 0 bis 150.

16. Zusammensetzung nach Anspruch 15, in der die Verbindung der Formel (VIII) unter Verbindungen der nachstehenden Formel (VIIIa) ausgewählt ist: 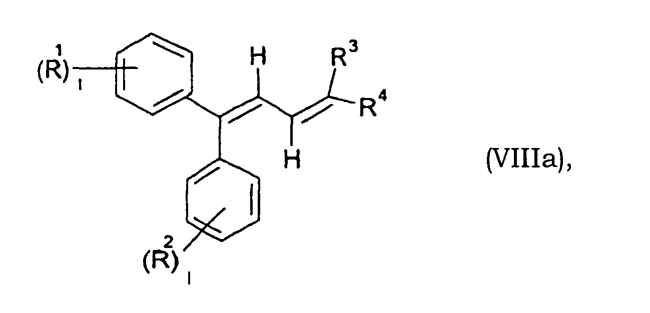 in der das Diensystem die Konfiguration ZZ; ZE; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe, eine C₁₋₈-Alkoxygruppe; einen wasserlöslich machenden Substituenten, der unter einer Carboxylatgruppe, einer Sulfonatgruppe und einem Ammoniumrest ausgewählt ist;
- R³ eine Gruppe COOR⁵; CONR⁵R⁶; CN;
- R⁴ eine Gruppe COOR⁶; CONR⁵R⁶;
- R⁵ Wasserstoff; [V]ₒ-R⁷; C₁₋₆-Alkylen-SO₃U; C₁₋₆-Alkylen-N(R⁸)₃⁺ D⁻;
- R⁶ [V]ₒ-R⁷; C₁₋₆-Alkylen-SO₃U; C₁₋₆-Alkylen-N(R⁸)₃⁺ D⁻;
- V eine Gruppe -CH₂-CH₂-O-, -CH₂CH₂CH₂O-, -CH(CH₃)-CH₂-O-;
- D Cl, Br, I, SO₄R⁹;
- U Wasserstoff, Na⁺, K⁺, Mg²⁺, Ca²⁺, Li⁺, Al³⁺, -N(R8)₄⁺;
- R⁷, R⁸ und R⁹, die gleich oder verschieden sind, Wasserstoff; eine geradkettige oder verzweigte C₁₋₃-Alkylgruppe;
- 1 einen Wert von 1 bis 3;
- o einen Wert von 0 bis 50.

17. Zusammensetzung nach Anspruch 16, in der die Verbindung der Formel (VIII) unter Verbindungen der nachstehenden Formel (VIIIb) ausgewählt ist: 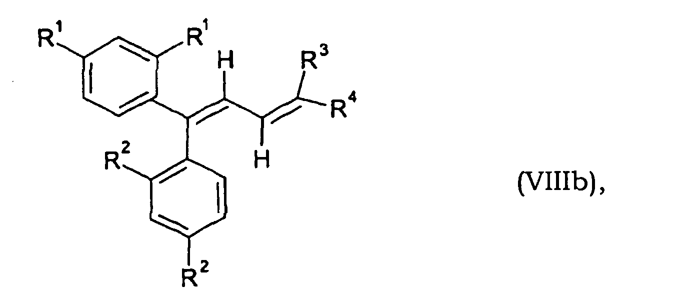 in der das Diensystem die Konfiguration ZZ; Z,E; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe; eine C₁₋₈-Alkoxygruppe;
- R³ eine Gruppe COOR⁵; CONR⁵R⁶; CN;
- R⁴ eine Gruppe COOR⁶; CONR5R⁶;
- R⁵ Wasserstoff; [V]ₒ-R⁷; C₁₋₆-Alkylen-SO₃U; C₁₋₆-Alkylen-N(R⁸)₃+ D⁻;
- R⁶ [V]ₒ-R⁷; C₁₋₆-Alkylen-SO₃U; C₁₋₆-Alkylen-N(R⁸)₃+ D⁻;
- V eine Gruppe -CH₂-CH₂-O-, -CH₂CH₂CH₂O-, -CH(CH₃)-CH₂-O-;
- D Cl, Br, I, SO₄R⁹;
- U Wasserstoff, Na⁺, K⁺, Mg²⁺, Ca²⁺, Li⁺, Al³⁺, -N(R⁸)₄+;
- R⁷, R⁸ und R⁹, die gleich oder verschieden sind, Wasserstoff; eine geradkettige oder verzweigte C₁₋₃-Alkylgruppe;
- o einen Wert von 0 bis 50.

18. Zusammensetzung nach Anspruch 17, in der die Verbindung der Formel (VIII) unter Verbindungen der nachstehenden Formel (VIIIc) ausgewählt ist: 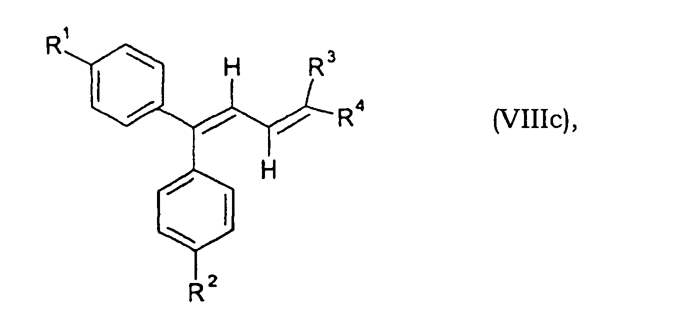 in der das Diensystem die Konfiguration Z,Z; Z,E; E,Z oder E,E aufweist oder Gemische dieser Konfigurationen vorliegen und wobei bedeuten:
- R¹ und R², die gleich oder verschieden sind, Wasserstoff; eine C₁₋₈-Alkylgruppe; eine C₁₋₈-Alkoxygruppe;
- R³ eine Gruppe COOR⁵. CONR⁵R⁶; CN;
- R⁴ eine Gruppe COOR⁶; CONR⁵R⁶;
- R⁵ Wasserstoff; [V]ₒ-R⁷; C₁₋₆-Alkylen-SO₃Y; C₁₋₆-Alkylen-N(R⁸)₃⁺ D⁻;
- R⁶ [V]o-R⁷; C₁₋₆-Alkylen-SO₃U; C₁₋₆-Alkylen-N(R⁸)₃⁺ D⁻;
- V eine Gruppe -CH₂-CH₂-O-, -CH₂CH₂CH₂O-, -CH(CH₃)-CH₂-O-;
- D Cl, Br, I, SO₄R⁹;
- U Wasserstoff, Na⁺, K⁺, Mg²⁺, Ca²⁺, Li⁺, Al3⁺, -N(R⁸)₄+;
- R⁷, R⁸ und R⁹, die gleich oder verschieden sind, Wasserstoff; eine geradkettige oder verzweigte C₁₋₃-Alkylgruppe;
- o einen Wert von 0 bis 50.

19. Zusammensetzung nach einem der Ansprüche 15 bis 17, in der die Verbindung der Formel (VIII) unter folgenden Verbindungen ausgewählt ist:

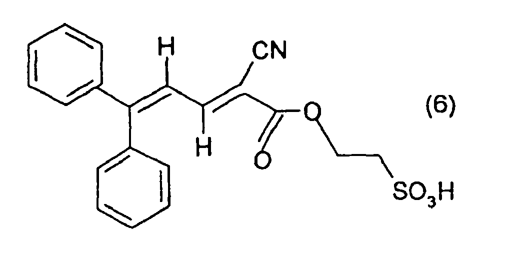

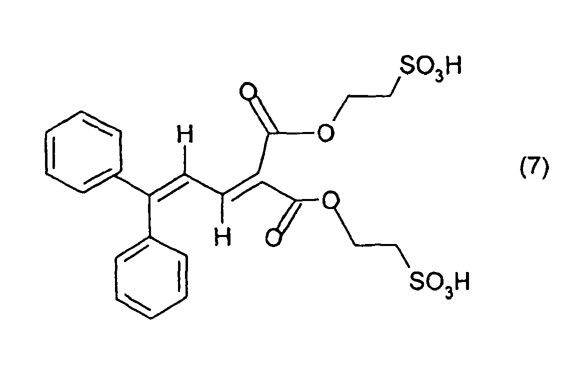

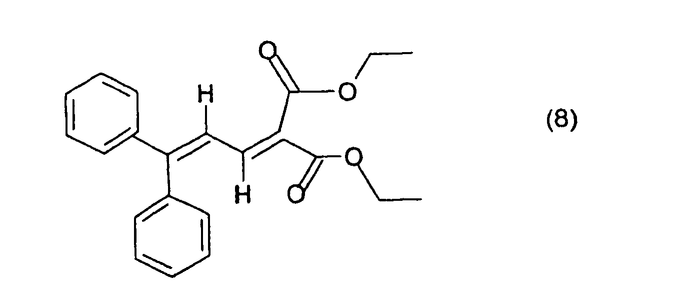

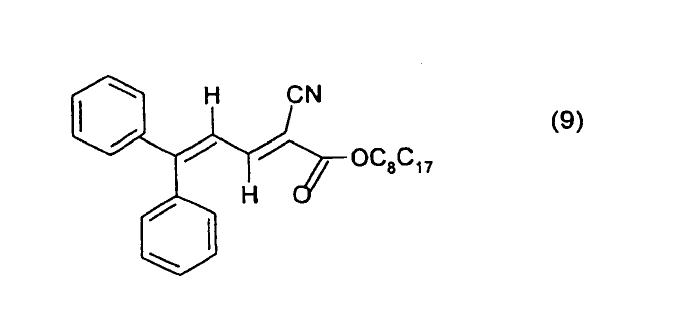

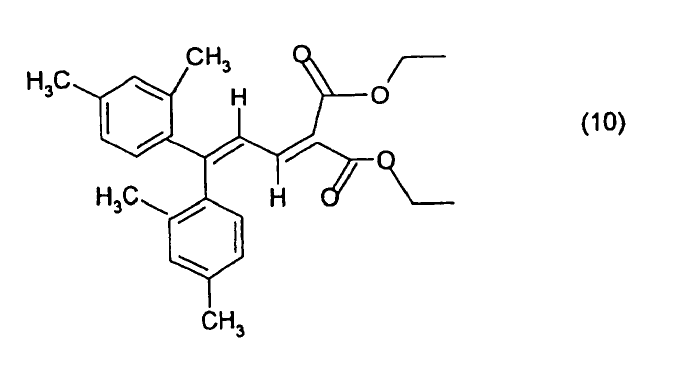

20. Zusammensetzung nach einem der Ansprüche 1 bis 14, in der die 4,4-Diarylbutadien-Verbindung ein Oligomer der nachstehenden Formel (IX) ist: 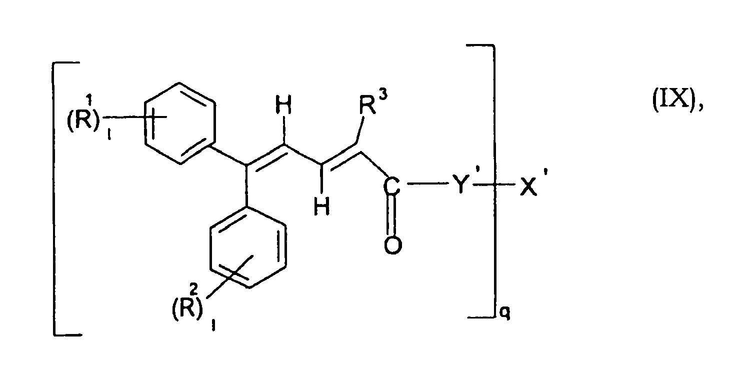 in der das Diensystem die Konfiguration Z,Z; Z,E; E,Z oder E,E aufweist oder Gemische dieser Konfigurationen vorliegen und wobei bedeuten:
- R¹, R², R³ und 1 dasselbe wie in Anspruch 17 für Formel (VIII) angegeben;
- Y' eine Gruppe -O- oder -NR¹⁰-;
- R¹⁰ Wasserstoff; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine Arylgruppe; eine Heteroarylgruppe;
- X' eine geradkettige oder verzweigte, aliphatische oder cycloaliphatische Polyolgruppe, die 2 bis 10 Hydroxygruppen aufweist und die Wertigkeit q besitzt, wobei die Kohlenstoffkette dieser Gruppe durch ein oder mehrere Schwefelatome oder Sauerstoffatome unterbrochen sein kann; eine oder mehrere Iminogruppen oder eine oder mehrere C₁-₄-Alkyliminogruppen;
- q einen Wert von 2 bis 10.

21. Zusammensetzung nach Anspruch 20, in der die Verbindung der Formel (IX) ausgewählt ist unter Verbindungen, bei denen bedeuten:
- R¹ und R², die gleich oder verschieden sind, Wasserstoff; eine C₁₋₁₂-Alkylgruppe; eine C₁₋₈-Alkoxygruppe, einen wasserlöslich machenden Substituenten, der unter einer Carboxylatgruppe, einer Sulfonatgruppe oder einer Ammoniumgruppe ausgewählt ist;
- R³ eine Gruppe COOR⁵; CONR⁵R⁶; CN; eine C₃₋₁₀-Cycloalkylgruppe; eine C₇₋₁₀-Bicycloalkylgruppe;
- R⁵ und R⁶, die gleich oder verschieden sind, eine geradkettige oder verzweigte C₁₋₂₀-Alkylgruppe; eine C₃₋₁₀-Cycloalkylgruppe, eine C₇₋₁₀-Bicycloalkylgruppe, Naphthyl oder ggf. substituiertes Phenyl;
- X' eine Polyolgruppe, die 2 bis 6 Hydroxygruppen und insbesondere 2 bis 4 Hydroxygruppen aufweist.

22. Zusammensetzung nach Anspruch 21, in der die Verbindung der Formel (IX) unter Verbindungen ausgewählt ist, bei denen
- X' einen Ethanolrest oder einen Pentaerythritrest bedeutet.

23. Zusammensetzung nach Anspruch 22, in der die Verbindung der Formel (IX) unter den folgenden Verbindungen ausgewählt ist:

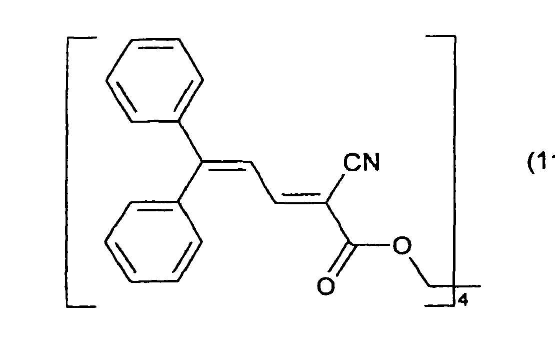

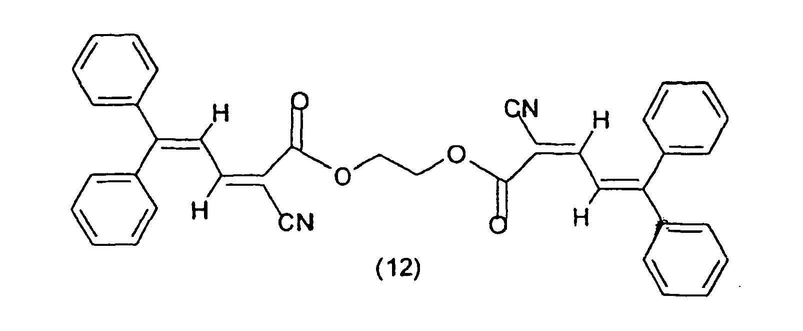

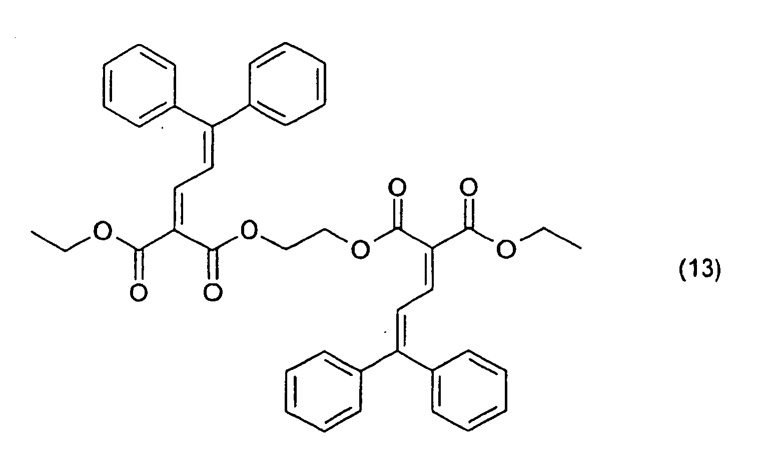

24. Zusammensetzung nach einem der Ansprüche 1 bis 23, **dadurch gekennzeichnet, dass** sie ferner weitere organische Filter enthält, die im UV-A-Bereich und/ oder im UV-B-Bereich aktiv sind.

25. Zusammensetzung nach Anspruch 24, bei der das oder die zusätzlichen organischen UV-Filter ausgewählt sind unter den Anthranilaten; den Salicylsäurederivaten; den Campherderivaten; den Benzophenon-Derivaten; den β,β-Diphenylacrylat-Derivaten; den Benzotriazol-Derivaten; den nicht lichtempfindlichen Triazinderivaten; den Benzalmalonat-Derivaten; den Benzimidazol-Derivaten; den Imidazolinen; den Bisbenzoazolyl-Derivaten; den Derivaten der p-Aminobenzoesäure (PABA); den Derivaten von Methylen-bis(hydroxyphenylbenzotriazol); polymeren Filtern und Silicon-Filtern; den von α-Alkylstyrolen abgeleiteten Dimeren.

26. Zusammensetzung nach Anspruch 25, **dadurch gekennzeichnet, dass** das organische UV-Filter oder die organischen UV-Filter unter folgenden Verbindungen ausgewählt sind:
- Ethylhexyl Salicylate,
- Octocrylene,
- Phenylbenzimidazole Sulfonic Acid,
- Benzophenone-3,
- Benzophenone-4,
- Benzophenone-5,
- 4-Methylbenzylidene Camphor,
- Terephthalylidene Dicamphor Sulfonic Acid,
- Disodium Phenyl Dibenzimidazole Tetra-sulfonate,
- 2,4,6-Tris(4'-amino-benzaldiisobutylmalonat)-s-triazin,
- Anisotriazine,
- Methylene Bis-Benzotriazolyl Tetramethylbutylphenol,
- Drometrizole Trisiloxane sowie Gemischen dieser Verbindungen.

27. Zusammensetzung nach einem der Ansprüche 1 bis 26, **dadurch gekennzeichnet, dass** sie ferner Pigmente oder Nanopigmente von Metalloxiden enthält, die gegebenenfalls ummantelt sind.

28. Zusammensetzung nach Anspruch 27, **dadurch gekennzeichnet, dass** die Pigmente oder Nanopigmente ausgewählt sind unter Titanoxid, Zinkoxid, Eisenoxid, Zirconiumoxid und Ceroxid sowie Gemischen dieser Verbindungen, wobei sie gegebenenfalls ummantelt sind.

29. Zusammensetzung nach einem der Ansprüche 1 bis 28, **dadurch gekennzeichnet, dass** sie ferner mindestens ein Mittel zur künstlichen Bräunung und/oder künstlichen Braunfärbung der Haut enthält.

30. Zusammensetzung nach einem der Ansprüche 1 bis 29, **dadurch gekennzeichnet, dass** sie ferner mindestens einen Hilfsstoff enthält, der ausgewählt ist unter Fettsubstanzen, organischen Lösungsmitteln, ionischen oder nichtionischen Verdickungsmitteln, reizlindernden Mitteln, Antioxidantien, Mitteln gegen freie Radikale, Trübungsmitteln, Stabilisatoren, Emollientien, Siliconen, α-Hydroxysäuren, Antischaummitteln, hydratisierenden Mitteln, Vitaminen, Insektenrepellents, Parfums, Konservierungsmitteln, grenzflächenaktiven Mitteln, entzündungshemmenden Mitteln, Antagonisten der Substanz P, Füllstoffen, Polymeren, Treibmitteln, Mitteln zum Alkalischmachen oder zum Ansäuern, Färbemitteln.

31. Verwendung einer 4,4-Diarylbutadien-Verbindung wie in den vorhergehenden Ansprüchen definiert in kosmetischen Zusammensetzungen, die mindestens ein Dibenzoylmethan-Derivat in Kombination mit mindestens einem lichtempfindlichen 1,3,5-Triazin-Derivat wie in den vorhergehenden Ansprüchen definiert enthalten, um die Stabilität des 1,3,5-Triazin-Derivats in diesen Zusammensetzungen gegen UV-Strahlung (Photostabilität) zu verbessern, wobei das Gewichtsverhältnis von 4,4-Diarylbutadien-Verbindung zu Dibenzoylmethan-Derivat größer als 2,5 ist und diese Zusammensetzungen kein Cinnamat-Derivat enthalten.
